# EUROPÄISCHE PATENTSCHRIFT

(11) **EP 2 064 959 B1**
(45) Veröffentlichungstag und Bekanntmachung des Hinweises auf die Patenterteilung: **25.07.2012**
(21) Anmeldenummer: 08167841.9
(22) Anmeldetag: 29.10.2008
(51) Int. Cl.: A23L 1/226, C07C 233/10, C07C 233/11

(54) **Aromatische Neo-Menthylamide als Geschmacksstoffe**
Aromatic Neomenthylamides as flavouring agents
Néomenthylamides aromatiques en tant qu'agents aromatisants

(30) Priorität: 31.10.2007 US 984023 P
(43) Veröffentlichungstag der Anmeldung: 03.06.2009
(73) Patentinhaber: Symrise AG, 37603 Holzminden (DE)
(72) Erfinder: Backes, Michael, 37603, Holzminden (DE); Vössing, Tobias, 37688, Beverungen (DE); Wöhrle, Ingo, 37603, Holzminden (DE)
(74) Vertreter: Eisenführ, Speiser & Partner

(56) Entgegenhaltungen:
- WO-A1-2006/099762
- READ J. AND STOREY R.A.: "CCCLXX. - Researches in the Menthone Series. Part VIII. Further Characterisation of the Optically Active Menthylamines." JOURNAL OF THE CHEMICAL SOCIETY, 1930, Seiten 2761-2769, XP009113338
- DATABASE BEILSTEIN [Online] Januar 2009 (2009-01), XP002519026 gefunden im XFIRE Database accession no. BRN: 3204404
- DATABASE BEILSTEIN [Online] Januar 2009 (2009-01), XP002519027 gefunden im XFIRE Database accession no. BRN: 3205137
- DATABASE BEILSTEIN [Online] Januar 2009 (2009-01), XP002519028 gefunden im XFIRE Database accession no. BRN: 3209060
- DATABASE BEILSTEIN [Online] Januar 2009 (2009-01), XP002519029 gefunden im XFIRE Database accession no. BRN:3210519

## Beschreibung

Die vorliegende Erfindung betrifft primär die Verwendung von bestimmten aromatischen Neo-Menthylamiden der Formeln (I) und (*ent-*I) (siehe unten) und entsprechenden Mischungen untereinander bzw. mit weiteren Verbindungen als Geschmacksstoffe bzw. Geschmacksstoffmischungen. Die erfindungsgemäß zu verwendenden Verbindungen sind insbesondere zum Erzeugen, Vermitteln, Modifizieren oder Verstärken eines Umami-Geschmacks geeignet. Die Erfindung betrifft des Weiteren bestimmte Zusammensetzungen, Zubereitungen und Halbfertigwaren, welche eine geschmacklich wirksame Menge der besagten Verbindungen der Formeln (I) und (*ent*-I) umfassen sowie bestimmte Verfahren zum Erzeugen, Vermitteln, Modifizieren und/oder Verstärken bestimmter Geschmackseindrücke, insbesondere Umami. Schließlich betrifft die Erfindung auch neue Verbindungen der Formeln (I) und (*ent*-I), welche besondere Geschmackseindrücke vermitteln, und entsprechende Mischungen.

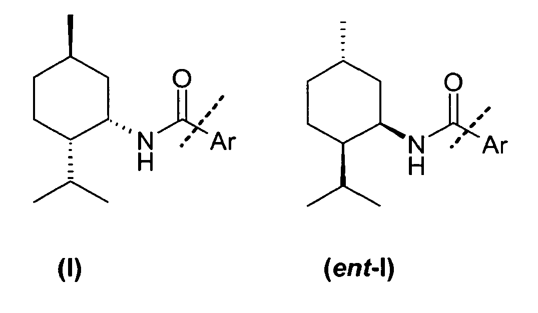

Weitere Aspekte ergeben sich aus der folgenden Beschreibung, den Ausführungsbeispielen, der Figur und den Ansprüchen.

Aromastoffe und Verbindungen mit außergewöhnlichen sensorischen Eigenschaften, die eine Amidgruppe tragen, sind seit langer Zeit bekannt. So haben viele bedeutende Kühlstoffe wie WS3, WS5 und WS 23 eine Amidstruktur:

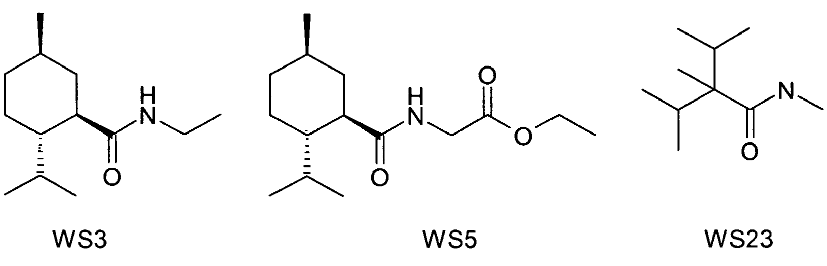

Ebenfalls zu den sensorisch bedeutsamen Amiden zählen die Scharfstoffe Capsaicin aus der Chilischote und das Piperin des weißen Pfeffers. Die natürlich vorkommenden Alkamide Pellitorin und Spilanthol zeigen neben einer speichelanregenden und kribbelnden Wirkung einen langanhaltenden und betäubenden Effekt im Mundraum:

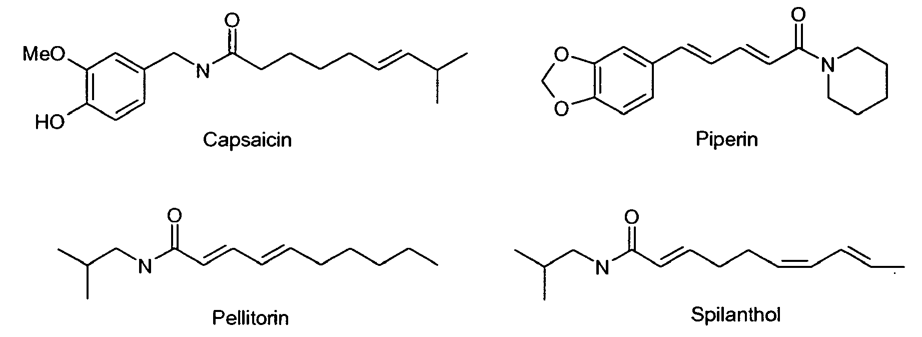

Angelehnt an die chemische Struktur des Spilanthols sind in den Veröffentlichungen US 2004/0202760 und US 2004/0202619 verschiedene Alkylidenamide vorgestellt worden, die ganz unterschiedliche sensorische Eindrücke wie Kribbeln, Betäuben, Bitterkeit, Mundfülle etc. bewirken. Für einige Verbindungen wie das N-Cyclopropyl-E2,Z6-nonadienamid (FEMA 4087), N-Ethyl-E2,Z6-dodecadienamid und N-Ethyl-E2,Z6-nonadienamid (FEMA 4113) wird dabei eine MSG-ähnliche Wirkung (MSG = monosodium glutamate, Natriumglutamat) bzw. ein Umami-ähnlicher Eindruck angegeben. Einige dieser Verbindungen erhielten bereits den GRAS (Generally Recognized as Safe) Status der FEMA (Flavor and Extract Manufacturers' Association) zur Verwendung in Lebensmitteln. In einer Weiterentwicklung (US 2006/057268 und 2006/068071) wurde u. a. - angelehnt an ein Geranylgrundgerüst - N-3,7-Dimethyl-2,6-octadienyl cyclopropylcarboxamid (FEMA 4267) als Salz- und Umami-Verstärker vorgestellt.

In der Veröffentlichung US 2005/084506 A1 wird eine Vielzahl geschmacksaktiver nicht-natürlicher Amide beschrieben, von denen N-(Heptan-4-yl)benzo[d][1,3]dioxol-5-carboxamid (FEMA 4232) bereits GRAS-Status besitzt.

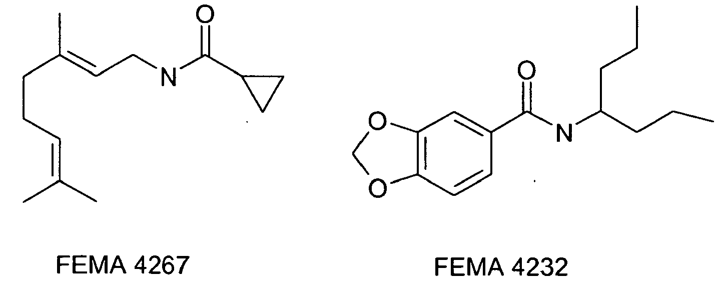

In den US-Provisional Applications 60/829,958 vom 18.10.2006 (Symrise) (entsprechend PCT/EP2007/061171) sowie 60/916,589 vom 08.05.2007 (Symrise) werden - angelehnt an ein Mentholgrundgerüst - neue künstliche Geschmacksstoffe auf Amidbasis vorgestellt, welche besonders zum Erzeugen, Modifizieren und Verstärken eines Umami-Geschmacks geeignet sind. In der Veröffentlichung WO 2006/099726 werden Verbindungen beschrieben, welche als Kühlstoffe wirken. Diese können u.a. die folgende Struktur aufweisen (Seite 5, Formel V):

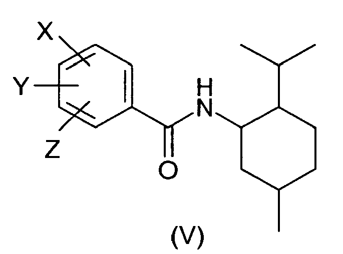

Als bevorzugte Stereoisomere werden (1R,2S,5R)-5-methyl-2-(1-methylethyl)-cyclohexanamin [(1R,2S,5R)-menthyl] und (2S,5R)-5-methyl-2-(1-methylethyl)-cyclohexanamin [(2S,5R)-menthyl] genannt (Seite 5, Zeilen 2 bis 4).

### Für die Formel V ist nur die Verbindung N(3-p-menthyl)o-methyl-terephtalamat

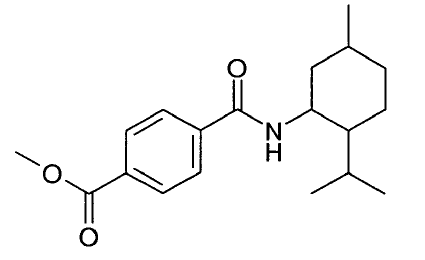

spezifisch offenbart (Seite 9, Beispiel 5, Formel D). Die Synthese dieser Verbindung erfolgt durch Umsetzung des entsprechenden Amins mit dem entsprechenden Säurechlorid. Offenbart sind zudem entsprechende Verfahren zum Erzielen einer Kühlwirkung in oral aufzunehmenden Produkten sowie Zusammensetzungen wie Mundpflegeprodukte, Medikamente (u.a. Tabletten), Lebensmittel, Süßwaren, Bonbons und Getränke.

In der Veröffentlichung WO 2005/020897 werden verschiedene Verbindungen offenbart, welche als Trp-r8-Modulatoren eingesetzt werden können, sowie Zusammensetzungen, einschließlich pharmazeutischer Zusammensetzungen, welche eine dieser Verbindungen sowie pharmazeutisch einsetzbare Hilfsstoffe, Träger oder Verdünnungsmittel enthalten. Die Struktur einiger in WO 2005/020897 offenbarter Verbindungen entspricht der folgenden Formel:

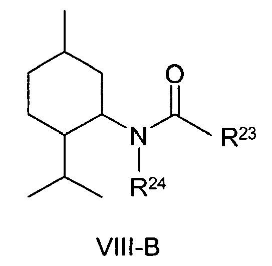

Für die Substituenten R²³ und R²⁴ wird insoweit je eine umfangreiche Liste möglicher, voneinander unabhängiger Bedeutungen offenbart.

Die Synthese von Verbindungen gemäß Formel VIIIB mit R²⁴ = Wasserstoff und beliebigem Substituenten R²³ wird gemäß der folgenden allgemeinen Vorschrift ausgeführt: Eine Lösung von Menthyl-3-amin-hydrochlorid (0,078 mmol in 0,7 ml Wasser) wird zu einer Lösung des entsprechenden Säurechlorids (0,078 mmol in 1 ml Diethylether) zugegeben, und anschließend 0,3 ml 0,5molarer Natronlauge hinzugefügt. Die Mischung wird bei Raumtemperatur 12 bis 18 Stunden lang geschüttelt. Die Diethylether-Schicht wird dann abgetrennt und das Lösungsmittel unter vermindertem Druck entfernt, um das Produkt zu erhalten. Beispielhaft gezeigt werden als Edukte (-)-Menthylamin und ein Säurechlorid der allgemeinen Formel ROCI sowie als Produkt ein Amid mit einem (-)-Menthylamin-Gerüst:

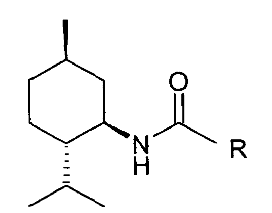

Der Geschmack der Verbindungen wird nicht beschrieben.

In der Patentanmeldung WO99/26927 werden Verbindungen offenbart, welche als Glutamatrezeptoren wirken. Deren grundsätzliche Struktur umfasst einen Rest R und einen aromatischen Ring oder ein aromatisches Ringsystem Ar, die durch einen Linker miteinander verbunden sind. Der Linker kann u.a. eine Amidgruppe sein. Der Geschmack der Verbindungen wird nicht beschrieben.

J. Chem. Soc. 1930, 2761 bis 2769, offenbart u.a. folgende Derivate des d-neo-Menthylamins: Benzoyl-d-neo-Menthylamin, Phenylcacetyl-d-neo-Menthylamin, Anisoyl-d-neo-(Menthylamin).

Diese Derivate werden nach folgender allgemeiner Vorschrift synthetisiert:
- Erwärmen einer Mischung enthaltend je 1 mol der entsprechenden freien Base und des entsprechenden Säurechlorids in trockenem Benzol
- schrittweise Zugabe einer verdünnten Lösung von wässrigem Natriumhydroxid bis zum Erreichen einer basischen Reaktion
- Waschen der Lösung in Benzol mit verdünnter Salzsäure und Wasser
- Umkristallisieren in wässrigem Alkohol, wässrigem Aceton, Ethylacetat oder leichtem Petroleum Zudem offenbart J. Chem. Soc. 1930, 2761 bis 2769 u.a. folgende Mischungen:
   Benzoyl-d-neo-Menthylamin (56 %) / Benzoyl-I-Menthylamin (44%)
   Benzoyl-d-neo-Menthylamin / Benzoyl-d-iso-Menthylamin.
      Der Geschmack der Verbindungen und mögliche Verwendungen werden nicht beschrieben.
      Transactions of the Faraday Society (1930) 26, 441-451 offenbart zudem die Mischungen
   Benzoyl-d-neo-Menthylamin / Benzoyl-I-neo-Menthylamin
   Phenylacetyl-d-neo-Menthylamin / Phenylacetyl-I-neo-Menthylamin sowie
   Anisoyl-d-neo-Menthylamin / Anisoyl-I-neo-Menthylamin

Die erste der genannten Mischungen ist auch in J. Chem. Soc. 1926, 2223-2234 offenbart.

Es besteht ein ständiger Bedarf, neue Geschmacks- und Aromastoffe aufzufinden, d.h. geschmacksaktive Verbindungen oder Verbindungen, die einen Geschmackseindruck erzeugen, vermitteln, modifizieren oder verstärken können. Insbesondere besteht Bedarf an solchen Verbindungen, die den Geschmackseindruck "Umami" erzeugen, vermitteln, modifizieren oder verstärken können.

Aufgabe der vorliegenden Erfindung war es daher, Verfahren und Verbindungen zur Verfügung zu stellen, mittels derer gewünschte Geschmacksnoten (vorzugsweise der Richtung "Umami") erzeugt, vermittelt, modifiziert oder verstärkt werden können.

Diese Aufgabe wird erfindungsgemäß gelöst durch die Verwendung einer Verbindung ausgewählt aus der Gruppe bestehend aus aromatischen Neo-Menthylamiden der Formeln (I) und (*ent-*I) 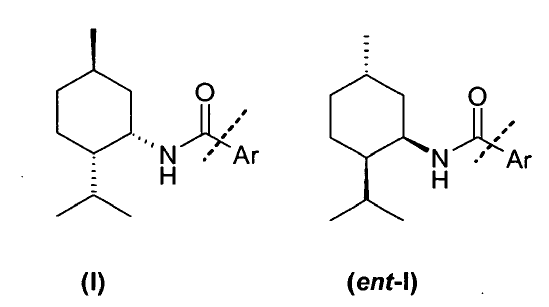 wobei in den Formeln (I) und (*ent-*I) gilt:
der aromatische Rest Ar ist ausgewählt aus der Gruppe bestehend aus: 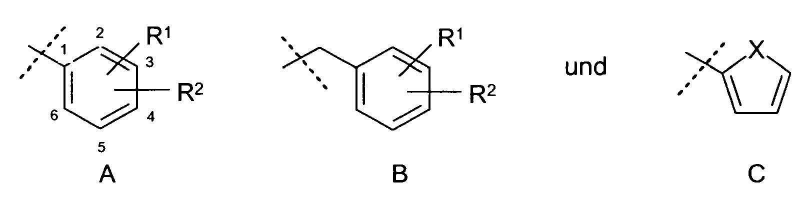
wobei die gestrichelte Linie die Bindung markiert, welche den aromatischen Rest Ar mit dem in Formel (I) oder (*engt*-I) benachbarten Carbonyl-C-Atom verknüpft, und wobei in den aromatischen Resten A und B gilt:
R¹ und R² sind unabhängig voneinander ausgewählt aus der Gruppe bestehend aus H, OH, OCH₃, OCH₂CH₃, OCH(CH₃)₂, COOCH₃, COOCH₂CH₃, COOCH(CH₃)₂,
   oder R¹ und R² sind benachbart und bilden zusammen eine Gruppe OCH₂O, und wobei im aromatischen Rest C gilt:
   X ist O oder S
   als Geschmacksstoffe.

Neben der Verwendung einzelner Verbindungen der Formeln (I) und (*ent-*I) wie oben definiert als Geschmacksstoffe betrifft die Erfindung auch die Verwendung von Mischungen bestehend aus zwei oder mehreren Verbindungen der Formeln (I) und (*ent-*I) wie oben definiert oder enthaltend eine oder mehrere Verbindungen der Formeln (I) und (*ent*-I) wie oben definiert als Geschmacksstoffmischungen.

Bevorzugt gilt für die Verbindung der Formeln (I) und (*ent-*I) bzw. für eine, mehrere oder sämtliche Verbindungen der Formeln (I) und (*engt-*I) in der Mischung:
R¹ und R² in den aromatischen Resten A bzw. B sind unabhängig voneinander ausgewählt aus der Gruppe bestehend aus H, OCH₃, COOCH₃,
   oder R¹ und R² sind benachbart und bilden zusammen eine Gruppe OCH₂O, und X im aromatischen Rest C ist O.

Besonders bevorzugt gilt für die Verbindung der Formeln (I) und (*ent-*I) wie oben definiert bzw. für eine, mehrere oder sämtliche Verbindungen der Formeln (I) und (*engt-*I) in der Mischung:
in den aromatischen Resten A bzw. B sind die Substituenten R¹ und R² beide H oder beide OCH₃,
oder einer der Substituenten R¹, R² ist H und der andere ist ausgewählt aus der Gruppe bestehend aus OCH₃ und COOCH₃,
oder R¹ und R² sind benachbart und bilden zusammen eine Gruppe OCH₂O.

Wiederum bevorzugt ist in den aromatischen Resten A bzw. B
(a) einer der Substituenten R¹ und R² H und der andere ist ausgewählt aus der Gruppe bestehend aus OCH₃ und COOCH₃, oder beide Substituenten R¹ und R² sind OCH₃, und die aus der Gruppe bestehend aus OCH₃ und COOCH₃ ausgewählten Substituenten befinden sich in der 3- oder/und 4-Stellung des Sechsrings des Substituenten A bzw. B, oder (b) R¹ und R² bilden zusammen eine Gruppe OCH₂O, welche die 3- und die 4-Stellung des Sechsrings miteinander verbindet.

Besonders bevorzugt ist die Verwendung von Verbindungen oder Mischungen wie oben definiert, wobei in der Verbindung der Formeln (I) und (*ent-*I) bzw. in einer, mehreren oder sämtlichen Verbindungen der Formeln (I) und (*ent-*I) in der Mischung der aromatische Rest Ar ausgewählt ist aus der Gruppe bestehend aus:

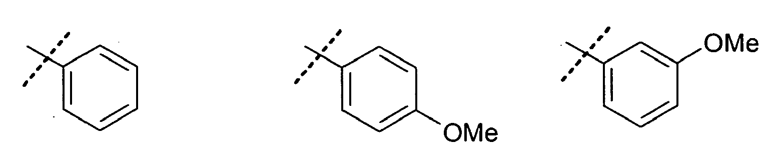

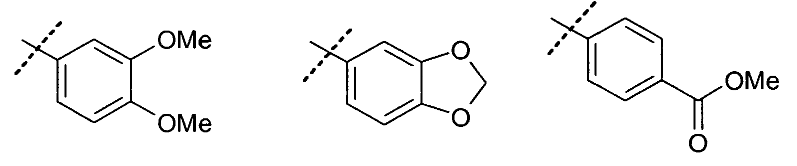

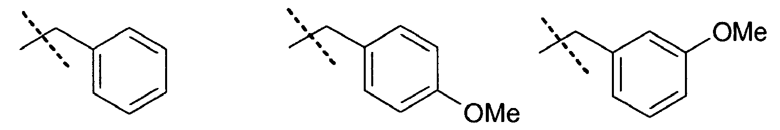

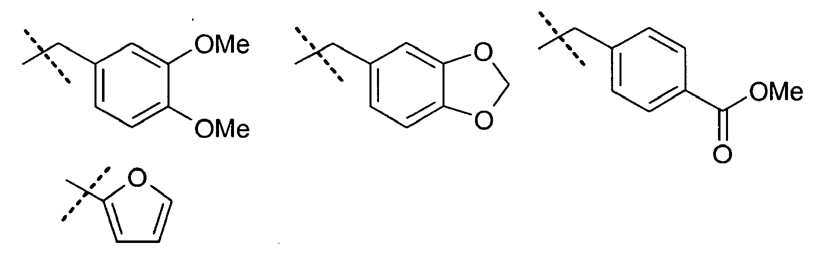

Spezielle bevorzugt verwendete Verbindungen der Formeln (I) und (*ent-*I) sind Verbindungen ausgewählt aus der Gruppe bestehend aus: 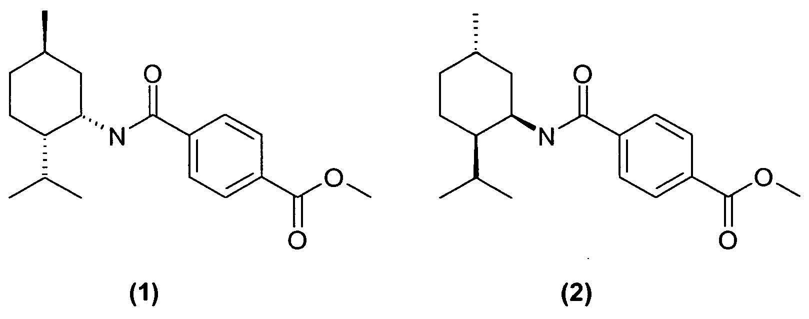 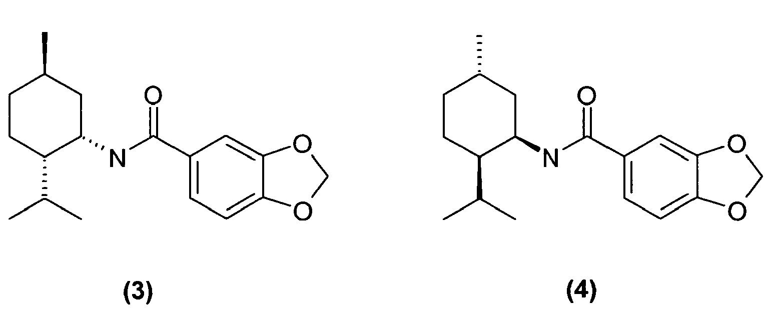 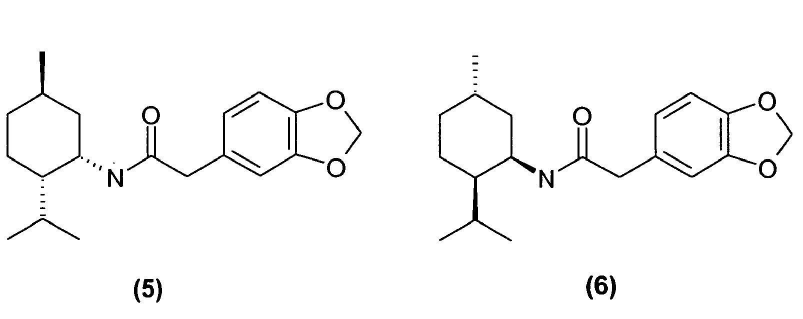
(1) *N*-((1*S*,2*S*,5*R*)-2-Isopropyl-5-methyl-cyclohexyl)-terephthalsäuremethylester
(2) *N*-((1*R*,2*R*,5*S*)-2-Isopropyl-5-methyl-cyclohexyl)-terephthalsäuremethylester
(3) Benzo[1,3]dioxol-5-carbonsäure ((1S,2S,5R)-2-isopropyl-5-methylcyclohexyl)-amid
(4) Benzo[1,3]dioxol-5-carbonsäure ((1*R*,2*R*,5*S*)-2-isopropyl-5-methylcyclohexyl)-amid
(5) 2-Benzo[1,3]dioxol-5-yl-*N*-((1S,2S,5R)-2-isopropyl-5-methylcyclohexyl)acetamid
(6) 2-Benzo[1,3]dioxol-5-yl-*N*-((1*R*,2*R*,5*S*)-2-isopropyl-5-methylcyclohexyl)acetamid,
wobei die Verbindungen einzeln oder in Mischungen (bestehend aus zwei oder mehreren Verbindungen der Formeln (I) und (*engt*-I) oder enthaltend eine oder mehrere Verbindungen der Formeln (I) und (*ent-*I) verwendet werden. Bevorzugt enthalten die erfindungsgemäß zu verwendenden Mischungen zwei oder mehrere der o.g. besonders bevorzugten Verbindungen der Formeln (I) und (*ent-*
I) bzw. bestehen aus zwei oder mehreren der o.g. besonders bevorzugten Verbindungen der Formeln (I) und (*ent-*I)*.*

Weiterhin bevorzugt ist die Verwendung von Mischungen enthaltend ein oder mehrere Enantiomerenpaare oder bestehend aus einem oder mehreren Enantiomerenpaaren aus jeweils einer Verbindung der Formel (I) und einer Verbindung der Formel (*ent-*I) wie oben definiert. Ein "Enantiomerenpaar" liegt somit vor, wenn eine Verbindung der Formel (I) und eine Verbindung der Formel (*ent-*I), in denen der aromatische Rest Ar identisch ist, nebeneinander vorliegen.

Am meisten bevorzugt ist die Verwendung einer Mischung, welche die folgenden Verbindungen enthält oder aus ihnen besteht: Benzo[1,3]dioxol-5-carbonsäure ((1*S*,2*S*,5*R*)-2-isopropyl-5-methyl-cyclohexyl)amid und Benzo[1,3]dioxol-5-carbonsäure ((1 *R*,2*R*,5*S*)-2-isopropyl-5-methyl-cyclohexyl)amid.

Ein weiterer Aspekt der Erfindung betrifft die Verwendung einer Mischung, welche aus folgenden Komponenten besteht oder diese enthält:
(a) eine Verbindung ausgewählt aus der Gruppe bestehend aus Verbindungen der Formeln (I) und (*ent-*I) wie oben definiert oder eine Mischung bestehend aus zwei oder mehreren Verbindungen oder enthaltend eine oder mehrere Verbindungen ausgewählt aus der Gruppe bestehend aus Verbindungen der Formeln (I) und (*ent*-I) wie oben definiert (vorzugsweise gemäß einer der vorstehend als bevorzugt bezeichneten Ausgestaltungen) sowie
(b) eine Verbindung ausgewählt aus der Gruppe bestehend aus Verbindungen der Formeln (II), (*ent*-II)*,* (III), (ent-III), (IV), (*ent*-IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent-*II), (III), (*ent*-III), (IV), (*ent*-IV): 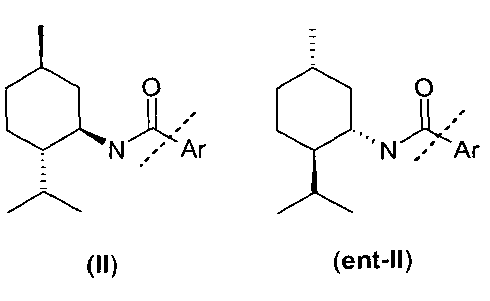 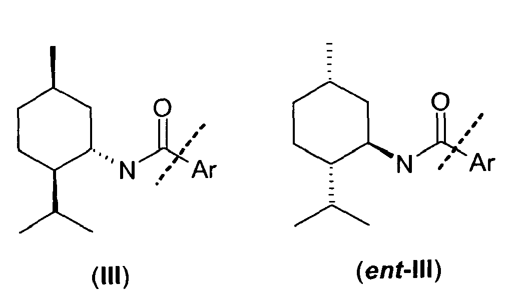 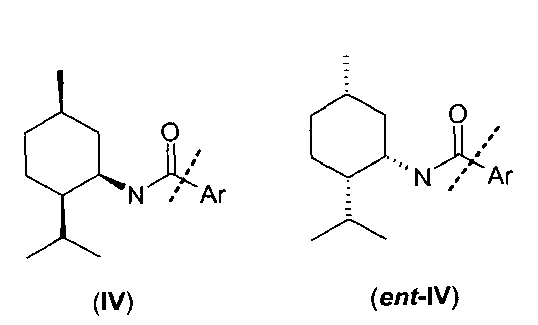
wobei in den Formeln (II), (*ent*-II), (III), (*ent-*III), (IV), (*ent-*IV) Ar unabhängig voneinander eine der oben für die Formeln (I) und (*ent-*I) angegebenen Bedeutungen besitzt, als Geschmacksstoffmischung.

In der vorstehend definierten Mischung beträgt das Gewichtsverhältnis von (a) der Gesamtheit von Verbindungen der Formeln (I) und (*ent-*I) zu (b) der Gesamtheit von Verbindungen der Formeln (II), (*ent-*II)*,* (III), (*ent*-III), (IV) und (*ent*-IV) mindestens 60:40, bevorzugt mindestens 90:10, insbesondere bevorzugt mindestens 95:5.

In eigenen Untersuchungen hat sich gezeigt, dass die erfindungsgemäß zu verwendenden Verbindungen der Formeln (I) und (*ent*-I) bzw. die oben beschriebenen Mischungen in stark Natriumglutamat-reduzierten oder in Natriumglutamat-freien Lebensmitteln, so zum Beispiel in würzigen Lebensmitteln wie Tomatensuppe, Hühnersuppe, Knabbergebäck, Fertigpizza, Kartoffelchips und Popcorn einen Umami-Geschmack sowohl im Anfangsgeschmack (Impact) als auch in der längeranhaltenden Geschmackswahrnehmung besonders gut erzeugen, vermitteln, modifizieren und/oder verstärken können und daher das Geschmackserlebnis als angenehm empfunden wird, in vielen Fällen sogar als bevorzugt gegenüber Natriumglutamat.

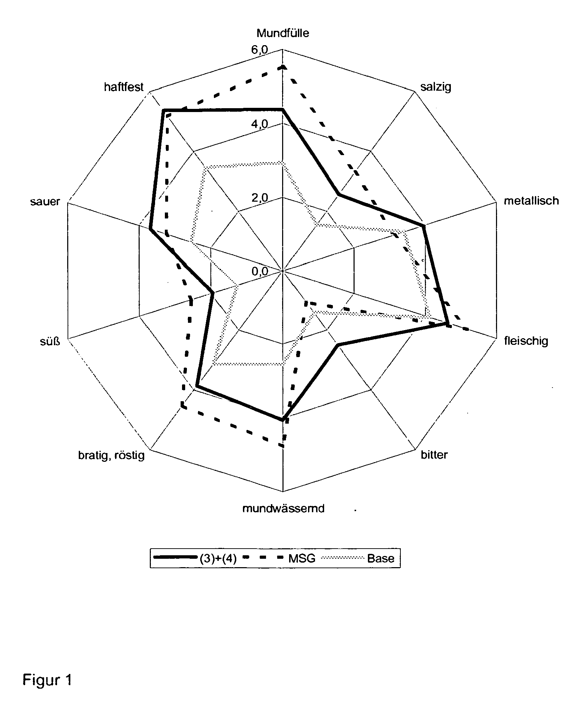

Im Vergleich zu anderen, nach Umami schmeckenden Verbindungen zeichnet sich beispielsweise eine Mischung aus Benzo[1,3]dioxol-5-carbonsäure ((1*S*,2*S*,5*R*)-2-isopropyl-5-methyl-cyclohexyl)-amid (3) und Benzo[1,3]dioxol-5-carbonsäure ((1*R*,2*R*,5*S*)-2-isopropyl-5-methyl-cyclohexyl)-amid (4) durch einen klaren, dem Natriumglutamat (MSG) sehr nahekommenden Umami-Geschmack aus. Dies zeigt auch das als Figur 1 beigefügte Spiderdiagramm, in welchem ein amerikanischer Rindfleischbouillon als Base mit (i) einer solchen Base mit einem Zusatz von 5 ppm einer 1:1 Mischung aus Benzo[1,3]dioxol-5-carbonsäure ((1*S*,2*S*,5*R*)-2-isopropyl-5-methyl-cyclohexyl)-amid (3) und Benzo[1,3]dioxol-5-carbonsäure ((1*R*,2*R*,5*S*)-2-isopropyl-5-methyl-cyclohexyl)-amid (4) und (ii) mit einer solchen Base mit einem Zusatz von 0,05 Gew.-% MSG (Natriumglutamat) verglichen wird.

Dementsprechend betrifft die Erfindung auch die Verwendung einer Verbindung der Formeln (I) und (*ent-*I) wie oben definiert oder einer Mischung bestehend aus zwei oder mehreren Verbindungen oder enthaltend eine oder mehrere Verbindungen der Formeln (I) und (*ent-*I) bzw. einer Mischung wie oben definiert umfassend (a) eine Verbindung der Formeln (I) und (*ent*-I) oder eine Mischung enthaltend oder bestehend aus Verbindungen der Formeln (I) und (*ent-*I) sowie (b) eine Verbindung der Formeln (II), (*ent*-II), (III), (*ent*-III), (IV), (*ent*-IV) wie oben definiert oder eine Mischung enthaltend oder bestehend aus Verbindungen der Formeln (II), (*ent*-II), (III), (*ent*-III), (IV), (*ent*-IV) zum Erzeugen, Vermitteln, Modifizieren oder Verstärken eines Umami-Geschmacks. Dabei gilt hinsichtlich bevorzugter Verbindungen und Mischungen das oben Gesagte entsprechend.

Gemäß einem weiteren Aspekt betrifft die vorliegende Erfindung ein Verfahren zum Erzeugen, Vermitteln, Modifizieren oder Verstärken eines Geschmackseindrucks, insbesondere eines Umami-Geschmacks. Entsprechend dem erfindungsgemäßen Verfahren wird einer Substanz oder Zusammensetzung eine Verbindung der Formeln (I) und (*ent*-I) wie oben definiert oder eine Mischung bestehend aus zwei oder mehreren Verbindungen oder enthaltend eine oder mehrere Verbindungen der Formeln (I) und (*ent*-I*)* bzw. eine Mischung wie oben definiert umfassend (a) eine Verbindung der Formeln (I) und (*ent*-I) oder eine Mischung enthaltend oder bestehend aus Verbindungen der Formeln (I) und (*ent-*I) sowie (b) eine Verbindung der Formeln (II), (*ent*-II), (III), (*ent*-III), (IV), (*ent*-IV) wie oben definiert oder eine Mischung enthaltend oder bestehend aus Verbindungen der Formeln (II), (*ent*-II), (III), (*ent*-III), (IV), (*ent*-IV) in einer geschmacklich wirksamen Menge zugesetzt. Dabei gilt hinsichtlich bevorzugter Verbindungen und Mischungen das oben Gesagte entsprechend.

Während ein leichter Umami-Geschmackseindruck mit bitteren Beinoten schon bei den Verbindungen der Formeln (I) und (*ent*-I)*,* in denen der aromatische Rest Ar ausgewählt ist aus der Gruppe bestehend aus den aromatischen Resten Benzyl, Phenyl und Furyl, festgestellt wurde, lässt sich dieser Eindruck durch Variation des Sechsrings des Phenyl- oder Benzylrests mit sauerstoffhaltigen Substituenten R¹, R², insbesondere in 3- oder 4-Stellung noch verstärken.

Erstaunlicherweise geht durch eine weitere Methoxygruppe in 5-Stellung am Sechsring des Phenyl- oder Benzylrests der Umami-Eindruck komplett verloren und die Verbindung schmeckt nur noch bitter.

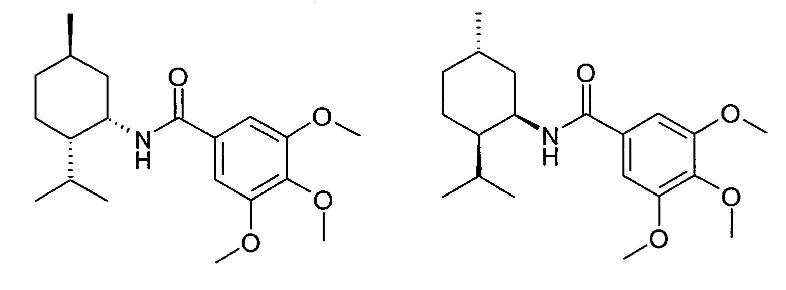

### Ähnliches gilt für die Verwendung von Zimtsäurederivaten wie z.B.:

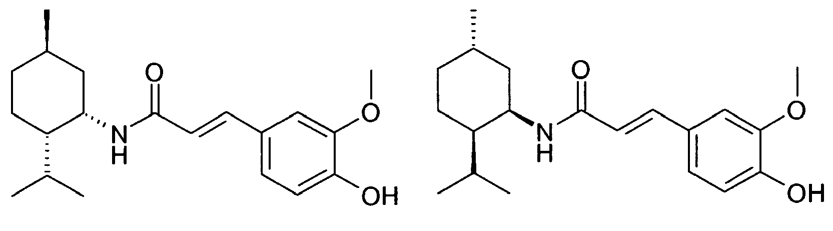

Die erfindungsgemäßen Verbindungen (1) und (2), in denen der aromatische Rest Ar in den Formeln (I) und (*ent*-I) 4-Benzoesäuremethylester ist, weisen hingegen einen Umami-Geschmack ohne stark bittere Beinoten auf. Ein sehr ausgeprägter Umami-Geschmack ist auch im Falle der [1,3]Benzodioxol-Derivate (3) und (4) festzustellen. Des Weiteren weisen die 5-Methyl[1,3]benzodioxol-Derivate (5) und (6) neben einem Umami-Geschmack auch eine leicht salzige Note auf.

Ein weiterer Aspekt der Erfindung betrifft neue Verbindungen der Formeln (I) und (*ent*-I)*.* Dementsprechend betrifft die Erfindung einzelne Verbindungen ausgewählt aus der Gruppe bestehend aus aromatischen Neo-Menthylamiden der Formeln (I) und (*ent*-I) 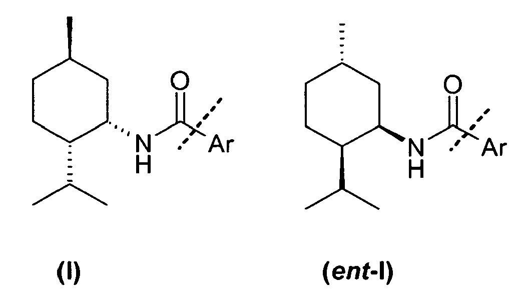 wobei in den Formeln (I) und (*ent*-I) gilt:
der aromatische Rest Ar ist ausgewählt aus der Gruppe bestehend aus: 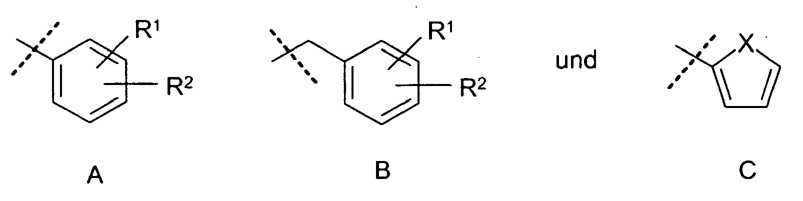
wobei die gestrichelte Linie die Bindung markiert, welche den aromatischen Rest Ar mit dem in Formel (I) oder (*ent-*I) benachbarten Carbonyl-C-Atom verknüpft, und wobei in den aromatischen Resten A und B gilt:
R¹ und R² sind unabhängig voneinander ausgewählt aus der Gruppe bestehend aus H, OH, OCH₃, OCH₂CH₃, OCH(CH₃)₂, COOCH₃, COOCH₂CH₃, COOCH(CH₃)₂,
   oder R¹ und R² sind benachbart und bilden zusammen eine Gruppe OCH₂O, und wobei im aromatischen Rest C gilt:
   X ist O oder S,
   mit der Maßgabe, dass (i) einzelne Verbindungen der Formel (I), in welchen der aromatische Rest Ar ausgewählt ist aus der Gruppe bestehend aus 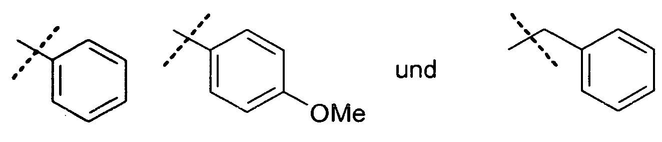 und (ii) Mischungen, welche aus einem Enantiomerenpaar der Formeln (I)/(*ent*-I) bestehen mit einem aromatischen Rest ausgewählt aus der Gruppe bestehend aus 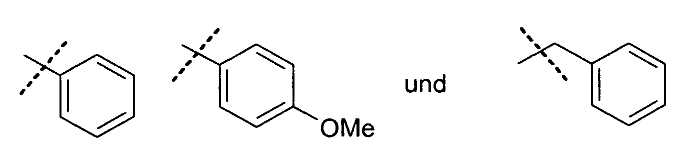 und (iii) Mischungen bestehend aus
   Benzoyl-d-neo-Menthylamin und Benzoyl-I-menthylamin bzw.
   Benzoyl-d-neo-Menthylamin und Benzoyl-d-iso-Menthylamin ausgeschlossen sind.

Vorzugsweise sind die einzelnen erfindungsgemäßen Verbindungen nicht Verbindungen der Formel (*ent*-I), in denen der aromatische Rest Ar ausgewählt ist aus der letztgenannten Gruppe.

Die vorliegende Erfindung betrifft nicht die in WO2006//099762 offenbarten Verbindungen der allgemeinen Formel V wie dort allgemein definiert sowie nicht die hinsichtlich der Stereochemie des Menthylgerüsts unspezifizierte Verbindung der Formel D (Beispiel 5), wohl aber entsprechende Neomenthyl-Derivate.

Neben einzelnen Verbindungen der Formeln (I) und (*ent*-I) wie oben definiert betrifft die Erfindung auch Mischungen bestehend aus zwei oder mehreren Verbindungen der Formeln (I) und (*ent-*I) wie oben definiert oder enthaltend eine oder mehrere Verbindungen der Formeln (I) und (*ent*-I) wie oben definiert.

Enthält die Mischung nur eine einzige Verbindung der Formeln (I) und (*ent*-I)*,* so ist diese besonders bevorzugt keine der vorstehend genannten nicht erfindungsgemäßen einzelnen Verbindungen der Formel (I). Auch Mischungen, welche als einzige Verbindung der Formeln (I) und (*ent*-I) eine Verbindung der Formel (*ent*-I) enthalten, in denen der aromatische Rest Ar ausgewählt ist aus der Gruppe bestehend aus 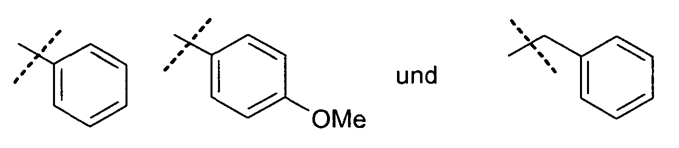 gehören nicht zu den bevorzugten erfindungsgemäßen Mischungen.

Mischungen, welche aus einem Enatiomerenpaar der Formeln (I)/(*ent*-I) mit einem aromatischen Rest ausgewählt aus der Gruppe bestehend aus 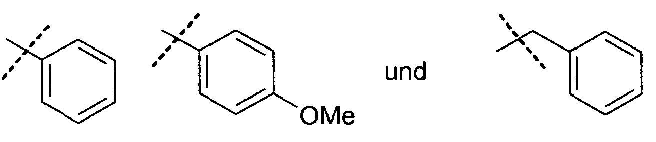 bestehen, sind ausgeschlossen.

Bevorzugt gilt für die oben definierte erfindungsgemäße einzelne Verbindung der Formeln (I) und (*ent*-I) bzw. für eine, mehrere oder sämtliche Verbindungen der Formeln (I) und (*ent-*I) in der Mischung:
R¹ und R² in den aromatischen Resten A bzw. B sind unabhängig voneinander ausgewählt aus der Gruppe bestehend aus H, OCH₃, COOCH₃,
   oder R¹ und R² sind benachbart und bilden zusammen eine Gruppe OCH₂O, und X im aromatischen Rest C ist O.

Besonders bevorzugt gilt für die erfindungsgemäße einzelne Verbindung der Formeln (I) und (*ent*-I) wie oben definiert bzw. für eine, mehrere oder sämtliche Verbindungen der Formeln (I) und (*ent*-I) in der Mischung:
in den aromatischen Resten A bzw. B sind die Substituenten R¹ und R² beide H oder beide OCH₃,
oder einer der Substituenten R¹, R² ist H und der andere ist ausgewählt aus der Gruppe bestehend aus OCH₃ und COOCH₃,
oder R¹ und R² sind benachbart und bilden zusammen eine Gruppe OCH₂O.

Wiederum bevorzugt ist in den aromatischen Resten A bzw. B (a) einer der Substituenten R¹ und R² H und der andere ist ausgewählt aus der Gruppe bestehend aus OCH₃ und COOCH₃, oder beide Substituenten R¹ und R² sind OCH₃, und die aus der Gruppe bestehend aus OCH₃ und COOCH₃ ausgewählten Substituenten befinden sich in der 3- oder/und 4-Stellung des Sechsrings des Substituenten A bzw. B, oder (b) R¹ und R² bilden zusammen eine Gruppe OCH₂O, welche die 3- und die 4-Stellung des Sechsrings miteinander verbindet.

Besonders bevorzugt sind erfindungsgemäße einzelne Verbindungen oder Mischungen wie oben definiert, wobei in der Verbindung der Formeln (I) und (*ent-*I) bzw. in einer, mehreren oder sämtlichen Verbindungen der Formeln (I) und (*ent-*I) in der Mischung der aromatische Rest Ar ausgewählt ist aus der Gruppe bestehend aus: 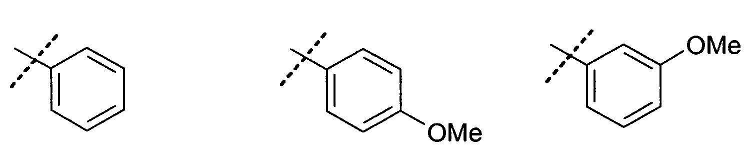 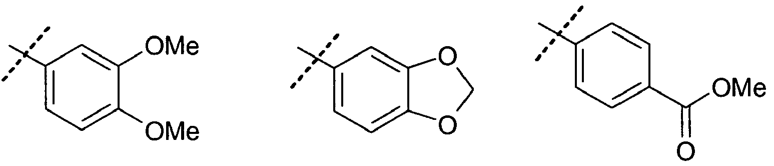 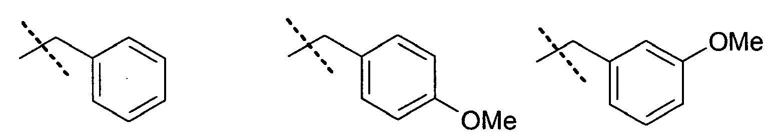 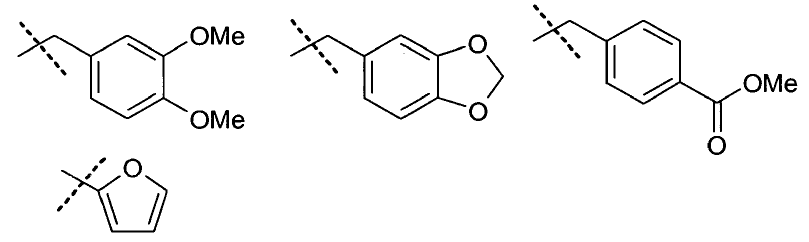 mit der Maßgabe, dass die einzelne Verbindung der Formel (I) keine ist, in welcher der aromatische Rest Ar ausgewählt ist aus der Gruppe bestehend aus 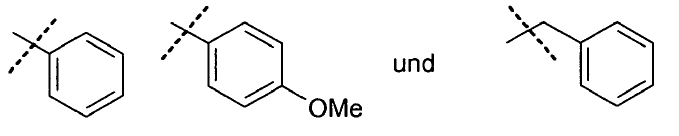 Spezielle bevorzugte erfindungsgemäße einzelne Verbindungen der Formeln (I) und (*ent-*I) sind Verbindungen ausgewählt aus der Gruppe bestehend aus: 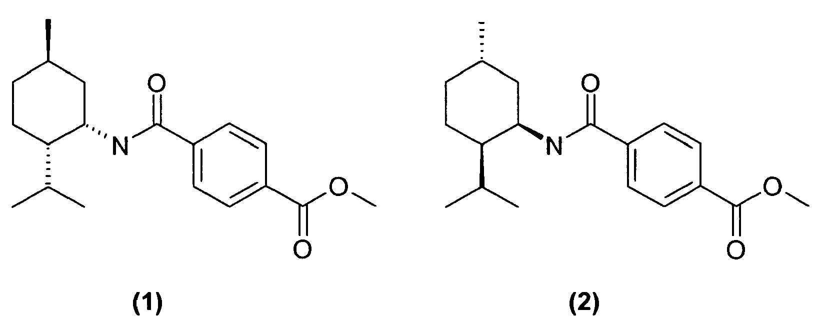 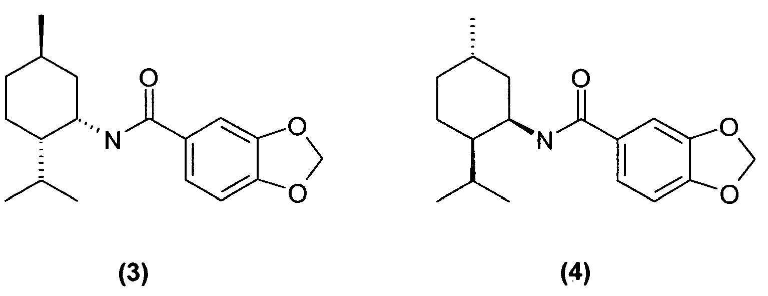 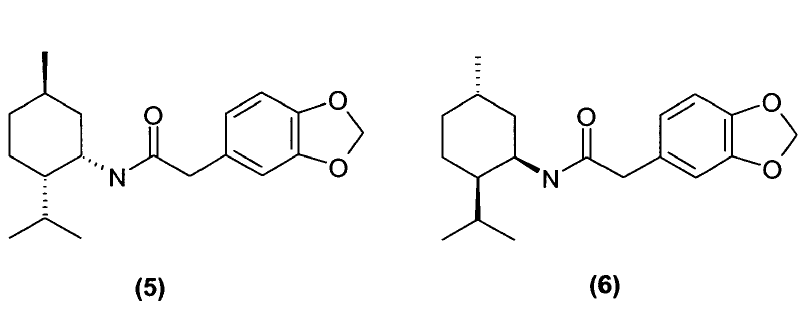
(1) *N*-((1*S*,2*S*,5*R*)-2-Isopropyl-5-methyl-cyclohexyl)-terephthalsäuremethylester
(2) *N*-((1*R*,2*R*,5*S*)-2-Isopropyl-5-methyl-cyclohexyl)-terephthalsäuremethylester
(3) Benzo[1,3]dioxol-5-carbonsäure ((1*S*,2*S*,5*R*)-2-isopropyl-5-methylcyclohexyl)-amid
(4) Benzo[1,3]dioxol-5-carbonsäure ((1*R*,2*R*,5*S*)-2-isopropyl-5-methylcyclohexyl)-amid
(5) 2-Benzo[1,3]dioxol-5-yl-*N*-((1S,2S,5R)-2-isopropyl-5-methylcyclohexyl)acetamid
(6) 2-Benzo[1,3]dioxol-5-yl-*N*-((1R,2R,5S)-2-isopropyl-5-methylcyclohexyl)acetamid,
wobei die Verbindungen einzeln oder in Mischungen (bestehend aus zwei oder mehreren Verbindungen der Formeln (I) und (*ent*-I) wie oben definiert oder enthaltend eine oder mehrere Verbindungen der Formeln (I) und (*ent*-I) wie oben definiert) vorliegen. Bevorzugt enthalten die erfindungsgemäßen Mischungen zwei oder mehrere der o.g. besonders bevorzugten Verbindungen der Formeln (I) und (*ent*-I) bzw. bestehen aus zwei oder mehreren der o.g. besonders bevorzugten Verbindungen der Formeln (I) und (*ent*-I)*.*

Weiterhin bevorzugt sind Mischungen enthaltend ein oder mehrere Enantiomerenpaare oder bestehend aus einem oder mehreren Enantiomerenpaaren aus jeweils einer Verbindung der Formel (I) und einer Verbindung der Formel (*ent*-I) wie oben definiert, natürlich weiterhin mit der oben ausgeführten Maßgabe, dass die dort angegebenen Mischungen ausgeschlossen sind.

Mischungen, welche neben einem der vorstehend genannten ausgeschlossenen Enantiomerenpaare keine weiteren Verbindungen der Formeln (I) und (*ent*-I) enthalten, sind nicht bevorzugt.

Die in J. Chem. Soc. 1930, 2761-2769, J. Chem. Soc. 1926, 2223-2234 und Transactions of the Faraday Society (1930) 26, 441-451 offenbarten Mischungen bzw. Zusammensetzungen, insbesondere solche enthaltend eine Verbindung der Formel (I), in der Ar ausgewählt ist aus der Gruppe bestehend aus 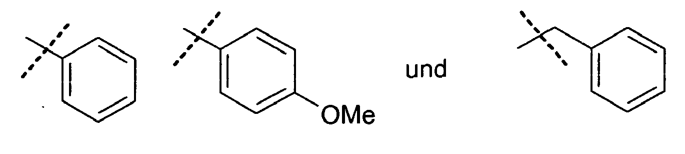 sind nicht Gegenstand der vorliegenden Erfindung.

Dies gilt insbesondere für solche Mischungen bzw. Zusammensetzungen, wie sie während des in J. Chem. Soc. 1930, 2761-2769, beschriebenen Prozesses umfassend die folgenden Schritte vorliegen (siehe oben).

Am meisten bevorzugt ist eine Mischung, welche die folgenden Verbindungen enthält oder aus ihnen besteht: Benzo[1,3]dioxol-5-carbonsäure ((1*S*,2*S*,5*R*)-2-isopropyl-5-methyl-cyclohexyl)-amid (3) und Benzo[1,3]dioxol-5-carbonsäure ((1*R*,2*R*,5*S*)-2-isopropyl-5-methylcyclohexyl)amid (4).

Ein weiterer Aspekt der Erfindung betrifft eine Mischung, welche aus folgenden Komponenten besteht oder diese enthält:
(a) eine Verbindung ausgewählt aus der Gruppe bestehend aus Verbindungen der Formeln (I) und (*ent*-I) wie oben definiert oder eine Mischung bestehend aus zwei oder mehreren Verbindungen oder enthaltend eine oder mehrere Verbindungen ausgewählt aus der Gruppe bestehend aus Verbindungen der Formeln (I) und (*ent*-I) wie oben definiert (vorzugsweise gemäß einer der vorstehend als bevorzugt bezeichneten Ausgestaltungen) sowie
(b) eine Verbindung ausgewählt aus der Gruppe bestehend aus Verbindungen der Formeln (II), (*ent*-II), (III), (*ent*-III), (IV), (*ent*-IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent-*II), (III), (*ent*-III), (IV), (*ent*-IV): 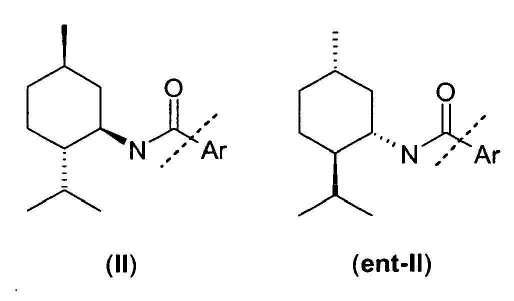 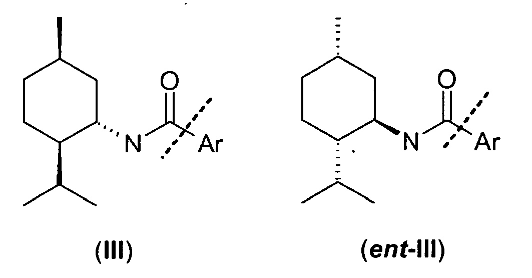 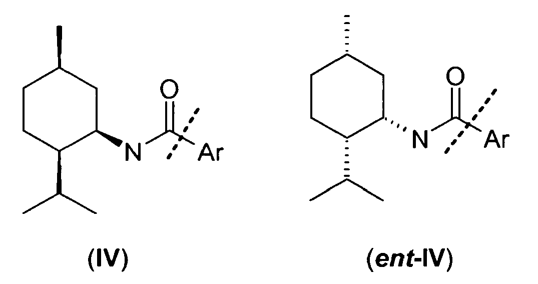
wobei in den Formeln (II), (*ent*-II), (III), (*ent*-III), (IV), (*ent*-IV) Ar unabhängig voneinander eine der in oben für die Formeln (I) und (*ent*-I) angegebenen Bedeutungen besitzt,
wobei Mischungen bestehend aus
Benzoyl-d-neo-Menthylamin und Benzoyl-I-menthylamin bzw.
Benzoyl-d-neo-Menthylamin und Benzoyl-d-iso-Menthylamin ausgeschlossen sind.

In der vorstehend definierten Mischung beträgt das Gewichtsverhältnis von (a) der Gesamtheit von Verbindungen der Formeln (I) und (*ent-*I) zu (b) der Gesamtheit von Verbindungen der Formeln (II), (*ent-*II), (III), (*ent*-III), (IV) und (*ent*-IV) mindestens 60:40, bevorzugt mindestens 90:10, insbesondere bevorzugt mindestens 95:5.

Nicht bevorzugt sind Mischungen, welche außer Benzoyl-d-neo-Menthylamin und Benzoyl-I-menthylamin bzw. Benzoyl-d-neo-Menthylamin und Benzoyl-d-iso-Menthylamin keine weiteren Verbindungen der Formeln (I), (*ent*-I)*,* (II), (*ent*-II)*,* (III), (*ent-*III), (IV), (*ent*-IV) enthalten.

Die in *J. Chem. Soc*. **1930**, 2761-2769 offenbarten Mischungen bzw. Zusammensetzungen, insbesondere solche enthaltend Benzoyl-d-neo-Menthylamin und Benzoyl-1-menthylamin bzw. Benzoyl-d-neo-Menthylamin und Benzoyl-d-iso-Menthylamin sind nicht Gegenstand der Erfindung. Dies gilt insbesondere für solche Mischungen bzw. Zusammensetzungen, wie sie während des Syntheseprozesses gemäß *J. Chem. Soc.* **1930**, 2761-2769 vorliegen.

Gemäß einem weiteren Aspekt betrifft die vorliegende Erfindung zum Verzehr geeignete Zusammensetzungen, umfassend oder bestehend aus einer geschmacklich wirksamen Menge einer oder mehrerer Verbindungen der Formeln (I) und (*ent*-I) bzw. einer Mischung wie oben definiert umfassend (a) eine oder mehrere Verbindungen der Formeln (I) und (*ent*-I) sowie (b) eine oder mehrere Verbindungen der Formeln (II), (*ent*-II), (II), (*ent*-III), (IV), (*ent*-IV) sowie einem oder mehreren weiteren zum Verzehr geeigneten Bestandteilen, wobei die Zusammensetzung keine Umkristal-lisationsmischung ist von einer Verbindung der Formel (I), in welcher der aromatische Rest Ar ausgewählt ist aus der Gruppe bestehend aus 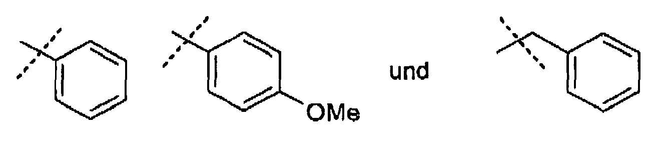 und wässrigem Alkohol als Lösungsmittel zur Umkristallisation. Dabei gilt hinsichtlich bevorzugter Verbindungen und Mischungen das oben Gesagte entsprechend.

Die erfindungsgemäßen zum Verzehr geeigneten der Ernährung, der Mundpflege oder dem Genuss dienenden Zubereitungen (Zusammensetzungen) sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe auch weiter unten) oder wieder aus der Mundhöhle entfernt zu werden (z.B. Kaugummis oder Zahnpasta). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Über oder sonstige Umschließungen verstanden, die dazu bestimmt sind, mitverschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Unter einer Halbfertigware ist im Zusammenhang des vorliegenden Textes ein Produkt zu verstehen, welches aufgrund eines sehr hohen Gehaltes an Aroma-und Geschmacksstoffen für die Verwendung als verzehrfertiges Lebensmittel ungeeignet ist. Erst durch Mischen mit mindestens einem weiteren Bestandteil (d.h. durch Verringern der Konzentration der betreffenden Aroma- und Geschmacksstoffe) und gegebenenfalls weitere Prozessschritte (z.B. Erwärmen, Gefrieren) wird die Halbfertigware in ein verzehrfertiges Lebensmittel überführt. Als Beispiel für Halbfertigwaren seien hier Tütensuppen, Backaromen und Puddingpulver genannt.

Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Areosole, Sprays, wie auch Kapseln, Granulate, Pastillen, Tabletten, Bonbons oder Kaugummis, ohne dass diese Aufzählung für die Zwecke dieser Erfindung limitierend verstanden werden soll.

Bevorzugte Mundpflegeprodukte (Mundhygieneprodukt) sind insbesondere solche in Form von Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi.

Kaugummis umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist auch die Kombination der Aromen mit weiteren Stoffen, die kühlende, wärmende und/oder mundwässerndernde Eigenschaften aufweisen.

Im Stand der Technik sind zahlreiche unterschiedliche Kaugummibasen bekannt,
wobei zwischen sogenannten "chewing gum" - oder "bubble gum" - Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono-und Diglyceride von Fettsäuren, z.B. Glycerinmonostearat.

Eine Reihe von erfindungsgemäßen Zusammensetzungen sind bevorzugt. Insbesondere bevorzugt ist eine (vorzugsweise sprühgetrocknete) Zusammensetzung, die neben einer geschmacklich wirksamen Menge einer oder mehrerer Verbindungen der Formeln (I) und (*ent*-I) bzw. einer Mischung wie oben definiert umfassend (a) eine oder mehrere Verbindungen der Formeln (I) und (*ent*-1) sowie (b) eine oder mehrere Verbindungen der Formeln (II), (*ent*-II), (III), (*ent*-III)*,* (IV), (*ent*-IV) einen oder mehrere zum Verzehr geeignete feste Trägerstoffe umfasst. Bevorzugte Zusammensetzungen bestehen aus der oder den erfindungsgemäß zu verwendenden Verbindungen der Formeln (I) und (*ent*-I) bzw. oben definierten Mischungen sowie dem oder den Trägerstoffen. Dabei gilt hinsichtlich bevorzugter Verbindungen und Mischungen das oben Gesagte entsprechend.

Vorteilhafte Trägerstoffe in diesen bevorzugten erfindungsgemäßen (vorzugsweise sprühgetrockneten) Zusammensetzungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliciumdioxid, Gummi Arabicum und Maltodextrine,
wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zusammensetzungen, die neben der oder den erfindungsgemäß zu verwendenden Verbindungen der Formeln (I) und (*ent*-I) bzw. oben definierten Mischungen noch einen oder mehrere feste Trägerstoffe umfassen, können beispielsweise durch mechanische Mischvorgänge, wobei gleichzeitig auch eine Zerkleinerung der Partikel der vorliegende Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Zusammensetzungen, die mittels Sprühtrocknung hergestellt wurden, besitzen eine mittlere Partikelgröße im Bereich von 30 bis 300 µm und eine Restfeuchte von kleiner oder gleich 5 Gew.-%.

Das Gewichtsverhältnis der Gesamtmasse der Verbindungen der Formeln (I) und (*ent-*I) bzw. -bei Verwendung der oben definierten Mischungen- das Gewichtsverhältnis der Gesamtmasse der Verbindungen der Formeln (I) und (*ent*-I)*,* (II), (*ent-*II), (III), (*ent-*III), (IV), (*ent*-IV) zu dem oder den zum Verzehr geeigneten festen Trägerstoffen liegt vorzugsweise im Bereich von 1 : 10 bis 1 : 100000, bevorzugt im Bereich von 1 : 50 (vorzugsweise von 1 : 100) bis 1 : 20000, besonders bevorzugt im Bereich von 1 : 100 (vorzugsweise von 1 : 1000) bis 1 : 5000, bezogen auf die Trockenmasse der Zusammensetzung.

In der erfindungsgemäßen Zusammensetzung liegt die Summe der Bestandteile umfassend (i) Verbindungen der Formeln (I) und (*ent-*I) bzw. oben definierte Mischungen umfassend (a) eine oder mehrere Verbindungen der Formeln (I) und (*ent-*I) sowie (b) eine oder mehrere Verbindungen der Formeln (II), (*ent*-II), (III), (*ent-*III)*,* (IV), (*ent-*IV) und (ii) Trägerstoffe vorzugsweise im Bereich von 70 bis 100 Gew.-%, bevorzugt im Bereich von 85 bis 100 Gew.-%.

Die Erfindung betrifft auch eine (vorzugsweise sprühgetrocknete) Zusammensetzung, die neben (i) einer geschmacklich wirksamen Menge einer oder mehrerer Verbindungen der Formeln (I) und (*ent-I*) bzw. einer Mischung wie oben definiert umfassend (a) eine oder mehrere Verbindungen der Formeln (I) und (*ent*-I) sowie (b) eine oder mehrere Verbindungen der Formeln (II), (*ent-*II), (III), (*ent-*III )*,* (IV), (*ent*-IV) und (ii) festen Trägerstoffen zusätzlich (iii) eine oder mehrere Aromakompositionen umfasst bzw. aus den genannten Komponenten besteht. Dabei gilt hinsichtlich bevorzugter Verbindungen und Mischungen das oben Gesagte entsprechend.

Eine solche Aromakomposition im Sinne der vorliegenden Erfindung umfasst mindestens einen flüchtigen Aromastoff (nicht gemeint sind hierbei allerdings Verbindungen der Formeln (I) und (*ent*-I) oder oben definierte erfindungsgemäß zu verwendende Mischungen). Der flüchtige Aromastoff ist dabei vorzugsweise eine sensorisch wirksame Komponente mit einem Dampfdruck von größer oder gleich 0,01 Pa bei 25°C, vorzugsweise einem Dampfdruck von größer oder gleich 0,025 Pa bei 25°C. Ein Großteil der flüchtigen Aromastoffe weist einen Dampfdruck von größer oder gleich 1 Pa bei 25°C auf, diese Aromastoffe werden für die Verwendung in erfindungsgemäßen Zusammensetzungen als bevorzugt angesehen.

Beispiele für Aromastoffe, die Bestandteil der Aromakomposition sein können, finden sich z.B. in K. Bauer, D. Garbe und H. Surburg, Common Fragrance and Flavor Materials, 4th. Ed., Wiley-VCH, Weinheim 2001. Es seien beispielsweise genannt: organische Säuren (gesättigt und ungesättigt) wie z.B. Buttersäure, Essigsäure, Methylbuttersäure, Capronsäure; Alkohole (gesättigt und ungesättigt) wie z.B. Ethanol, Propylenglykol, Octenol, cis-3-Hexenol, Benzylalkohol; Sulfide und Disulfide wie z.B. Dimethylsulfid, Difurfuryldisulfid, Methylthiopropanal; Thiole wie z.B. Methylfuranthiol; Pyrazine und Pyrroline wie z.B. Methylpyrazin, Acetylpyrazin, 2-Propionylpyrrolin, 2-Acetylpyrrolin.

Die Aromakomposition können auch in Form von Reaktionsaromen (Maillard-Produkte) und/oder Extrakten bzw. ätherischen Ölen von Pflanzen oder Pflanzenteilen bzw. Fraktionen davon eingesetzt werden.

Eine weitere bevorzugte, zum Verzehr geeignete erfindungsgemäße Zusammensetzung, welche (a) eine oder mehrere erfindungsgemäß zu verwendende einzelne Verbindungen der Formeln (I) und (*ent-*I) bzw. (b) eine oben definierte Mischung umfasst, ist eine Wasser-in-Öl (W/O) - Emulsion. Neben der oder den erfindungsgemäß zu verwendenden Verbindungen der Formel (I) und (*ent*-I) bzw. oben definierten Mischungen, umfasst eine solche Emulsion Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung. Dabei gilt hinsichtlich bevorzugter Verbindungen und Mischungen das oben Gesagte entsprechend.

Vorzugsweise umfasst eine solche erfindungsgemäße Zusammensetzung (W/O - Emulsion)
- insgesamt 0,01 bis 0,1 Gew.% an Verbindungen der Formeln (I) und (*ent*-I) bzw. -bei Verwendung der oben definierten Mischungen- an Verbindungen der Formeln (I), (*ent-*I)*,* (II), (*ent*-II), (III), (*ent*-III), (IV), (*ent-*IV)
- 5 bis 30 Gew.-%, bevorzugt 8 bis 25 Gew.-% Wasser,
- 50 bis 90 Gew.-%, bevorzugt 60 bis 80 Gew.-% einer Ölphase,
- 0,1 bis 5 Gew.-% eines verzehrbaren W/O-Emulgators
- bezogen auf die Gesamtmasse der Zusammensetzung
- sowie gegebenenfalls ein oder mehrere Antioxidantien und gegebenenfalls einen oder mehrere Stoffe zur Verstärkung einer antioxidativen Wirkung Besonders bevorzugt besteht eine solche erfindungsgemäße W/O-Emulsion aus den genannten Bestandteilen in den genannten Mengen.

Die Ölphase einer solchen erfindungsgemäßen W/O-Emulssion umfasst (oder besteht aus) vorzugsweise ein fettes Öl und/oder eine Aromakomposition. Bevorzugt sind Ölphasen umfassend oder bestehend aus einem fetten Öl und einer Aromakomposition.

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Vorzugsweise wird der verzehrbare W/O-Emulgator gewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e), Sorbitanmonostearat (E 491).

Geeignete Antioxidantien und Stoffe, welche die antioxidative Wirkung verstärken können sind die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

Die erfindungsgemäßen W/O-Emulsionen eignen sich besonders zum Aufbringen auf Lebensmitteloberflächen, wobei die Lebensmittel vorzugsweise einen Wassergehalt von höchstens 10 Gew.%, bevorzugt von höchstens 5 Gew.-% aufweisen. In einer bevorzugten Ausführungsform weist die erfindungsgemäße W/O - Emulsion bei Aufbringungstemperatur eine ausreichend niedrige Viskosität auf, so dass ein Aufbringen der W/O - Emulsion mittels Sprühen möglich ist. Bevorzugte Lebensmittel, auf deren Oberflächen eine erfindungsgemäße W/O - Emulsion aufgebracht werden kann sind beispielsweise Cracker, Chips (z.B. auf Basis von Kartoffeln, Mais, Getreide oder Brot), extrudierte Knabberartikel (Snacks, z.B. Flips) oder Laugengebäck (z.B. Salzstangen). Erfindungsgemäße W/O - Emulsionen werden regelmäßig in einer Menge von 0,5 bis 6 Gew.-% auf die Lebensmitteloberflächen aufgebracht, bezogen auf das Gesamtgewicht des Lebensmittels.

Wie bereits erwähnt, betrifft ein Aspekt der vorliegenden Erfindung die Verwendung einer Verbindung der oben definierten Formeln (I) und (*ent-*I)*,* insbesondere der oben als bevorzugt angegebenen Verbindungen (1) bis (6), bzw. der oben definierten Mischungen umfassend (a) eine oder mehrere Verbindungen der Formeln (I) und (*ent*-I) sowie (b) eine oder mehrere Verbindungen der Formeln (II), (*ent-*II), (III), (*ent*-III), (IV), (*ent*-IV) zum Erzeugen, Vermitteln, Modifizieren oder Verstärken eines Umami-Geschmacks.

Vorzugsweise werden die Verbindungen der Formeln (I) und (*ent*-I) (in geschmacklich wirksamer Menge), die oben definierten Mischungen umfassend (a) eine oder mehrere Verbindungen der Formeln (I) und (*ent*-I) sowie (b) eine oder mehrere Verbindungen der Formeln (II), (*ent-*II), (III), (*ent*-III), (IV), (*ent*-IV) oder die oben definierten erfindungsgemäßen Zusammensetzungen in der Ernährung oder dem Genuss dienenden (i) gebrauchs- oder verzehrfertigen Zubereitungen oder (ii) Halbfertigwaren eingesetzt, insbesondere in der Ernährung oder dem Genuss dienenden Natriumglutamat-reduzierten oder-freien Zubereitungen. Dabei gilt hinsichtlich bevorzugter Verbindungen und Mischungen das oben Gesagte entsprechend.

Die Erfindung betrifft auch eine verzehrbare Zubereitung oder Halbfertigware, umfassend eine geschmacklich wirksame Menge einer erfindungsgemäßen Verbindung oder Mischung oder einer erfindungsgemäßen Zusammensetzung,
wobei die verzehrbare Zubereitung oder Halbfertigware keine Umkristallisationsmischung ist von einer Verbindung der Formel (I), in welcher der aromatische Rest Ar ausgewählt ist aus der Gruppe bestehend aus 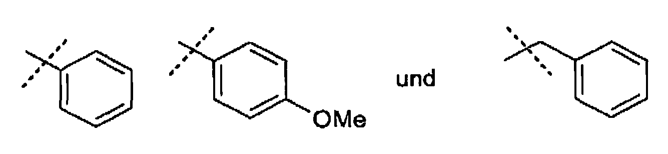 und wässrigem Alkohol als Lösungsmittel zur Umkristallisation.

Der Begriff "Natriumglutamat-reduziert" bedeutet dabei, dass die erfindungsgemäße Zubereitung oder Halbfertigware deutlich weniger Natriumglutamat enthält, als in der üblichen Zubereitung oder Halbfertigware enthalten ist; der Natriumglutamatgehalt liegt dabei um 5 bis <100 Gew.-%, bevorzugt 10 bis 50 Gew.-%, besonders bevorzugt 20 bis 50 Gew.-% unter dem Natriumglutamatgehalt der üblichen Zubereitung. Sofern neben einer oder mehreren Verbindungen der Formeln (I) und (*ent*-I) bzw. einer oben definierten Mischung umfassend (a) eine oder mehrere Verbindungen der Formeln (I) und (*ent*-I) sowie (b) eine oder mehrere Verbindungen der Formeln (II), (*ent*-II), (III), (*ent*-III), (IV), (*ent*-IV) auch Natriumglutamat in einer erfindungsgemäßen Zubereitung oder Halbfertigware vorliegt, liegt das Gewichtsverhältnis der Gesamtmenge an Verbindungen der Formeln (I) und (*ent*-I) bzw. oben definierten Mischungen zu Natriumglutamat vorzugsweise im Bereich von 1 : 1 bis 1 : 200.

Erfindungsgemäße der Ernährung oder dem Genuss dienende gebrauchs- oder verzehrfertige Zubereitungen enthalten eine oder mehrere erfingdungsgemäß zu verwendende Verbindungen der Formeln (I) und (*ent*-I) bzw. -bei Verwendung der oben definierten Mischungen- Verbindungen der Formeln (I), (*ent*-I), (II), (*ent-*II), (III), (*ent*-III)*,* (IV), (*ent*-IV) vorzugsweise in einer Menge im Bereich von 0,01 ppm bis 100 ppm, bevorzugt im Bereich von 0,1 ppm bis 50 ppm, insbesondere bevorzugt im Bereich von 0,5 ppm (vorzugsweise von 1 ppm) bis 30 ppm bezogen auf das Gesamtgewicht der gebrauchs- oder verzehrfertigen Zubereitung.

Erfindungsgemäße Halbfertigwaren zur Herstellung von der Ernährung oder dem Genuss dienenden gebrauchs- oder verzehrfertigen Zubereitungen enthalten eine oder mehrere erfindungsgemäß zu verwendende Verbindungen der Formeln (I) und (*ent*-I) bzw. -bei Verwendung der oben definierten Mischungen-Verbindungen der Formeln (I), (*ent*-I)*,* (II), (*ent*-II)*,* (III), (*ent*-III), (IV), (*ent*-IV), vorzugsweise in einer Menge im Bereich von 10 ppm bis 100000 ppm (vorzugsweise bis 800 ppm), bevorzugt im Bereich von 25 ppm bis 5000 ppm (vorzugsweise bis 750 ppm), insbesondere bevorzugt im Bereich von 50 ppm bis 1200 ppm (vorzugsweise bis 700 ppm), bezogen auf das Gesamtgewicht der Halbfertigware.

Besonders relevant sind erfindungsgemäße Natriumglutamat-reduzierte Zubereitungen, die Natriumglutamat umfassen, wobei die Menge des Natriumglutamats nicht ausreicht, um in einer Vergleichszubereitung, die keine erfindungsgemäße Mischung umfasst, aber ansonsten identisch zusammengesetzt ist (normale Natriumglutamat-reduzierte Zubereitung), als befriedigender Umami-Geschmack wahrgenommen zu werden, und die Menge der erfindungsgemäßen Mischung ausreicht, um einen befriedigenden Umami-Geschmackseindruck zu erreichen.

Der Ernährung oder dem Genuss dienende Zubereitungen im Sinne der Erfindung sind insbesondere Backwaren (z.B. Brot, Trockenkekse, Kuchen, sonstiges Gebäck), Getränke (z.B. Gemüsesäfte, Gemüsesaftzubereitungen), Instantgetränke (z.B. Instant-Gemüsegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. vorgegarte Fertigreis-Produkte, Reismehlprodukte, Hirse-und Sorghum-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Gemüsezubereitungen (z.B. Ketchup, Soßen, Trockengemüse, Tiefkühlgemüse, vorgegarte Gemüse, in Essig eingelegte Gemüse, Gemüsekonzentrate oder - pasten, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais-, Reis- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Soßen (Instant-Soßen, Trockensoßen, Fertigsoßen), Gewürze oder Gewürzzubereitungen (z.B. Senfzubereitungen, Meerrettichzubereitungen), Würzmischungen sowie insbesondere Aufstreuwürzungen (englisch: Seasonings), die beispielsweise im Snackbereich Anwendung finden.

Besonders bevorzugt sind Halbfertigwaren oder der Ernährung oder dem Genuss dienende Zubereitungen (vorzugsweise mit reduziertem Gehalt an NatriumGlutamat), z.B. Backwaren (z.B. Brot, Trockenkekse, Kuchen, sonstiges Gebäck), Gemüsesaftzubereitungen, Fleischprodukte (z.B. Schinken, Frischwurst-oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. vorgegarte Fertigreis-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fischsoßen wie z.B. Anchovis-Soßen, Austernsoßen, Gemüsezubereitungen (z.B. Ketchup, Soßen, Trockengemüse, Tiefkühlgemüse, vorgegarte Gemüse, in Essig eingelegte Gemüse, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), Fertiggerichte, Suppen (z.B. Trockensuppen, Instant-Suppen, vorgegarte Suppen), Brühwürfel, Soßen (Instant-Soßen, Trockensoßen, Fertigsoßen), Gewürze, Würze, Würzmittel, Würzmischungen sowie insbesondere Aufstreuwürzungen (englisch: Seasonings), die beispielsweise im Snackbereich Anwendung finden.

Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die erfindungsgemäßen Halbfertigwaren dienen in der Regel zur Herstellung von der Ernährung oder dem Genuss dienenden gebrauchs- oder verzehrfertigen Zubereitungen.

Insbesondere können erfindungsgemäße Halbfertigwaren zur additiven Verstärkung des Umami-Geschmacks von Natriumglutamat-reduzierten Nahrungs-und Genussmitteln und auch direkt als Würzmittel für die industrielle oder nichtindustrielle Zubereitung von Nahrungs- und/oder Genussmitteln dienen.

Erfindungsgemäße Halbfertigwaren enthalten bevorzugt:
- eine Gesamtmenge von 10 ppm bis 100000 ppm (vorzugsweise bis 800 ppm), bevorzugt 25 ppm bis 5000 ppm (vorzugsweise bis 750 ppm), insbesondere 50 ppm bis 1200 ppm (vorzugsweise bis 700 ppm), an Verbindungen der Formeln (I) und (*ent*-I) bzw. -bei Verwendung der oben definierten Mischungen- an Verbindungen der Formeln (I), (*ent*-I)*,* (II), (*ent*-II), (III), (*ent-*III), (IV), (*ent*-IV),
- kein Natriumglutamat oder einen Anteil von 0,00001 bis 10 Gew.-%, bevorzugt 0,0001 bis 5 Gew.-%, insbesondere 0,001 Gew.-% bis 2 Gew.-% an Natriumglutamat,
- und gegebenenfalls einen Anteil von 0,0001 Gew.-% bis 90 Gew.-%, bevorzugt 0,001 Gew.-% bis 30 Gew.-% einer Aromakomposition, jeweils bezogen auf das Gesamtgewicht der Halbfertigware.

Die erfindungsgemäßen Zusammensetzungen, Zubereitungen bzw. Halbfertigwaren werden vorzugsweise hergestellt, indem die Verbindungen der Formeln (I) und (*ent-*I) oder oben definierten Mischungen in Mischungen aus Ethanol und gegebenenfalls demineralisiertem und/oder gereinigtem Wasser gelöst und gemischt werden; anschließend werden die Lösungen durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Zubereitung überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel), erfolgen.

Vorzugsweise erfolgt die Trocknung mittels Sprühtrocknung oder Vakuumgefriertrocknung.

Bevorzugte erfindungsgemäße Zusammensetzungen, Zubereitungen bzw. Halbfertigwaren sind Würze, Würzmischungen, Würzmittel, Brühwürfel, Instant-Suppen, Instant-Soßen, vegetarische Fertiggerichte, fleischhaltige Fertiggerichte, Fischsoßen wie z.B. Anchovis-Soßen, Austernsoßen und Sojasoßen.

Gemäß einer weiteren bevorzugten Ausführungsform werden zur Herstellung erfindungsgemäßer Zusammensetzungen, Zubereitungen bzw. Halbfertigwaren Verbindungen der Formeln (I) und (*ent-*I) bzw. oben definierte Mischungen sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

In einem weiteren bevorzugten Herstellungsverfahren werden Verbindungen der Formeln (I) und (*ent-*I) bzw. oben definierten Mischungen mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt sind erfindungsgemäße Zubereitungen, bei denen die Matrix so gewählt ist, dass die Verbindungen der Formeln (I) und (*ent*-I) bzw. oben definierten Mischungen verzögert von der Matrix freigegeben werden, so dass man eine langanhaltende Wirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

Weitere Bestandteile einer erfindungsgemäßen, der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zusammensetzungen, Zubereitungen oder Halbfertigwaren eine Aromakomposition, um den Geschmack und/oder den Geruch abzurunden und zu verfeinern. Eine erfindungsgemäße Zusammensetzung, die als weitere Bestandteile einen festen Trägerstoff und eine Aromakomposition umfasst, wurde bereits weiter oben beschrieben. Geeignete Aromakompositionen enthalten z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die nicht-erfindungsgemäßen Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

In einer weiteren Ausgestaltung der vorliegenden Erfindung werden Verbindungen der Formeln (I) und (*ent*-I) bzw. oben definierte Mischungen in Zusammensetzungen, Zubereitungen und Halbfertigwaren in Kombination mit zumindest einer (weiteren, nicht für sich erfindungsgemäßen) Substanz zum Maskieren oder Vermindern eines unangenehmen (bitteren, metallischen, kalkigen, sauren, adstringierenden) Geschmackseindrucks oder zur Verstärkung oder Erzeugung eines angenehmen Geschmackseindrucks (süß, salzig, Umami) verwendet.

Auf diese Weise kann eine Verstärkung des Geschmacks, insbesondere des Umami-Geschmacks, erreicht werden. Diese weiteren Substanzen können aus der folgenden Liste ausgewählt werden, ohne die Erfindung damit einzuschränken: Mononatriumglutamat, Glutaminsäure, Nucleotide (z.B. Adenosin-5'-monophosphat, Cytidin-5'-monophosphat, Inosin-5'-monophosphat, Guanosin-5'-monophosphat) oder deren pharmazeutisch akzeptable Salze, Lactisole, Hydroxyflavanone (z.B. Eriodictyol, Homoeriodictyol oder deren Natriumsalze), insbesondere gemäß EP 1 258 200, Hydroxybenzoesäureamide (z. B. 2,4-Dihydroxybenzoesäurevanillylamid, 4-Hydroxybenzoesäurevanillylamid), Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen.

Modulierende Aroma- und/oder Geschmackstoffe werden vorzugsweise ausgewählt aus der Gruppe bestehend aus Adenosin-5'-monophosphat, Cytidin-5'-monophosphat, Inosin-5'-monophosphat, und deren pharmazeutisch akzeptablen Salzen; Lactisolen; 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxyflavanonen, wie zum Beispiel Eriodictyol, Homoeriodictyol, und deren Natriumsalzen; Hydroxybenzoesäureamiden, wie zum Beispiel 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-N-(4-hydroxy-3-methoxybenzyl)-amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethyl-amid, 2,4-Dihydroxybenzoesäure-N-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird) und Divanillinen (insbesondere Divanillin wie beschrieben in WO 2004/078302, die bezüglich der darin offenbarten entsprechenden Verbindungen auf dem Wege der Verweisung Bestandteil dieser Anmeldung wird); Bicyclo[4.1.0]heptan-7-carbonsäureamide, insbesondere solche wie beschrieben in PCT/EP2007/061171 sowie den darauf basierenden Dokumenten (Symrise), die bezüglich der darin offenbarten entsprechenden Verbindungen auf dem Wege der Verweisung Bestandteil dieser Anmeldung wird; Cyclopropancarbonsäure(3-methyl-cyclohexyl)amide, insbesondere solche wie beschrieben in US-Provisional 60/916,589 vom 08.05.2007 sowie den darauf basierenden Dokumenten (Symrise), die bezüglich der darin offenbarten entsprechenden Verbindungen auf dem Wege der Verweisung Bestandteil dieser Anmeldung wird.

Gemäß einer weiteren Ausgestaltung der vorliegenden Erfindung umfasst eine jeweils wie oben beschriebene erfindungsgemäße Zusammensetzung, gebrauchs- oder verzehrfertige Zubereitung oder Halbfertigware insbesondere zusätzlich einen oder mehrere süßverstärkende Stoffe. Insbesondere werden die erfindungsgemäßen Verbindungen der Formeln (I) oder (II) bzw. die erfindungsgemäßen Mischungen (wie oben beschrieben) hierbei in Kombination mit zumindest einem süßverstärkenden Stoff eingesetzt, insbesondere mit einer oder mehreren Verbindungen gemäß WO 2007/014879 A1 oder WO 2007/107596 A1, speziell zusammen mit Hesperetin und/oder Phloretin. Hierdurch wird vorteilhafterweise eine Verstärkung und eine Vertiefung sowie Abrundung des Geschmacksprofils, insbesondere des würzigen und/oder salzigen Geschmacks der Zusammensetzung, Zubereitung oder Halbfertigware erreicht. Für Halbfertigwaren liegt der Gesamtanteil an Hesperetin und/oder Phloretin hierbei vorzugsweise im Bereich von 10 bis 100000 ppm, bezogen auf das Gesamtgewicht der Halbfertigware, während im verzehrfertigen Lebensmittel der Gesamtanteil an Hesperetin und/oder Phloretin bezogen auf das Gesamtgewicht des Lebensmittels vorzugsweise im Bereich von 1 bis 400 ppm, bevorzugt im Bereich von 5 bis 200 ppm liegt.

Vorzugsweise sind in den erfindungsgemäßen Zusammensetzungen, Zubereitungen bzw. Halbfertigwaren zusätzlich ein oder mehrere süßverstärkende Stoffe und ein oder mehrere weitere Geschmacksstoffe, die einen trigeminalen Reiz (tingling, kribbeln, scharf, kühlend etc.) bewirken, enthalten. Insbesondere bei der Kombination der erfindungsgemäßen Verbindungen der Formeln (I) oder (II) bzw. der entsprechenden erfindungsgemäßen Mischungen mit Hesperetin und/oder Phloretin aber auch mit cis- und/oder trans-Pellitorin (siehe WO 2004/000787 bzw. WO 2004/043906) wird ein weiter verbessertes und vom Konsumenten bevorzugtes Geschmacksprofil erreicht. Dabei liegt der Gesamtanteil an cis- und/oder trans-Pellitorin in diesen Zusammensetzungen bzw. Zubereitungen oder Halbfertigwaren vorzugsweise im Bereich von 0,1 bis 500 ppm, bevorzugt im Bereich von 5 bis 100 ppm, bezogen auf das Gesamtgewicht der Zusammensetzung, Zubereitung oder Halbfertigware.

Aus dem vorstehenden Text ergibt sich, dass ein weiterer Aspekt der vorliegenden Erfindung auch ein Verfahren zum Erzeugen, Vermitteln, Modifizieren oder Verstärken eines Geschmacks, insbesondere eines Umami-Geschmacks, in einer der Ernährung, der Mundpflege oder dem Genuss dienenden (i) gebrauchs-oder verzehrfertigen Zubereitung oder (ii) Halbfertigware betrifft. Ein solches erfindungsgemäßes Verfahren umfasst den folgenden Schritt:
Vermischen einer geschmacklich wirksamen Menge einer oder mehrerer Verbindungen der Formeln (I) und (*ent-*I) oder oben definierter Mischungen oder einer erfindungsgemäßen Zusammensetzung mit einem oder mehreren weiteren Bestandteilen der (i) verzehr- oder gebrauchsfertigen Zubereitung oder der (ii) Halbfertigware, oder
Applizieren einer geschmacklich wirksamen Menge einer oder mehrerer Verbindungen der Formeln (I) und (*ent*-I) oder oben definierter Mischungen oder einer erfindungsgemäßen Zusammensetzung auf einen oder mehrere weitere Bestandteile der (i) verzehr- oder gebrauchsfertigen Zubereitung oder der (ii) Halbfertigware, oder Einbetten einer geschmacklich wirksamen Menge einer oder mehrerer Verbindungen der Formeln (I) und (*ent*-I) oder oben definierter Mischungen oder einer erfindungsgemäßen Zusammensetzung in ein Hüll- oder Matrixmaterial.
Dabei gilt hinsichtlich bevorzugter Verbindungen und Mischungen das oben Gesagte entsprechend.

### Beispiele:

Die nachfolgenden Beispiele erläutern die Erfindung. Sofern nicht anders angegeben, beziehen sich alle Angaben auf das Gewicht.

Die als Ausgangsmaterial benötigten Neo-Menthylamide werden nach einer Vorschrift von Wallach et al. (Ann. Chem. 1893, 276, 296-313) aus den entsprechenden Menthonen in einer Reinheit ≥ 90%, bevorzugt ≥ 95%, dargestellt. Ein Gemisch aller möglichen isomeren Menthylamine kann nach oben genannter Vorschrift ohne entsprechende Kristallisation der Menthylformamide (ein Zwischenprodukt) in einer Reinheit von 99.3% erhalten werden (24.1% Menthylamin, 55.5% Neo-Menthylamin, 2.4% *Iso*-Menthylamin, 17.3% *Neo-Iso*-Menthylamin). Dabei können sowohl enantiomerenreine D- bzw. L-Menthone als auch eine racemische D/L-Menthonmischung verwandt werden. Alle eingesetzten Menthone können mit bis zu 25% der entsprechenden *Iso-*Menthone vermischt sein.

Die Darstellung einzelner enantiomerenreiner Menthylamine gelingt durch Umsetzung des entsprechenden Menthols zum Azid (Synthesis 1999, 8, 1373) und anschießender Reduktion mit LiAlH₄ (J. Am. Chem. Soc. 1962, 2925).

**Allgemeine Arbeitsvorschrift (AAV):** Umsetzung mit Säurechloriden

Zu der Lösung eines entsprechenden Amins sowie 1.5 - 2.5 Äquivalenten Triethylamin in Dichlormethan werden unter Kühlung langsam 1.1 - 1.5 Äquivalente des entsprechenden Säurechlorides zugetropft. Die Mischung wird auf RT (ca. 20°C) erwärmt und für 12 h gerührt. Anschließend wird mit Dichlormethan verdünnt und mit Wasser gewaschen. Nach Trocknen über Natriumsulfat und anschließender Entfernung des Lösungsmittels erfolgt die Aufreinigung durch Umkristallisation aus Hexan/*iso*-Propanol.

**Synthesebeispiel** 1: *N*-((1*S*,2*S*,5*R*)-2-Isopropyl-5-methyl-cyclohexyl)-terephthal-säuremethylester

Aus Neo-L-Menthylamin wird analog der **AAV** durch Umsetzung mit 4-Chlorocarbonylbenzosäuremethylester das genannte Produkt als farbloser Feststoff erhalten. Analysendaten: **¹H-NMR (400 MHz, CDCl₃):** 0.90 (d, 3H, J = 6.5 Hz); 0.93 (d, 3H, J = 6.6 Hz); 0.95 (d, 3H, J = 6.6 Hz); 0.98 - 1.21 (m, 4H); 1.40 (m, 1 H); 1.53 (m, 1 H); 1.80 (m, 1H); 1.95 (m, 1H); 2.01 (m, 1H); 3.98 (s, 3H); 4.58 (m, 1H,); 6.19 (bd, 1H, J = 8.9 Hz); 7.80 (m, 2H); 8.10 (m, 2H) ppm. **¹³C-NMR (100 MHz,** CDCl₃): 20.8 (CH₃); 21.1 (CH₃); 22.2 (CH₃); 25.6 (CH₂); 27.1 (CH); 29.9 (CH); 34.7 (CH₂); 40.0 (CH₂); 46.4 (CH); 46.8 (CH); 52.4 (CH₃); 126.9 (CH); 129.9 (CH); 132.5 (C); 139.2 (C); 165.9 (C=O); 166.3 (C=O) ppm. **Massenspektrum (EI):** m/z (%) = 317 (M^{•+}, 11); 286 (7); 274 (19); 232 (21); 193 (6); 192 (6); 181 (10); 180 (92); 164 (11); 163 (100); 138 (12); 135 (17); 123 (7); 120 (5); 104 (11); 103 (12); 96 (5); 95 (21); 82 (7); 81 (12) 77 (7); 76 (6); 67 (5); 55 (7); 43 (5); 41 (8). **Synthesebeispiel 2:** *N*-((1*R*,2*S*,5*R*)-2-Isopropyl-5-methyl-cyclohexyl)-terephthal-säuremethylester (nicht erfindungsgemäß)

Aus einer oben beschriebenen Mischung aller möglichen isomeren Menthylamine wird analog der **AAV** durch Umsetzung mit 4-Chlorocarbonylbenzosäure-methylester und anschließender Auftrennung der Mischung durch HPLC das genannte Produkt als farbloser Feststoff erhalten. Analysendaten: **¹H-NMR (400 MHz, CDCl₃)**: 0.85 (d, 3H, J = 6.9 Hz); 0.91 (d, 3H, J = 6.4 Hz); 0.93 (d, 3H, J = 6.9 Hz); 0.85 - 0.95 (m, 2H); 1.11 - 1.24 (m, 2H); 1.55 (m, 1 H); 1.70 - 1.80 (m, 2H); 1.96 (m, 1 H); 2.09 (m, 1 H); 3.94 (s, 3H); 4.01 (m, 1 H,); 5.81 (bd, 1 H, J = 9.3 Hz); 7.80 (m, 2H); 8.09 (m, 2H) ppm. **¹³C-NMR (100 MHz, CDCl₃):** 16.3 (CH₃); 21.2 (CH₃); 22.1 (CH₃); 23.9 (CH₂); 27.1 (CH); 31.9 (CH); 34.5 (CH₂); 43.1 (CH₂); 48.4 (CH); 50.7 (CH); 52.4 (CH₃); 126.9 (CH); 129.8 (CH); 132.5 (C); 139.0 (C); 165.8 (C=O); 166.4 (C=O) ppm. **Massenspektrum (EI):** m/z (%) = 317 (M^{•+}, 7); 286 (7); 274 (13); 232 (18); 193 (5); 192 (5); 181 (11); 180 (100); 164 (10); 163 (94); 138 (17); 135 (17); 123 (8); 120 (5); 104 (11); 103 (12); 96 (5); 95 (22); 82 (8); 81 (13) 77 (6); 76 (6); 67 (5); 55 (7); 43 (5); 41 (8). **Synthesebeispiel 3**: Benzo[1,3]dioxoie-5-carbonsäure((1*S*,2*S*,5*R*)-2-isopropyl-5-methyl-cyclohexyl)amid (3) / Benzo[1,3]dioxole-5-carbonsäure((1*R*,2*R*,5*S*)-2-isopropyl-5-methyl-cyclohexyl)amid (4)

Aus racemischem Neo-Menthylamin wird analog der **AAV** durch Umsetzung mit Benzo[1,3]dioxole-5-carbonylchlorid das genannte Produkt als farbloser Feststoff der insgesamt 95.7% der gewünschten Neo-Menthylaminderivate (3) und (4) erhält. Des Weiteren sind zwei weitere isomere Menthylaminderivate mit jeweils 2.1% vorhanden, was einer Reinheit an isomeren Benzo[1,3]dioxole-5-carbonsäure(-2-isopropyl-5-methyl-cyclohexyl)amiden von 99.9% entspricht.

Analysendaten (Hauptkomponente): **¹H-NMR (400 MHz, CDCl₃):** 0.89 (d, 3H, J = 6.4 Hz); 0.92 (d, 3H, J = 6.7 Hz); 0.94 (d, 3H, J = 6.9 Hz); 0.96 - 1.18 (m, 4H); 1.39 (m, 1 H); 1.49 (m, 1 H); 1.78 (m, 1 H); 1.92 (m, 1 H); 1.99 (m, 1 H); 4.52 (m, 1 H); 6.02 (s, 2H); 6.05 (m, 1 H); 6.82 (m, 1 H); 7.26 (m, 2H) ppm. **¹³C-NMR (100 MHz, CDCl₃):** 20.9 (CH₃); 21.0 (CH₃); 22.3 (CH₃); 25.7 (CH₂); 27.1 (CH); 29.9 (CH); 34.8 (CH₂); 40.1 (CH₂); 46.4 (CH); 46.6 (CH); 101.7 (CH₂); 107.6 (CH); 108.0 (CH); 121.2 (CH); 129.6 (C); 148.0 (C); 150.2 (C); 166.0 (C=O) ppm. **Massenspektrum (EI):** m/z (%) = 303 (M^{•+}, 9); 260 (5); 166 (41); 165 (27); 150 (8); 149 (100); 121 (13); 65 (7). **Synthesebeispiel 4:** 2-Benzo[1,3]dioxol-5-yl-*N*-((1S,2S,5R)-2-isopropyl-5-methyl-cyclohexyl)acetamid (5) / 2-Benzo[1,3]dioxol-5-yl-*N*-((1*R*,2*R*,5*S*)-2-isopropyl-5-methyl-cyclohexyl)acetamid (6)

Aus racemischem Neo-Menthylamin wird analog der AAV durch Umsetzung mit Benzo[1,3]dioxol-5-yl-acetylchlorid das genannte Produkt als farbloser Feststoff der insgesamt 92.9% der gewünschten Neo-Menthylaminderivate (5) und (6) erhält. Des Weiteren sind zwei weitere isomere Menthylaminderivate mit 3.0% und 3.3% vorhanden, was einer Reinheit an isomeren 2-Benzo[1,3]dioxol-5-yl-*N-*(2-isopropyl-5-methyl-cyclohexyl)acetamiden von 99.2% entspricht.

Analysendaten (Hauptkomponente): **¹H-NMR (400 MHz, CDCl₃):** 0.57 (dq, 1 H, J = 13.0 und 3.5 Hz); 0.81 - 0.87 (m, 1 H); 0.82 (d, 3H, J = 6.5 Hz); 0.83 (d, 3H, J = 6.4 Hz); 0.84 (d, 3H, J = 6.5 Hz); 0.93 - 1.22 (m, 4H); 1.63 (m, 1 H); 1.72 (m, 1 H); 1.83 (m, 1 H); 3.49 (s, 2H); 4.27 (m, 1 H); 5.47 (bd, 1 H, J = 8.5 Hz); 5.97 (d, 1 H, J = 1.4 Hz); 5.98 (d, 1 H, J = 1.4 Hz); 6.69 (m, 2H); 6.81 (d, 1 H, J = 8.0 Hz) ppm. **¹³C-NMR (100 MHz, CDCl₃):** 20.7 (CH₃); 21.0 (CH₃); 22.2 (CH₃); 25.4 (CH₂); 26.9 (CH); 29.7 (CH); 34.6 (CH₂); 39.9 (CH₂); 43.8 (CH₂); 46.1 (CH); 46.2 (CH); 101.2 (CH₂); 108.7 (CH); 109.7 (CH); 122.5 (CH); 128.9 (C); 146.9 (C); 148.2 (C); 170.2 (C=O) ppm. **Massenspektrum (EI):** m/z (%) = 318 (6); 317 (M^{•+}, 27); 182 (9); 180 (21); 179 (24); 137 (10); 136 (100); 135 (53); 112 (5); 97 (7); 95 (7); 83 (37); 81 (7); 79 (5); 77 (14); 70 (15); 69 (18); 67 (5); 57 (11); 55 (21); 51 (9); 43 (11); 41 (14). **Synthesebeispiel 5:** *N*-((1*S*,2*S*,5*R*)-2-Isopropyl-5-methyl-cyclohexyl)-benzamid /*N*-((1*R*,2*R*,5*S*)-2-Isopropyl-5-methyl-cyclohexyl)-benzamid

Aus racemischem Neo-Menthylamin wird analog der **AAV** durch Umsetzung mit Benzoylchlorid das genannte Produkt als farbloser Feststoff erhalten, der insgesamt 97.6% der gewünschten Neo-Menthylaminderivate erhält. Des Weiteren sind zwei weitere isomere Menthylaminderivate mit 1.3% und 1.0% vorhanden, was einer Reinheit an isomeren *N*-(-2-Isopropyl-5-methyl-cyclohexyl)-benzamiden von 99.9% entspricht.

Analysendaten: **¹H-NMR (400 MHz, CDCl₃):** 0.89 (d, 3H, J = 6.5 Hz); 0.92 (d, 3H, J = 6.5 Hz); 0.95 (d, 3H, J = 6.6 Hz); 0.98 - 1.19 (m, 4H); 1.41 (m, 1 H); 1.50 (m, 1 H); 1.79 (m, 1 H); 1.93 (m, 1 H); 2.01 (m, 1 H); 4.57 (m, 1 H); 6.17 (bd, 1 H, J = 8.3 Hz); 7.44 (m, 2H); 7.50 (m, 1 H); 7.75 (m, 2H) ppm. **¹³C-NMR (100 MHz,** CDCl₃): 20.9 (CH₃); 21.1 (CH₃); 22.3 (CH₃); 25.7 (CH₂); 27.1 (CH); 29.9 (CH); 34.8 (CH₂); 40.1 (CH₂); 46.45 (CH); 46.55 (CH); 126.8 (CH); 128.6 (CH); 131.2 (CH); 135.3 (C); 166.7 (C=O) ppm. **Massenspektrum (EI):** m/z (%) = 259 (M^{•+}, 12); 216 (10); 174 (15); 135 (5); 122 (59); 106 (7); 105 (100); 95 (9); 77 (30); 41 (5). **Synthesebeispiel 6:** Furan-2-carbonsäure((1S,2S,5R)-2-isopropyl-5-methylcyclohexyl)amid / Furan-2-carbonsäure((1R,2R,5S)-2-isopropyl-5-methylcyclohexyl)amid

Aus racemischem Neo-Menthylamin wird analog der **AAV** durch Umsetzung mit Furan-2-carbonylchlorid das genannte Produkt als farbloser Feststoff erhalten, der insgesamt 94.7% der gewünschten Neo-Menthylaminderivate erhält. Des Weiteren sind zwei weitere isomere Menthylaminderivate mit 3.8% und 1.3% vorhanden, was einer Reinheit über alle Stereoisomere von 99.8% entspricht.

Analysendaten: **¹H-NMR (400 MHz, CDCl₃):** 0.89 (d, 3H, J = 6.4 Hz); 0.91 (d, 3H, J = 6.6 Hz); 0.93 (d, 3H, J = 6.5 Hz); 0.95 - 1.18 (m, 4H); 1.39 (m, 1 H); 1.52 (m, 1 H); 1.80 (m, 1 H); 1.88 - 1.99 (m, 2H); 4.53 (m, 1 H); 6.43 (bd, 1 H, J = 8.7 Hz); 6.50 (dd, 1 H, J = 3.4 und 1.8 Hz); 7.10 (dd, 1 H, J = 3.4 und 0.9 Hz); 7.43 (dd, 1 H, J = 1.8 und 0.9 Hz) ppm. **¹³C-NMR (100 MHz, CDCl₃):** 20.9 (CH₃); 21.0 (CH₃); 22.2 (CH₃); 25.5 (CH₂); 27.0 (CH); 29.6 (CH); 34.8 (CH₂); 40.2 (CH₂); 45.8 (CH); 46.4 (CH); 112.2 (CH); 113.8 (CH); 143.6 (CH); 148.4 (C); 157.7 (C=O) ppm. **Massenspektrum (EI)**: m/z (%) = 250 (5); 249 (M^{•+}, 24); 243 (15); 206 (15); 164 (35); 138 (12); 125 (12); 124 (9); 123 (7); 113 (5); 112 (79); 96 (18); 95 (100); 82 (8); 81 (12); 69 (7); 67 (9); 55 (9); 43 (7); 41 (13); 39 (10). **Synthesebeispiel 7:** *N*-((1*S*,2*S*,5*R*)-2-Isopropyl-5-methyl-cyclohexyl)-3,4-di-methoxy-benzamid / *N*-((1*R*,2*R*,5*S*)-2-Isopropyl-5-methyl-cyclohexyl)-3,4-di-methoxy-benzamid Aus racemischem Neo-Menthylamin wird analog der **AAV** durch Umsetzung mit 3,4-Dimethoxybenzoylchlorid das genannte Produkt als farbloser Feststoff erhalten, der insgesamt 97.8% der gewünschten Neo-Menthylaminderivate erhält. Des Weiteren sind zwei weitere isomere Menthylaminderivate mit 1.3% und 0.9% vorhanden, was einer Reinheit über alle Stereoisomere von 100.0% entspricht. Analysendaten: **¹H-NMR (400 MHz, CDCl₃):** 0.89 (d, 3H, J = 6.5 Hz); 0.93 (d, 3H, J = 6.6 Hz); 0.95 (d, 3H, J = 6.6 Hz); 0.97 - 1.19 (m, 4H); 1.41 (m, 1 H); 1.50 (m, 1 H); 1.79 (m, 1 H); 1.93 (m, 1 H); 2.00 (m, 1 H); 3.92 (s, 3H); 3.94 (s, 3H); 4.55 (m, 1 H); 6.13 (bd, 1 H, J = 8.8 Hz); 6.86 (d, 1 H, J = 8.4 Hz); 7.12 (dd, 1 H, J = 8.3 und 2.1 Hz); 7.46 (d, 1H, *J* = 2.1 Hz) ppm. **¹³C-NMR (100 MHz, CDCl₃):** 20.9 (CH₃); 21.1 (CH₃); 22.3 (CH₃); 25.7 (CH₂); 27.2 (CH); 29.9 (CH); 34.8 (CH₂); 40.1 (CH₂); 46.46 (CH); 46.51 (CH); 56.0 (CH₃); 56.1 (CH₃); 110.2 (CH); 110.9 (CH); 118.6 (CH); 128.0 (C); 149.2 (C); 151.6 (C); 166.2 (C=O) ppm. **Massenspektrum (EI):** m/z (%) = 319 (M^{•+}, 14); 276 (7); 182 (36); 181 (58); 166 (11); 165 (100); 139 (6); 137 (8); 79 (6); 77 (8); 41 (5). **Synthesebeispiel 8:** 2-(3,4-Dimethoxy-phenyl)-*N*-((1S,2S,5R)-2-isopropyl-5-methyl-cyclohexyl)-acetamid / 2-(3,4-Dimethoxy-phenyl)-*N*-((1*R*,2*R*,5*S*)-2-isopropyl-5-methyl-cyclohexyl)-acetamid

Aus racemischem Neo-Menthylamin wird analog der **AAV** durch Umsetzung mit (3,4-Dimethoxyphenyl)acetylchlorid das genannte Produkt als farbloser Feststoff erhalten, der insgesamt 94.2% der gewünschten Neo-Menthylaminderivate erhält. Des Weiteren sind zwei weitere isomere Menthylaminderivate mit 2.7% und 2.5% vorhanden, was einer Reinheit über alle Stereoisomere von 99.4% entspricht.

Analysendaten: **¹H-NMR (400 MHz, CDCl₃):** 0.49 (m, 1H); 0.75 - 0.89 (m, 1H); 0.81 (d, 3H, J = 6.4 Hz); 0.82 (d, 3H, J = 6.4 Hz); 0.85 (d, 3H, J = 6.5 Hz); 0.92 - 1.18 (m, 4H); 1.57 - 1.73 (m, 2H); 1.84 (m, 1 H); 3.53 (s, 2H); 3.86 (s, 3H); 3.90 (s, 3H); 4.27 (m, 1 H); 5.46 (bd, 1 H, J = 8.6 Hz; 6.76 (m, 1 H); 6.79 (m, 1 H); 6.87 (m, 1 H) ppm. **¹³C-NMR (100 MHz, CDCl₃):** 20.7 (CH₃); 21.0 (CH₃); 22.2 (CH₃); 25.4 (CH₂); 26.9 (CH); 29.7 (CH); 34.5 (CH₂); 40.0 (CH₂); 43.8 (CH₂); 46.08 (CH); 46.14 (CH); 55.8 (CH₃); 56.0 (CH₃); 111.5 (CH); 112.2 (CH); 121.6 (CH); 127.7 (C); 148.4 (C); 149.4 (C); 170.4 (C=O) ppm.

**Massenspektrum (EI):** m/z (%) = 334 (11); 333 (M^{•+}, 45); 196 (17); 195 (28); 182 (7); 178 (5); 153 (10); 152 (100); 151 (78); 137 (31); 112 (7); 107 (10); 97 (5); 95 (7); 91 (5); 83 (28); 81 (8); 70 (14); 69 (14); 57 (8); 55 (20); 43 (10); 41 (13).

**Synthesebeispiel 9:** *N*-((1*S*,2*S*,5*R*)-2-Isopropyl-5-methyl-cyclohexyl)-3,4,5-trimethoxy-benzamid / *N*-((1*R*,2*R*,5*S*)-2-Isopropyl-5-methyl-cyclohexyl)-3,4,5-trimethoxy-benzamid (nicht erfindungsgemäß)

Aus racemischem Neo-Menthylamin wird analog der **AAV** durch Umsetzung mit 3,4,5-Trimethoxy-benzoylchlorid das genannte Produkt als farbloser Feststoff erhalten, der insgesamt 92.2% der gewünschten Neo-Menthylaminderivate erhält. Des Weiteren sind zwei weitere isomere Menthylaminderivate mit 3.8% und 4.0% vorhanden, was einer Reinheit über alle Stereoisomere von 100.0% entspricht.

Analysendaten: **¹H-NMR (400 MHz, CDCl₃):** 0.90 (d, 3H, J = 6.4 Hz); 0.94 (d, 3H, J = 6.6 Hz); 0.96 (d, 3H, J = 6.6 Hz); 0.99 - 1.20 (m, 4H); 1.40 (m, 1 H); 1.49 (m, 1 H); 1.80 (m, 1 H); 1.94 (m, 1 H); 2.00 (m, 1 H); 3.88 (s, 3H); 3.92 (s, 6H); 4.55 (m, 1 H); 6.06 (bd, 1 H, J = 9.0 Hz); 6.97 (s, 2H) ppm. **¹³C-NMR (100 MHz, CDCl₃):** 20.9 (CH₃); 21.1 (CH₃); 22.3 (CH₃); 25.8 (CH₂); 27.2 (CH); 30.0 (CH); 34.7 (CH₂); 40.0 (CH₂); 46.4 (CH); 46.7 (CH); 56.5 (CH₃); 60.9 (CH₃); 104.5 (CH); 130.9 (C); 141.1 (C); 153.3 (C); 166.4 (C=O) ppm.

**Massenspektrum (EI)**: m/z (%) = 350 (6); 349 (M^{•+}, 26); 306 (6); 212 (35); 211 (76); 196 (23); 195 (100); 169 (6); 152 (5); 95 (5); 81 (7); 41 (5).

### Beispiel 1: Sprühgetrocknete Zusammensetzung zur Erzeugung eines Umami-Geschmacks

| **1.1** | **Bestandteil** | **Anteil** |
|---|---|---|
| | Verbindung der Formel (I) | 2 g |
| | Maltodextrin | 98 g |

| **1.2** | **Bestandteil** | **Anteil** |
|---|---|---|
| | 1 : 1 Mischung aus Benzo[1,3]dioxol-5-carbonsäure ((1*S*,2*S*,5*R*)- | 4 g |
| | 2-isopropyl-5-methyl-cyclohexyl)-amid (3) und Benzo[1,3]dioxol-5- | |
| | carbonsäure ((1*R*,2*R*,5*S*)-2-isopropyl-5-methyl-cyclohexyl)-amid | |
| | (4), Synthesebeispiel 3 | |
| | Maltodextrin | 96 g |

Die Bestandteile werden in einer Mischung aus Ethanol und demineralisiertem Wasser gelöst und anschließend sprühgetrocknet.

### Beispiel 2: Aromakomposition, nicht erfindungsgemäß

| **Inhaltsstoff** | **Anteil** |
|---|---|
| 10 Gew.-% Pellitorin in 1,2-Propylenglycol/Diethylmalonat | 0,25 g |
| Hesperetin | 2,50 g |
| Phloretin | 1,50 g |
| Propylenglycol | 95,75 g |

Die Aromakomposition wurde in nachfolgend beschriebenen Anwendungsbeispielen eingesetzt.

### Beispiel 3: Würzmittel, enthaltend Verbindung zur Erzeugung eines Umami-Geschmacks und eine Aromakomposition

| **Teil** | **Bestandteil** | **Anteil** |
|---|---|---|
| A | 1 : 1 Mischung der Verbindungen (**3**)+(**4**) | 0,02 g |
| | Natriumchlorid | 15 g |
| B | Senfsamenmehl | 5g |
| | Senfaroma | 0,1g |

Teil A wurde eingewogen. Es wurden 290 ml Wasser vorgelegt und unter Rühren Teil A zugegeben und gelöst. Die Lösung wird mit Wasser auf 1,84 kg verdünnt (pH 6,0) und anschließend gefriergetrocknet (Eutektischer Punkt: -15°C; Arbeitsvakuum: 0,52 mbar; Stellflächentemperatur: -5°C bis +25°C). Das Produkt wird mit Senfsamenmehl und dem Senfaroma aus Teil B gemischt und zu einem Würzmittel konfektioniert.

### Beispiel 4: Reaktionsaroma Typ Umami

| **Inhaltsstoff** | **Einsatz [g]** |
|---|---|
| L-Alanin | 41,0 |
| L-Asparaginsäure | 123,0 |
| Bernsteinsäure | 4,7 |
| Calciumchlorid Dihydrat | 7,0 |
| L-Cystein·HCl Monohydrat | 11,0 |
| Dikaliumphosphat | 6,0 |
| Fructose gemahlen | 1,0 |
| L-Isoleucin | 1,6 |
| Kaliumchlorid | 228,0 |
| L-Leucin | 1,6 |
| L-Lysin·HCl | 3,6 |
| Magnesiumchlorid Hexahydrat | 19,0 |
| Maltodextrin | 49,0 |
| L-Phenylalanin | 2,0 |
| L-Prolin | 74,0 |
| L-Serin | 6,5 |
| L-Threonin | 3,0 |
| L-Valin | 9,0 |
| Wasser | 399,0 |
| 1 : 1 Mischung der Verbindungen (**3**)+(**4**), 20 Gew.-% in EtOH | 10,0 |

Alle Komponenten werden bei 40°C gemischt und anschließend bei 85°C für 10 Minuten erhitzt (Rückflussreaktion). Nach dem Abkühlen auf 40°C wird mit Kalilauge auf pH 5 eingestellt. Dieses Umami-Reaktionsaroma wurde anstelle der 1 : 1 Mischung der reinen Verbindung ((**3**)+(**4**)) in die Bouillon - Zubereitungen C bzw. D des Anwendungsbeispiels 5 eingearbeitet, wobei in Zubereitung C 7 g und in Zubereitung D 16 g des Umami-Reaktionsaromas verwendet wurden.

### Anwendungsbeispiel 1: Instantsuppe, Typ Lauch-Creme

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Kartoffelstärke | 20,000 g | 20,000 g | 20,000 g | 20,000 g |
| Fettpulver | 25,000 g | 25,000 g | 25,000 g | 25,000 g |
| Lactose | 20,000 g | 20,000 g | 20,000 g | 20,000 g |
| Maltodextrin | 11,730 g | 14,724 g | 14,715 g | 14,690 g |
| Kochsalz | 8,000 g | 8,000 g | 8,000 g | 8,000 g |
| Natriumglutamat | 3,000 g | - | - | - |
| Spinatpulver | 2,000 g | 2,000 g | 2,000 g | 2,000 g |
| Grünes Lauchpulver | 2,000 g | 2,000 g | 2,000 g | 2,000 g |
| Citronensäure, als Pulver | 0,300 g | 0,300 g | 0,300 g | 0,300 g |
| Gehärtetes Pflanzenfett | 3,000 g | 3,000 g | 3,000 g | 3,000 g |
| Gefriergetrockneter Lauch | 1,000 g | 1,000 g | 1,000 g | 1,000 g |
| Huhnaroma | 1,000 g | 1,000 g | 1,000 g | 1,000 g |
| Würzmischung Typ "grüner Lauch", Pulver | 2,000 g | 2,000 g | 2,000 g | 2,000 g |
| Würzmischung, Typ "gekochte Zwiebel" | 0,600 g | 0,600 g | 0,600 g | 0,600 g |
| Hefe-Würzmischung, Typ "Gemüsebrühe", Pulver | 0,300 g | 0,300 g | 0,300 g | 0,300 g |
| Curcuma-Extrakt | 0,070 g | 0,070 g | 0,07 g | 0,070 g |
| 1 : 1 Mischung der Verbindungen (**3**)+(**4**) | - | 0,006 g | 0,015 g | 0,040 g |

5 g der jeweiligen Pulvermischung wurden mit je 100 ml heißem Wasser aufgegossen, um eine verzehrfertige Suppe zu erhalten. Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Anwendungsbeispiel 2: Instantsuppe, Typ Hühnersuppe mit Nudeln

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Stärke | 16,000 g | 16,000 g | 16,000 g | 16,000 g |
| Kochsalz | 7,000 g | 7,000 g | 7,000 g | 7,000 g |
| Saccharose, raffiniert | 3,200 g | 3,200 g | 3,200 g | 3,200 g |
| Natriumglutamat | 3,200 g | - | - | - |
| Natriuminosinat / | 0,800 g | 0,800 g | 0,800 g | 0,800 g |
| Natriumguanylat im Verhältnis 1:1 | | | | |
| Säurehydrolysiertes Pflanzenprotein | 8,000 g | 8,000 g | 8,000 g | 8,000 g |
| Fettpulver | 2,000 g | 2,000 g | 2,000 g | 2,000 g |
| Gemüsefett, sprüh- | 1,000 g | 1,000 g | 1,000 g | 1,000 g |
| getrocknet | | | | |
| Gefriergetrocknetes | 2,000 g | 2,000 g | 2,000 g | 2,000 g |
| Hühnerfleisch, in Stückchen | | | | |
| Suppen-Nudeln | 32,000 g | 32,000 g | 32,000 g | 32,000 g |
| Maltodextrin | 12,160 g | 15,354 g | 15,345 g | 14,120 g |
| Chinesisches | 4,600 g | 4,600 g | 4,600 g | 4,600 g |
| Gemüse, gefriergetrocknet | | | | |
| Huhnaroma | 8,000 g | 8,000 g | 8,000 g | 8,000 g |
| Lebensmittelfarbstoff | 0,040 g | 0,040 g | 0,040 g | 0,040 g |
| Riboflavin | | | | |
| 1 : 1 Mischung der Verbindungen (**5**)+(**6**) | - | 0,006 g | 0,015 g | 0,040 g |
| Aromenkomposition nach Beispiel 2 | - | - | - | 1,200 g |

4,6 g der jeweiligen Pulvermischung wurden 10 Minuten in je 100 ml Wasser gekocht, um eine verzehrfertige Suppe zu erhalten.

Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Anwendungsbeispiel 3: Würzmischung, Typ "Pfeffer":

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Milchprotein | 0,800 g | 0,800 g | 0,800 g | 0,800 g |
| Johannisbrotkernmehl | 2,000 g | 2,000 g | 2,000 g | 2,000 g |
| Maisstärke | 22,000 g | 27,988 g | 27,950 g | 27,880 g |
| Kochsalz | 14,000 g | 14,000 g | 14,000 g | 14,000 g |
| Paprikapulver | 13,000 g | 13,000 g | 13,000 g | 13,000 g |
| Tomatenpulver | 13,000 g | 13,000 g | 13,000 g | 13,000 g |
| Saccharose | 4,000 g | 4,000 g | 4,000 g | 4,000 g |
| Knoblauchpulver | 0,500 g | 0,500 g | 0,500 g | 0,500 g |
| Gehärtetes Pflanzenfett | 8,000 g | 8,000 g | 8,000 g | 8,000 g |
| Fettpulver | 11,000 g | 11,000 g | 11,000 g | 11,000 g |
| Natriumglutamat | 6,000 g | - | - | - |
| Lebensmittelfarbstoff Rote Bete und Paprika | 2,000 g | 2,000 g | 2,000 g | 2,000 g |
| Aroma Typ "Pfeffer" | 2,000 g | 2,000 g | 2,000 g | 2,000 g |
| Aroma Typ "Pizza" | 1,200 g | 1,200 g | 1,200 g | 1,200 g |
| Aroma Typ "Tomate" | 0,400 g | 0,400 g | 0,400 g | 0,400 g |
| Extrakt aus schwarzem Pfeffer | 0,100g | 0,100g | 0,100g | 0,100g |
| Verbindung der Formel (**3**)+(**4**) | - | 0,012 g | 0,050 g | 0,120 g |

Jeweils 100 g Nackensteak vom Schwein wurde mit jeweils 1,7 g der Zubereitungen A, B, C und D gleichmäßig bestreut und gebraten. Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Anwendungsbeispiel 4: Tomatenketchup

| **Bestandteil** | **Vergleichszubereitung A** | **Vergleichszubereitung B** | **Erfindungsgemäße Zubereitung C** |
|---|---|---|---|
| Natriumglutamat | 0,4 g | - | - |
| Kochsalz | 2,0 g | 2,0 g | 2,0 g |
| Stärke, Farinex WM 55 | 1,0 g | 1,0 g | 1,0 g |
| Sucrose | 12,0 g | 9,2 g | 9,2 g |
| Tomaten-Konzentrat 2-fach | 40,0 g | 40,0 g | 40,0 g |
| Glucosesirup 80 Brix | 18,0 g | 18,0 g | 18,0 g |
| Branntweinessig 10% | 7,0 g | 7,0 g | 7,0 g |
| Wasser | 19,6 g | 22,8 g | 22,2 g |
| 1%ige Lösung einer 1 : 1 Mischung der Verbindungen (**3**)+(**4**) in Propylenglycol | - | - | 0,2 g |
| Aromenkomposition nach Beispiel 2 | - | - | 0,4 g |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 5: Bouillon

| **Bestandteil** | **Vergleichszubereitung A** | **Natriumglutamatreduzierte Vergleichszubereitung B** | **Erfindungsgemäße Natriumglutamatreduzierte Zubereitung C** | **Erfindungsgemäße Natriumglutamatfreie Zubereitung D** |
|---|---|---|---|---|
| Fettpulver | 8,77 g | 8,77 g | 8,77 g | 8,77 g |
| Natrium glutamat | 8,77 g | 5,00 g | 5,00 g | - |
| Hefeextrakt Pulver | 12,28 g | 12,28 g | 12,28 g | 12,28 g |
| Kochsalz | 29,83 g | 29,83 g | 29,83 g | 29,83 g |
| Maltodextrin | 37,28 g | 41,05 g | 41,03 g | 46,00 g |
| Natürlicher Gemüseextrakt | 3,07 g | 3,07 g | 3,07 g | 3,07 g |
| Verbindung der Formel (3)+(4) | - | - | 0,02 g | 0,05 g |

15 g der jeweiligen Pulvermischung wurden mit je 1000 ml heißem Wasser aufgegossen. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 6: Würzmischung für Kartoffelchips

| **Bestandteil** | **Vergleichszubereitung A** | **Natriumglutamatreduzierte Vergleichszubereitung B** | **Erfindungsgemäße Natriumglutamatreduzierte Zubereitung C** | **Erfindungsgemäße Natriumglutamatfreie Zubereitung D** |
|---|---|---|---|---|
| Natriumglutamat | 3,50 g | 2,00 g | 2,00 g | - |
| Käsepulver | 10,00 g | 10,00 g | 10,00 g | 10,00 g |
| Knoblauchpulver | 2,00 g | 2,00 g | 2,00 g | 2,00 g |
| Molkenpulver | 38,86 g | 40,36 g | 40,16 g | 41,66 g |
| Würzextraktöl | 0,20 g | 0,20 g | 0,20 g | 0,20 g |
| Paprikapulver | 9,80 g | 9,80 g | 9,80 g | 9,80 g |
| Kochsalz | 21,00 g | 21,00 g | 21,00 g | 21,00 g |
| Tomatenpulver | 9,00 g | 9,00 g | 9,00 g | 9,00 g |
| Trockenaroma | 2,50 g | 2,50 g | 2,50 g | 2,50 g |
| Siliciumdioxid | 0,02 g | 0,02 g | 0,02 g | 0,02 g |
| Pflanzenöl | 0,02 g | 0,02 g | 0,02 g | 0,02 g |
| Zwiebelpulver | 3,00 g | 3,00 g | 3,00 g | 3,00 g |
| Sahne Aroma- konzentrat | 0,03 g | 0,03 g | 0,03 g | 0,03 g |
| Käse Aroma | 0,03 g | 0,03 g | 0,03 g | 0,03 g |
| Tomaten Aromakonzentrat | 0,04 g | 0,04 g | 0,04 g | 0,04 g |
| **Sprühgetrocknete Zusammensetzung gemäß Beispiel 1.1** | - | - | 0,20 g | 0,70 g |

6 g der Würzmischung wurden auf 94 g Kartoffelchips aufgezogen. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 7: Weiße Soße

| **Bestandteil** | **Vergleichszubereitung A** | **Natriumglutamatreduzierte Vergleichszubereitung B** | **Erfindungsgemäße Natriumglutamatreduzierte Zubereitung C** | **Erfindungsgemäße Natriumglutamatfreie Zubereitung D** |
|---|---|---|---|---|
| Maltodextrin | 25,98 g | 27,18 g | 27,13 g | 27,78 g |
| Kochsalz | 7,50 g | 7,50 g | 7,50 g | 7,50 g |
| Natriumglutamat | 2,00 g | 0,80 g | 0,80 g | - |
| Pflanzenfett | 5,00 g | 5,00 g | 5,00 g | 5,00 g |
| Pfeffer, weiß | 0,02 g | 0,02 g | 0,02 g | 0,02 g |
| Zwiebelpulver | 1,50 g | 1,50 g | 1,50 g | 1,50 g |
| vorverkleisterte Maisstärke | 30,00 g | 30,00 g | 30,00 g | 30,00 g |
| Fettpulver | 28,00 g | 28,00 g | 28,00 g | 28,00 g |
| **Sprühgetrocknete Zusammensetzung gemäß Beispiel 1.2** | - | - | 0,05 g | 0,20 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 8: Braune Soße

| **Bestandteil** | **Vergleichszubereitung A** | **Natriumglutamat-reduzierte Vergleichszubereitung B** | **Erfindungsgemäße Natriumglutamat-reduzierte Zubereitung C** | **Erfindungsgemäße Natriumglutamat-freie Zubereitung D** |
|---|---|---|---|---|
| Stärke | 40,00 g | 40,70 g | 40,65 g | 41,80 g |
| Maltodextrin | 33,10 g | 33,10 g | 33,10 g | 33,10 g |
| Kochsalz | 6,00 g | 6,00 g | 6,00 g | 6,00 g |
| Zuckerkulör, sprüh getrocknet | 5,00 g | 5,00 g | 5,00 g | 5,00 g |
| Hefeextraktpulver | 3,00 g | 3,00 g | 3,00 g | 3,00 g |
| Natriumglutamat | 2,00 g | 1,30 g | 1,30 g | - |
| Zucker | 0,50 g | 0,50 g | 0,50 g | 0,50 g |
| Fettpulver | 5,00 g | 5,00 g | 5,00 g | 5,00 g |
| Tomatenpulver | 3,00 g | 3,00 g | 3,00 g | 3,00 g |
| Natürlicher Gemüseextrakt | 1,00 g | 1,00 g | 1,00 g | 1,00 g |
| Zwiebelextrakt | 0,30 g | 0,30 g | 0,30 g | 0,30 g |
| Pfefferextrakt | 0,10 g | 0,10 g | 0,10 g | 0,10 g |
| Trockenaroma | 1,00 g | 1,00 g | 1,00 g | 1,00 g |
| **SprühgetrockneteZusammensetzung gemäß Beispiel 1.2** | - | - | 0,05 g | 0,20 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 9: Tomatensuppe

| **Bestandteil** | **Vergleichszubereitung A** | **Natriumglutamat-reduzierte Vergleichszubereitung B** | **Erfindungsgemäße Natriumglutamat-reduzierte Zubereitung C** | **Erfindungsgemäße Natriumglutamat-freie Zubereitung D** |
|---|---|---|---|---|
| Wasser | 50,650 g | 50,800 g | 50,792 g | 51,025 g |
| Pflanzenöl | 5,500 g | 5,500 g | 5,500 g | 5,500 g |
| Tomatenpaste | 24,000 g | 24,000 g | 24,000 g | 24,000 g |
| Sahne | 1,050 g | 1,050 g | 1,050 g | 1,050 g |
| Zucker | 2,000 g | 2,000 g | 2,000 g | 2,000 g |
| Kochsalz | 1,700 g | 1,700 g | 1,700 g | 1,700 g |
| Natriumglutamat | 0,400 g | 0,250 g | 0,250 g | - |
| Weizenmehl | 5,500 g | 5,500 g | 5,500 g | 5,500 g |
| Stärke | 1,200 g | 1,200 g | 1,200 g | 1,200 g |
| gewürfelte Tomaten | 8,000 g | 8,000 g | 8,000 g | 8,000 g |
| **Sprühgetrocknete Zusammensetzung gemäß Beispiel 1.2** | - | - | 0,008 g | 0,025 g |

Die festen Bestandteile wurden eingewogen, gemischt und dem Wasser hinzugefügt. Das Pflanzenöl wurde zudosiert und die Tomatenpaste hinzugegeben. Die Mischung wurde unter Rühren aufgekocht. Bei der Verkostung durch ein Panel ausgebildeter Testpersonen wurden die Vergleichszubereitung A und die erfindungsgemäße Natriumglutamat-reduzierte Zubereitung C bzw. Natriumglutamat-freie Zubereitung D sehr ähnlich bezeichnet. Insgesamt wurden die erfindungsgemäßen Zubereitungen C und D hinsichtlich ihres Umami-Geschmacks (und Mundfülle) als sehr ausgeprägt und deutlich stärker als die Natriumglutamat-reduzierte Vergleichszubereitung B bewertet.

### Anwendungsbeispiel 10: Anwendung in einem zuckerfreien Kaugummi

| **Teil** | **Inhaltsstoff** | **Einsatz in Gew.-%** |
|---|---|---|
| A | Kaugummibase, Company "Jagum T" | 29,995 |
| B | Sorbit, pulverisiert | 39,000 |
| | Isomalt® (Palatinit GmbH) | 9,500 |
| | Xylit | 2,000 |
| | Mannit | 3,000 |
| | Aspartam® | 0,100 |
| | Acesulfam® K | 0,100 |
| | Emulgum® (Colloides Naturels, Inc.) | 0,300 |
| C | Sorbitol, 70% | 14,000 |
| | Glycerin | 1,000 |
| D | Aromakomposition, entsprechend Beispiel 2 | 1,000 |
| | 1 : 1 Mischung der Verbindungen (**4**) + (**5**) | 0,005 |

Teile A bis D werden gemischt und intensiv geknetet. Die Rohmasse kann z.B. in Form von dünnen Streifen zu verzehrfertigen Kaugummis verarbeitet werden.

### Anwendungsbeispiel 11: Vergleichsuntersuchung "Anwendung in einem Grünteegetränk"

| **Inhaltsstoff** | **Einsatz in Gew.-%** |
|---|---|
| Grünteekonzentrat | 18,000 |
| 1% ige Lösung einer 1 : 1 Mischung der Verbindungen (**3**) + (**4**) in Propylenglycol | 0,004 |
| entmineralisiertes Wasser | 81,996 |

Das Grünteekonzentrat wird mit der 1% igen Lösung einer 1 : 1 Mischung der Verbindungen (**3**) + (**4**) in Propylenglycol vermischt. Anschließend wird mit entmineralisiertem Wasser aufgefüllt und erneut gründlich durchmischt. Dann wird das Produkt gefiltert, verbrauchsfertig verpackt und bei 118°C sterilisiert. Der Geschmack wird durch ein Panel ausgebildeter Testpersonen als deutlich bevorzugt gegenüber der nicht aromatisierten Grünteebase bewertet.

### Anwendungsbeispiel 12: Vergleichsuntersuchung "Rindfleischwürz-mischung für (Fertig)-Nudeln"

| **Inhaltsstoff** | **Gew.-%** |
|---|---|
| Rindsfettaroma | 5,00 |
| Zuckercouleur | 3,00 |
| Zitronensäure (wasserfrei) | 0,40 |
| Schnittlauch (entwässert) | 2,00 |
| Knoblauchpulver | 3,50 |
| Maltodextrin (ex Tapoica) | 10,30 |
| Mononatriumglutamat | 15,00 |
| Zwiebelpulver | 5,00 |
| Ribotide | 0,80 |
| Natriumchlorid | 45,65 |
| Zucker | 2,80 |
| Süßmolkepulver | 6,50 |
| 1% ige Lösung einer 1 : 1 Mischung der Verbindungen (**3**) + (**4**) in Propylenglycol | 0,05 |

Alle Inhaltsstoffe werden vermischt bis sich eine homogene Mischung ergibt.

### Anwendungsbeispiel 13: Verqleichsuntersuchung "(Fertig)-Nudeln"

| **Teil** | **Inhaltsstoff** | **Gew.-%** |
|---|---|---|
| A | Weizenmehl | 62,00 |
| | Kartoffelstärke | 10,90 |
| B | Salz | 1,10 |
| | Guarkernmehl | 0,06 |
| | Natriumcarbonat | 0,07 |
| | Kaliumcarbonat | 0,25 |
| | Na₂H₂P₂O₇ | 0,07 |
| | 1% ige Lösung einer 1 : 1 Mischung der Verbindungen (**5**) + (**6**) in Propylenglycol | 0,05 |
| C | Wasser | 25,5 |

Eine Suspension der Zutaten B in Wasser wird zu einer Mischung der Zutaten A gegeben und zu einem Teig geknetet. Nachdem der Teig für ca. 5 Minuten geruht hat, wird dieser mit Hilfe einer Nudelmaschine zu Platten verarbeitet, die in einem letzten Arbeitsschritt in eine übliche Form zurechtgeschnitten werden. Die Nudeln sind nach einer Kochzeit von 3 Minuten verzehrfertig und werden mit 8 g der Rindfleischwürzmischung (Anwendungsbeispiel 12) angerichtet.

Im Folgenden wird die vorliegende Erfindung anhand weiterer Ausführungsbeispiele erläutert, ohne diese dadurch einzuschränken:

### Weitere Ausführungsbeispiele

### Ausführungsbeispiel 1:

Verwendung einer Verbindung oder einer Mischung bestehend aus zwei oder mehreren Verbindungen oder enthaltend eine oder mehrere Verbindungen ausgewählt aus der Gruppe bestehend aus Verbindungen der Formeln (I) und (*ent*-I) 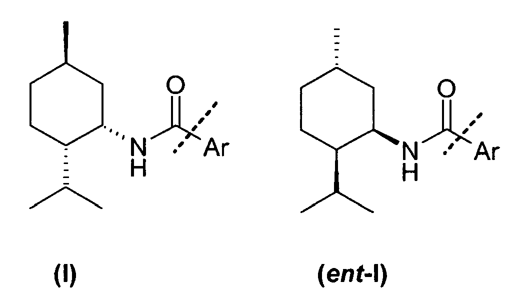 wobei in den Formeln (I) und (*ent-*I) gilt:
der aromatische Rest Ar ist ausgewählt aus der Gruppe bestehend aus: 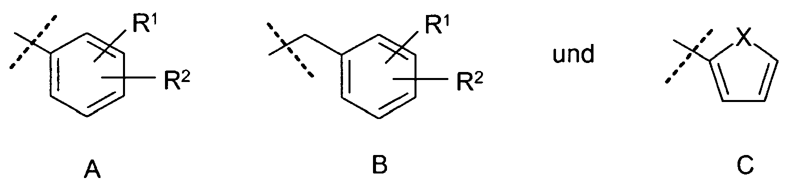
wobei die gestrichelte Linie die Bindung markiert, welche den aromatischen Rest Ar mit dem in Formel (I) oder (*ent*-I) benachbarten Carbonyl-C-Atom verknüpft, und wobei in den aromatischen Resten A und B gilt:
R¹ und R² sind unabhängig voneinander ausgewählt aus der Gruppe bestehend aus H, OH, OCH₃, OCH₂CH₃, OCH(CH₃)₂, COOCH₃, COOCH₂CH₃, COOCH(CH₃)₂,
   oder R¹ und R² sind benachbart und bilden zusammen eine Gruppe OCH₂O, und wobei im aromatischen Rest C gilt:
   X ist O oder S, als Geschmacksstoff bzw. Geschmacksstoffmischung.

### Ausführungsbeispiel 2:

Verwendung einer Verbindung oder einer Mischung wie in Ausführungsbeispiel 1 definiert, wobei für die Verbindung der Formeln (I) und (*ent-*I) bzw. für eine, mehrere oder sämtliche Verbindungen der Formeln (I) und (*ent-*I) in der Mischung gilt:
R¹ und R² in den aromatischen Resten A bzw. B sind unabhängig voneinander ausgewählt aus der Gruppe bestehend aus H, OCH₃, COOCH₃,
   oder R¹ und R² sind benachbart und bilden zusammen eine Gruppe OCH₂O, und X im aromatischen Rest C ist O.

### Ausführungsbeispiel 3:

Verwendung einer Verbindung oder einer Mischung wie in einem der vorhergehenden Ausführungsbeispiele definiert, wobei in der Verbindung der Formeln (I) und (*ent-*I) bzw. in einer, mehreren oder sämtlichen Verbindungen der Formeln (I) und (*ent*-I) in der Mischung der aromatische Rest Ar ausgewählt ist aus der Gruppe bestehend aus:

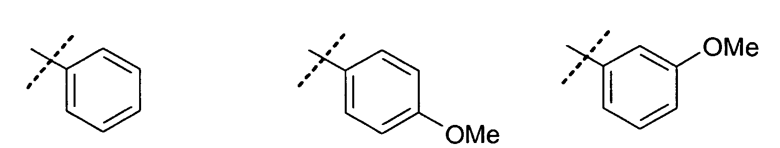

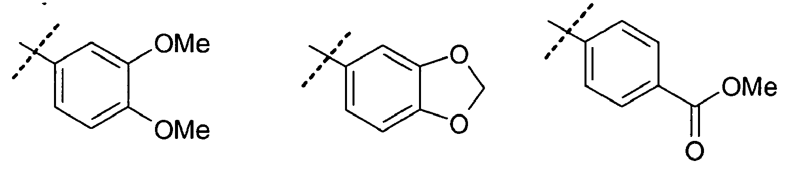

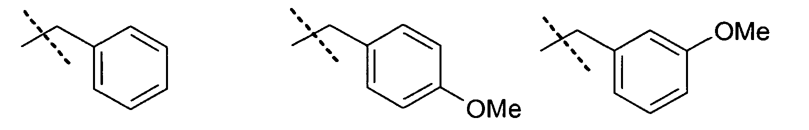

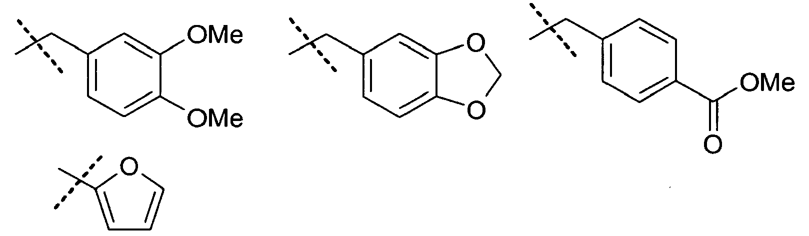

### Ausführungsbeispiel 4:

Verwendung einer Verbindung oder einer Mischung wie in einem der vorhergehenden Ausführungsbeispiele definiert, wobei die Verbindung der Formeln (I) und (*ent*-I) bzw. eine, mehrere oder sämtliche Verbindungen der Formeln (I) und (*ent*-I) in der Mischung ausgewählt sind aus der Gruppe bestehend aus:
(1) *N*-((1*S*,2*S*,5*R*)-2-Isopropyl-5-methyl-cyclohexyl)-terephthalsäuremethylester
(2) *N*-((1*R*,2*R*,5*S*)-2-Isopropyl-5-methyl-cyclohexyl)-terephthalsäuremethylester
(3) Benzo[1,3]dioxol-5-carbonsäure ((1 *S*,2*S*,5*R*)-2-isopropyl-5-methylcyclohexyl)-amid
(4) Benzo[1,3]dioxol-5-carbonsäure ((1*R*,2*R*,5*S*)-2-isopropyl-5-methylcyclohexyl)-amid
(5) 2-Benzo[1,3]dioxol-5-yl-*N*-((1*S*,2*S*,5*R*)-2-isopropyl-5-methylcyclohexyl)acetamid
(6) 2-Benzo[1,3]dioxol-5-yl-*N*-((1*R*,2*R*,5*S*)-2-isopropyl-5-methylcyclohexyl)acetamid

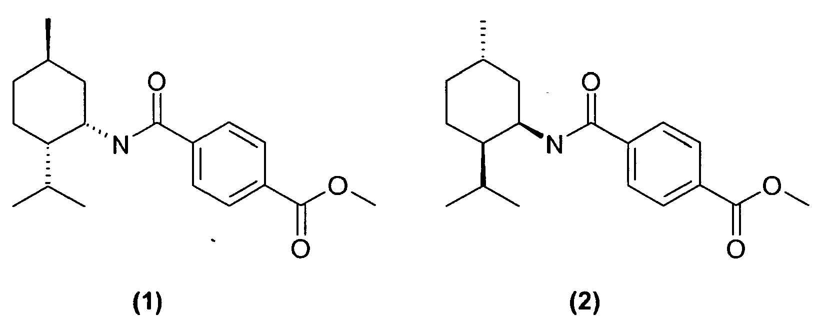

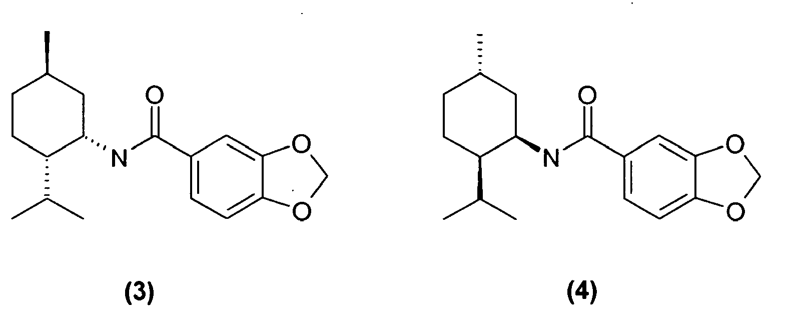

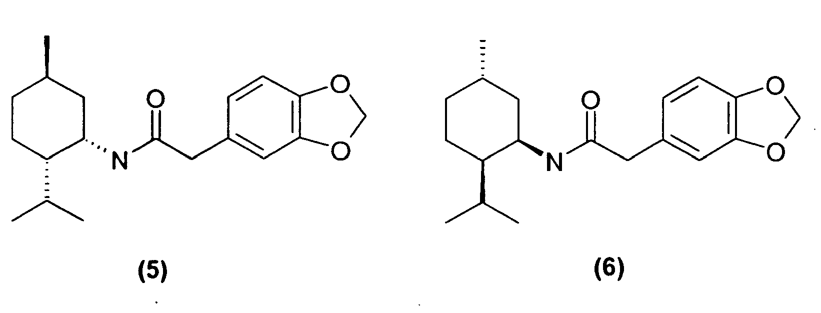

### Ausführungsbeispiel 5:

Verwendung einer Mischung wie in einem der vorhergehenden Ausführungsbeispiele definiert, enthaltend ein oder mehrere Enantiomerenpaare oder bestehend aus einem oder mehreren Enantiomerenpaaren bestehend aus jeweils einer Verbindung der Formel (I) und einer Verbindung der Formel (*ent*-I).

### Ausführungsbeispiel 6:

Verwendung einer Mischung wie in einem der vorhergehenden Ausführungsbeispiele definiert, wobei die Mischung die folgenden Verbindungen enthält oder aus ihnen besteht:
Benzo[1,3]dioxol-5-carbonsäure ((1*S*,2*S*,5*R*)-2-isopropyl-5-methylcyclohexyl)amid und
Benzo[1,3]dioxol-5-carbonsäure ((1*R*,2*R*,5*S*)-2-isopropyl-5-methyl-cyclohexyl)amid.

### Ausführungsbeispiel 7:

Verwendung einer Mischung, welche aus folgenden Komponenten besteht oder diese enthält:
eine Verbindung oder eine Mischung wie in einem der Ausführungsbeispiele 1 bis 6 definiert sowie eine Verbindung ausgewählt aus der Gruppe bestehend aus Verbindungen der Formeln (II), (*ent-*II), (III), (*ent*-III), (IV), (*ent*-IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent*-II), (III), (*ent*-III), (IV), (*ent*-IV): 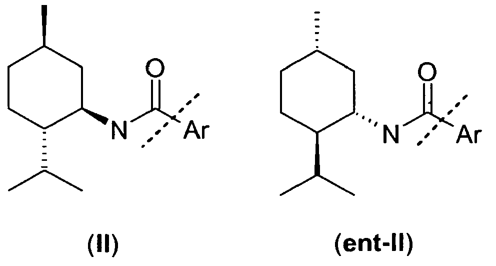 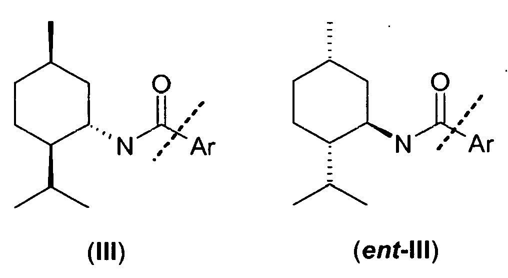 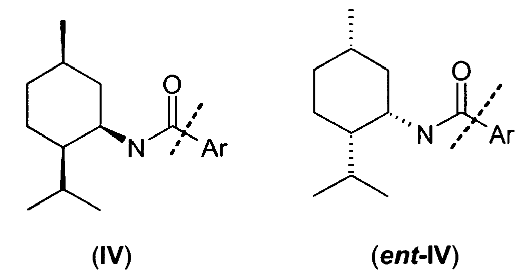
wobei in den Formeln (II), (*ent*-II), (III), (*ent*-III), (IV), (*ent*-IV) Ar unabhängig voneinander eine der in den Ausführungsbeispielen 1 bis 6 für die Formeln (I) und (*ent*-I) angegebenen Bedeutungen besitzt, als Geschmacksstoffmischung.

### Ausführungsbeispiel 8:

Verwendung einer Mischung wie in Ausführungsbeispiel 7 definiert, wobei das Gewichtsverhältnis von (a) der Gesamtheit von Verbindungen der Formeln (I) und (*ent*-I) zu (b) der Gesamtheit von Verbindungen der Formeln (II), (*ent*-II), (III), (*ent*-III), (IV) und (*ent*-IV) mindestens 60:40, bevorzugt mindestens 90:10, insbesondere bevorzugt mindestens 95:5 beträgt.

### Ausführungsbeispiel 9:

Verwendung nach einem der Ausführungsbeispiele 1 bis 8 zum Erzeugen, Vermitteln, Modifizieren oder Verstärken eines Umami-Geschmacks.

### Ausführungsbeispiel 10:

Einzelne Verbindung oder Mischung wie in den Ausführungsbeispielen 1 bis 6 definiert, mit der Maßgabe, dass
(i) einzelne Verbindungen der Formel (I), in welchen der aromatische Rest Ar ausgewählt ist aus der Gruppe bestehend aus 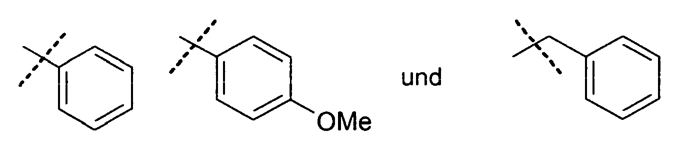 und
(ii) Mischungen, welche aus einem Enantiomerenpaar der Formeln (I)/(*ent*-I) bestehen mit einem aromatischen Rest ausgewählt aus der Gruppe bestehend aus 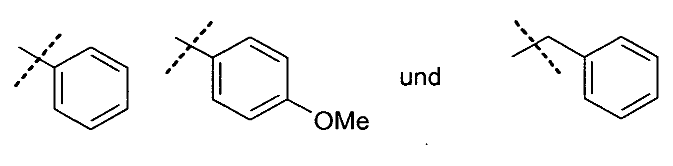 und
(iii) Mischungen bestehend aus
   Benzoyl-d-neo-Menthylamin und Benzoyl-I-menthylamin bzw.
   Benzoyl-d-neo-Menthylamin und Benzoyl-d-iso-Menthylamin ausgeschlossen sind.

### Ausführungsbeispiel 11:

Einzelne Verbindung oder Mischung wie in Ausführungsbeispiel 10 definiert,
wobei für die einzelne Verbindung der Formeln (I) und (*ent-*I) bzw. für eine, mehrere oder sämtliche Verbindungen der Formeln (I) und (*ent-*I) in der Mischung gilt:
R¹ und R² in den aromatischen Resten A bzw. B sind unabhängig voneinander ausgewählt aus der Gruppe bestehend aus H, OCH₃, COOCH₃,
   oder R¹ und R² sind benachbart und bilden zusammen eine Gruppe OCH₂O, und X im aromatischen Rest C ist O.

### Ausführungsbeispiel 12:

Einzelne Verbindung oder Mischung wie in einem der Ausführungsbeispiele 10 oder 11 definiert, wobei in der einzelnen Verbindung der Formeln (I) und (*ent-*I) bzw. in einer, mehreren oder sämtlichen Verbindungen der Formeln (I) und *(ent-*
I) in der Mischung der aromatische Rest Ar ausgewählt ist aus der Gruppe bestehend aus:

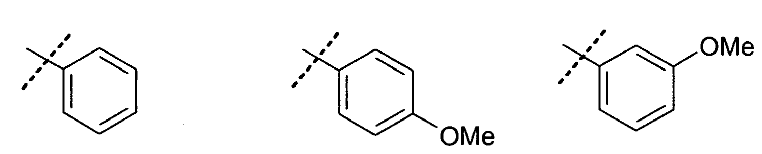

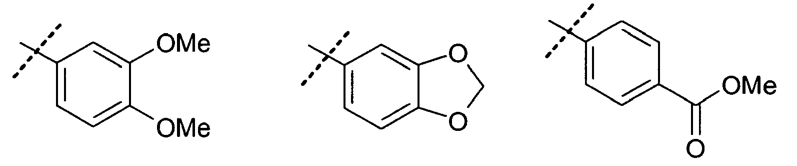

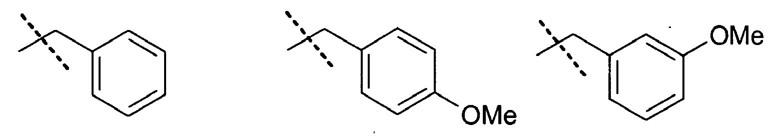

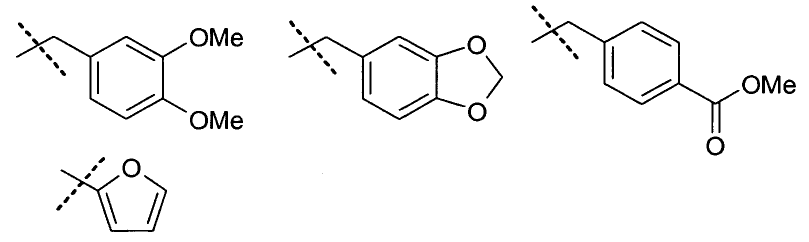

### Ausführungsbeispiel 13:

Einzelne Verbindung oder Mischung wie in einem der Ausführungsbeispiele 10 bis 12 definiert, wobei die einzelne Verbindung der Formeln (I) und (*ent*-I) bzw. eine, mehrere oder sämtliche Verbindungen der Formeln (I) und (*ent*-I) in der Mischung ausgewählt sind aus der Gruppe bestehend aus:
(1) *N*-((1*S*,2*S*,5*R*)-2-Isopropyl-5-methyl-cyclohexyl)-terephthalsäuremethylester
(2) *N*-((1*R*,2*R*,5*S*)-2-Isopropyl-5-methyl-cyclohexyl)-terephthalsäuremethylester
(3) Benzo[1,3]dioxol-5-carbonsäure ((1*S*,2*S*,5*R*)-2-isopropyl-5-methylcyclohexyl)-amid
(4) Benzo[1,3]dioxol-5-carbonsäure (1*R*,2*R*,5*S*)-2-isopropyl-5-methylcyclohexyl)-amid
(5) 2-Benzo[1,3]dioxol-5-yl-*N*-((1*S*,2*S*,5*R*)-2-isopropyl-5-methylcyclohexyl)acetamid
(6) 2-Benzo[1,3]dioxol-5-yl-*N*-((1*R*,2*R*,5*S*)-2-isopropyl-5-methyl cyclohexyl)acetamid

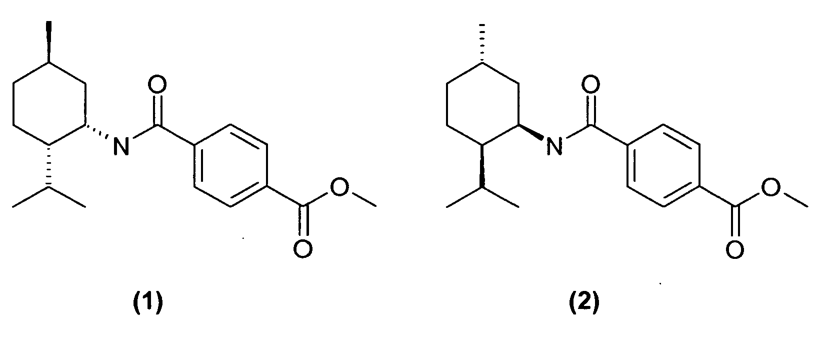

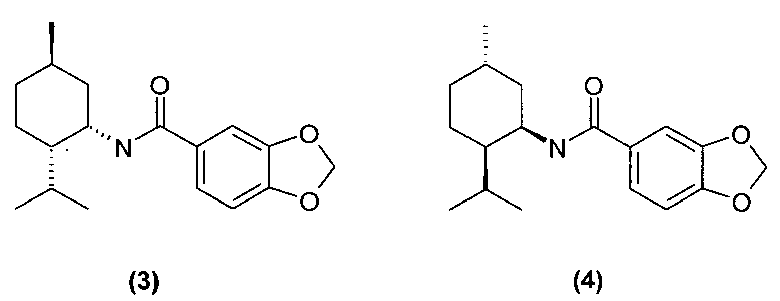

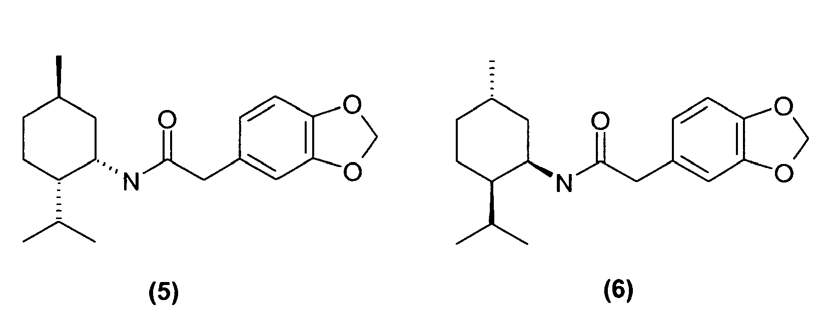

### Ausführungsbeispiel 14:

Mischung wie in einem der Ausführungsbeispiele 10 bis 13 definiert, enthaltend ein oder mehrere Enantiomerenpaare oder bestehend aus einem oder mehreren Enantiomerenpaaren bestehend aus jeweils einer Verbindung der Formel (I) und einer Verbindung der Formel (*ent*-I), mit der Maßgabe, dass Mischungen, welche aus einem Enatiomerenpaar der Formeln (I)/(*ent*-I) mit einem aromatischen Rest ausgewählt aus der Gruppe bestehend aus 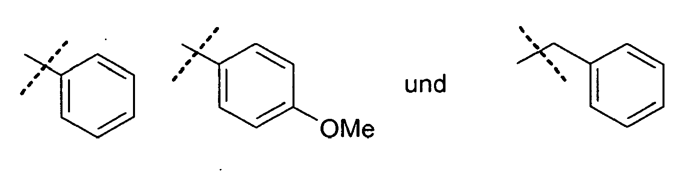 bestehen, ausgeschlossen sind.

### Ausführungsbeispiel 15:

Mischung wie in einem der Ausführungsbeispiele 10 bis 14 definiert, wobei die Mischung die folgenden Verbindungen enthält oder aus ihnen besteht:
Benzo[1,3]dioxol-5-carbonsäure ((1*S*,2*S*,5*R*)-2-isopropyl-5-methylcyclohexyl)amid und
Benzo[1,3]dioxol-5-carbonsäure ((1*R*,2*R*,5*S*)-2-isopropyl-5-methyl-cyclohexyl)amid.

### Ausführungsbeispiel 16:

Mischung, welche aus folgenden Komponenten besteht oder diese enthält:
   eine Verbindung oder eine Mischung wie in einem der Ausführungsbeispiele 1 bis 6 definiert sowie eine Verbindung ausgewählt aus der Gruppe bestehend aus Verbindungen der Formeln (II), (*ent*-II), (III), (*ent*-III), (IV), (*ent-*IV) oder eine Mischung bestehend aus zwei oder mehreren Verbindungen oder enthaltend eine oder mehrere Verbindungen ausgewählt aus der Gruppe bestehend aus Verbindungen der Formeln (II), (*ent*-II), (III), (*ent*-III), (IV), (*ent*-IV): 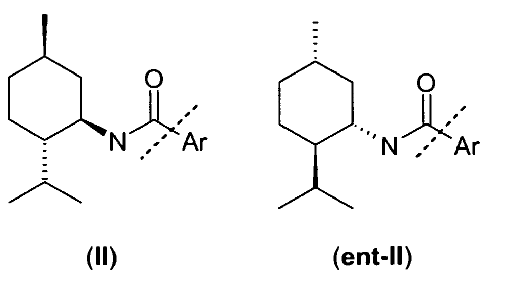 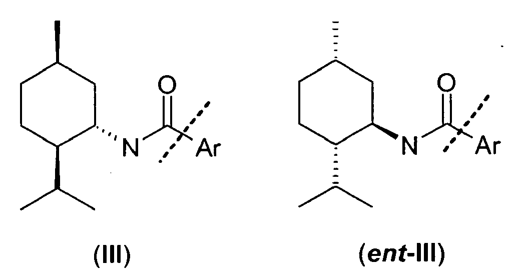 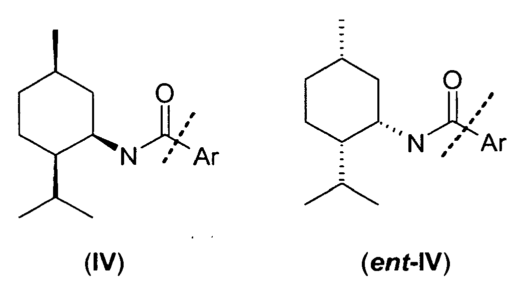
wobei in den Formeln (II), (*ent*-II), (III), (*ent*-III), (IV), (*ent*-IV) Ar unabhängig voneinander eine der in den Ausführungsbeispiele 1 bis 6 für die Formeln (I) und (*ent*-I) angegebenen Bedeutungen besitzt, mit der Maßgabe, dass Mischungen bestehend aus
Benzoyl-d-neo-Menthylamin und Benzoyl-I-menthylamin bzw.
Benzoyl-d-neo-Menthylamin und Benzoyl-d-iso-Menthylamin ausgeschlossen sind.

### Ausführungsbeispiel 17:

Mischung wie in Ausführungsbeispiel 16 definiert, wobei das Gewichtsverhältnis von (a) der Gesamtheit von Verbindungen der Formeln (I) und (*ent*-I) zu (b) der Gesamtheit von Verbindungen der Formeln (II), (*ent*-II), (III), (*ent*-III), (IV) und (*ent*-IV) mindestens 60:40, bevorzugt mindestens 90:10, insbesondere bevorzugt mindestens 95:5 beträgt.

### Ausführungsbeispiel 18:

Zusammensetzung, insbesondere zum Verzehr geeignete Zusammensetzung, umfassend bzw. bestehend aus einer geschmacklich wirksamen Menge einer Verbindung oder einer Mischung wie in einem der Ausführungsbeispiele 1 bis 6 definiert bzw. einer Mischung wie in einem der Ausführungsbeispiele 7 und 8 definiert und einem oder mehreren weiteren zum Verzehr geeigneten Bestandteilen.

### Ausführungsbeispiel 19:

Zusammensetzung nach Ausführungsbeispiel 18, wobei die weiteren Bestandteile sind:
- feste Trägerstoffe oder
- feste Trägerstoffe und Aromakompositionen oder
- Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung.

### Ausführungsbeispiel 20:

Zusammensetzung nach Ausführungsbeispiel 19, wobei die weiteren Bestandteile feste Trägerstoffe sind und das Gewichtsverhältnis der Gesamtmasse der Verbindungen der Formeln (I) und (*engt*-I) wie in einem der Ausführungsbeispiele 1 bis 6 definiert bzw. der Gesamtmasse der Verbindungen der Formeln (I), (*ent*-I), (II), (*ent*-II), (III), (*ent-*III), (IV), (*ent*-IV) wie in einem der Ausführungsbeispiele 7 und 8 definiert zu den festen Trägerstoffen im Bereich von 1 : 10 bis 1 : 100000, bevorzugt im Bereich von 1 : 50 (vorzugsweise von 1 : 100) bis 1 : 20000, besonders bevorzugt im Bereich von 1 : 100 (vorzugsweise von 1 : 1000) bis 1 : 5000, liegt, bezogen auf die Trockenmasse der Zusammensetzung.

### Ausführungsbeispiel 21:

Zusammensetzung nach Ausführungsbeispiel 19, umfassend oder bestehend aus
- insgesamt 0,01 bis 0,1 Gew.-% an einer oder mehreren Verbindungen der Formeln (I) und (*ent-*I), wie in einem der Ausführungsbeispiele 1 bis 6 definiert bzw. der Formeln (I), (*ent*-I), (II), (*ent*-II), (III), (*ent*-III), (IV), (*ent*-IV) wie in einem der Ausführungsbeispiele 7 und 8 definiert
- 5 bis 30 Gew.-% Wasser,
- 50 bis 90 Gew.-% einer Ölphase,
- 0,1 bis 5 Gew.-% eines verzehrbaren W/O-Emulgators bezogen auf die Gesamtmasse der Zusammensetzung, sowie gegebenenfalls ein oder mehrere Antioxidantien und gegebenenfalls ein oder mehrere Stoffe zur Verstärkung einer antioxidativen Wirkung.

### Ausführungsbeispiel 22:

Verzehrbare Zubereitung oder Halbfertigware, umfassend eine geschmacklich wirksame Menge
- einer Verbindung oder Mischung wie in einem der Ausführungsbeispiele 1 bis 6 definiert oder
- einer Mischung wie in einem der Ausführungsbeispiele 7 und 8 definiert oder
- eine Zusammensetzung nach einem der Ausführungsbeispiele 18 bis 21.

### Ausführungsbeispiel 23:

Der Ernährung, der Mundpflege oder dem Genuss dienende gebrauchs- oder verzehrfertige Zubereitung nach Ausführungsbeispiel 22, umfassend insgesamt 0,01 ppm bis 100 ppm, bevorzugt 0,1 ppm bis 50 ppm, besonders bevorzugt 0,5 ppm (vorzugsweise von 1 ppm) bis 30 ppm an einer oder mehreren Verbindungen der Formel (I) und (*ent*-I) wie in einem der Ausführungsbeispiele 1 bis 6 definiert bezogen auf das Gesamtgewicht der verzehrfertigen Zubereitung.

### Ausführungsbeispiel 24:

Halbfertigware gemäß Ausführungsbeispiel 22 umfassend
- insgesamt 10 ppm bis 100000 ppm (vorzugsweise bis 800 ppm), bevorzugt 25 ppm bis 5000 ppm (vorzugsweise bis 750 ppm), insbesondere 50 ppm bis 1200 ppm (vorzugsweise bis 700 ppm) an einer oder mehreren Verbindungen der Formel (I) und (*ent*-I), wie in einem der Ausführungsbeispiele 1 bis 6 definiert
- kein Natriumglutamat oder einen Anteil von 0,00001 bis 10 Gew.-%, bevorzugt 0,0001 bis 5 Gew.-%, insbesondere 0,001 Gew.-% bis 2 Gew.-% an Natriumglutamat,
- und gegebenenfalls einen Anteil von 0,0001 Gew.-% bis 90 Gew.-%, bevorzugt 0,001 Gew.-% bis 30 Gew.-% einer Aromakomposition, bezogen auf das Gesamtgewicht der Halbfertigware.

### Ausführungsbeispiel 25:

Zusammensetzung, Zubereitung oder Halbfertigware nach einem der Ausführungsbeispiele 18 bis 24, zusätzlich umfassend eine Substanz zum Maskieren oder Vermindern eines unangenehmen Geschmackseindrucks und/oder eine Substanz zum Verstärken des angenehmen Geschmackseindrucks eines angenehm schmeckenden Stoffes.

### Ausführungsbeispiel 26:

Verfahren zum Erzeugen, Vermitteln, Modifizieren oder Verstärken eines Geschmacks, insbesondere eines Umami-Geschmacks, in einer der Ernährung, der Mundpflege oder dem Genuss dienenden (i) gebrauchs- oder verzehrfertigen Zubereitung oder (ii) Halbfertigware umfassend den folgenden Schritt:
Vermischen einer geschmacklich wirksamen Menge einer Verbindung oder Mischung wie in einem der Ausführungsbeispiele 1 bis 6 definiert oder einer Mischung wie in einem der Ausführungsbeispiele 7 und 8 definiert oder einer Zusammensetzung nach einem der Ausführungsbeispiele 18 bis 21 mit einem oder mehreren weiteren Bestandteilen der (i) verzehr- oder gebrauchsfertigen Zubereitung oder der (ii) Halbfertigware oder
Applizieren einer geschmacklich wirksamen Menge einer Verbindung oder Mischung wie in einem der Ausführungsbeispiele 1 bis 6 definiert oder einer Mischung wie in einem der Ausführungsbeispiele 7 und 8 definiert oder einer Zusammensetzung nach einem der Ausführungsbeispiele 18 bis 21 auf einen oder mehrere weitere Bestandteile der (i) verzehr- oder gebrauchsfertigen Zubereitung oder der (ii) Halbfertigware oder
Einbetten einer geschmacklich wirksamen Menge einer Verbindung oder Mischung wie in einem der Ausführungsbeispiele 1 bis 6 definiert oder einer Mischung wie in einem der Ausführungsbeispiele 7 und 8 definiert oder einer Zusammensetzung nach einem der Ausführungsbeispiele 18 bis 21 in ein Hüll-oder Matrixmaterial.

### Figurenbeschreibung:

**Fig. 1****:** Geschmacklicher Vergleich von einer Mischung aus Benzo[1,3]dioxol-5-carbonsäure ((1*S*,2*S*,5*R*)-2-isopropyl-5-methyl-cyclohexyl)-amid (3) und Benzo[1,3]dioxol-5-carbonsäure ((1*R*,2*R*,5*S*)-2-isopropyl-5-methyl-cyclohexyl)-amid (4) mit Natriumglutamat

Der Geschmack eines 0,5% amerikanischen Rindfleischextrakts als Base (durchgezogene, helle Linie) wurde anhand einer Verkostung durch ein Panel ausgebildeter Testpersonen verglichen mit dem Geschmack erstens einer solchen Base, der 5 ppm einer Mischung aus Benzo[1,3]dioxol-5-carbonsäure ((1*S*,2*S*,5*R*)-2-isopropyl-5-methyl-cyclohexyl)-amid (3) und Benzo[1,3]dioxol-5-carbonsäure ((1*R*,2*R*,5*S*)-2-isopropyl-5-methyl-cyclohexyl)-amid (4) zugesetzt wurde (durchgezogene, dunkle Linie), und zweitens einer solchen Base, der 0,05 Gew.-% MSG zugesetzt wurde (punktierte Linie).

Die Testpersonen bewerteten die Stärke der angegebenen Geschmacksrichtungen jeweils durch Vergeben von Punktzahlen auf einer Skala von 0 (kein entsprechender Geschmack) bis 6 (sehr starker entsprechender Geschmack). Dargestellt sind die Mittelwerte der jeweiligen Punktzahlen.

## Patentansprüche

1. Verwendung einer Verbindung oder einer Mischung bestehend aus zwei oder mehreren Verbindungen oder enthaltend eine oder mehrere Verbindungen ausgewählt aus der Gruppe bestehend aus Verbindungen der Formeln (I) und (*ent-*I) 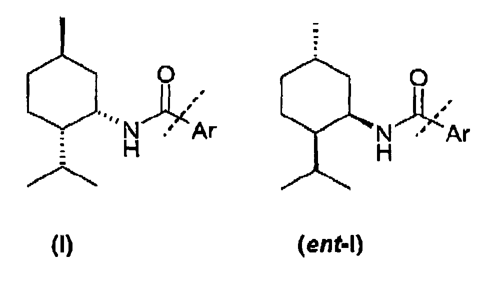 wobei in den Formeln (I) und (*ent*-I) gilt:
der aromatische Rest Ar ist ausgewählt aus der Gruppe bestehend aus: 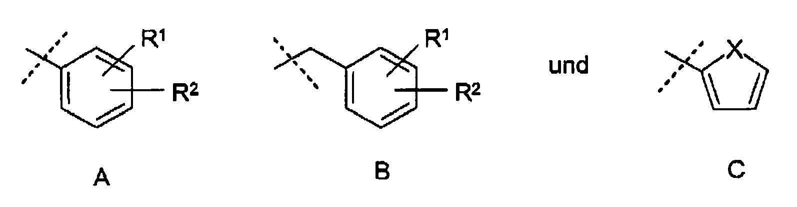 wobei die gestrichelte Linie die Bindung markiert, welche den aromatischen Rest Ar mit dem
in Formel (I) oder (*ent*-I) benachbarten Carbonyl-C-Atom verknüpft,
und wobei in den aromatischen Resten A und B gilt:
R¹ und R² sind unabhängig voneinander ausgewählt aus der Gruppe bestehend aus H, OH, OCH₃, OCH₂CH₉, OCH(CH₃)₂, COOCH₃, COOCH₂CH₃, COOCH(CH₃)₂, oder R¹ und R² sind benachbart und bilden zusammen eine Gruppe OCH₂O, und wobei im aromatischen Rest C gilt:
X ist O oder S,
als Geschmacksstoff bzw. Geschmacksstoffmischung.

2. Verwendung einer Verbindung oder einer Mischung wie in Anspruch 1 definiert, wobei für die Verbindung der Formeln (I) und (*ent*-I) bzw. für eine, mehrere oder sämtliche Verbindungen der Formeln (I) und (*ent*-I) in der Mischung gilt:
R¹ und R² in den aromatischen Resten A bzw. B sind unabhängig voneinander ausgewählt aus der Gruppe bestehend aus H, OCH₃, COOCH₃,
oder R¹ und R² sind benachbart und bilden zusammen eine Gruppe OCH₂O, und X im aromatischen Rest C ist O.

3. Verwendung einer Verbindung oder einer Mischung wie in einem der vorhergehenden Ansprüche definiert, wobei in der Verbindung der Formeln (I) und (*ent*-I) bzw. in einer, mehreren oder sämtlichen Verbindungen der Formeln (I) und (*ent*-I) in der Mischung der aromatische Rest Ar ausgewählt ist aus der Gruppe bestehend aus:

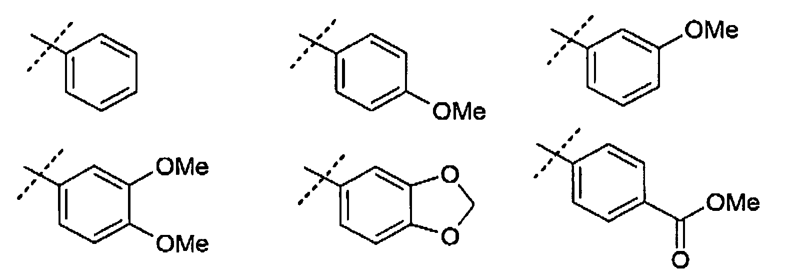

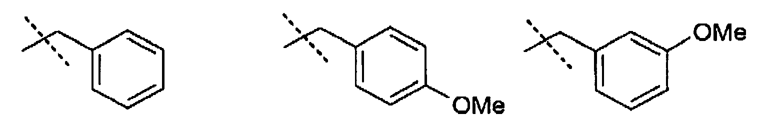

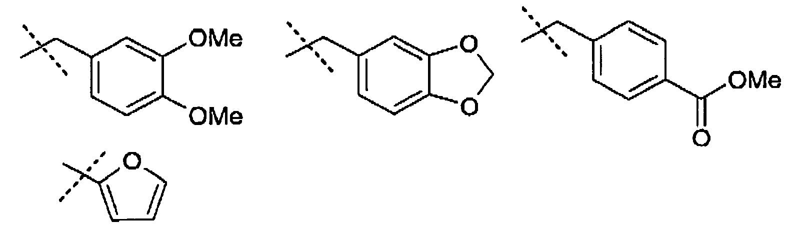

4. Verwendung einer Verbindung oder einer Mischung wie In einem der vorhergehenden Ansprüche definiert, wobei die Verbindung der Formeln (I) und (*ent*-I) bzw. eine, mehrere oder sämtliche Verbindungen der Formeln (I) und (*ent*-I) in der Mischung ausgewählt sind aus der Gruppe bestehend aus:
(1) *N*-((1*S*,2*S*,5*R*)-2-Isopropyl-5-methyl-cyclohexyl)-terephthalsäuremethylester
(2) *N*-((1*R*,2*R*,5*S*)-2-Isopropyl-5-methyl-cyclohexyl)-terephthalsäuremethylester
(3) Benzo[1,3]dioxol-5-carbonsäure ((1*S*,2*S*,5*R*)-2-isopropyl-5-methylcyclohexyl)-amid
(4) Benzo[1,3]dioxol-5-carbonsäure ((1*R*,2*R*,5*S-*)-2-isopropyl-5-methylcyclohexyl)-amid
(5) 2-Benzo[1,3]dioxol-5-yl-*N*-((1*S*,2*S*,5*R*)-2-isopropyl-5-methylcyclohexyl)acetamid
(6) 2-Benzo[1,3]dioxol-5-yl-*N*-((1*R*,2*R*,5*S*)-2-isopropyl-5-methylcyclohexyl)acetamid

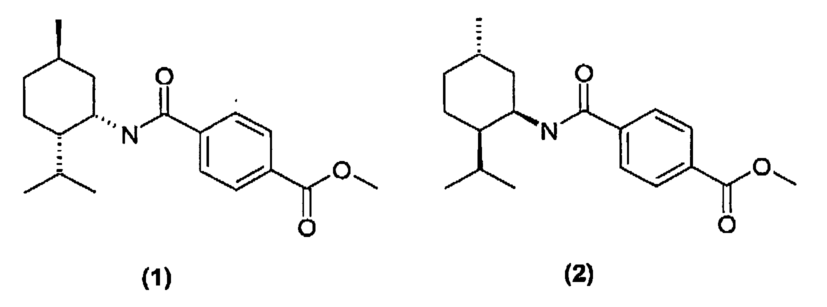

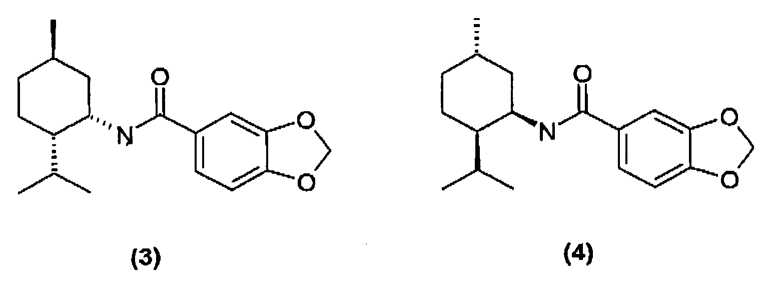

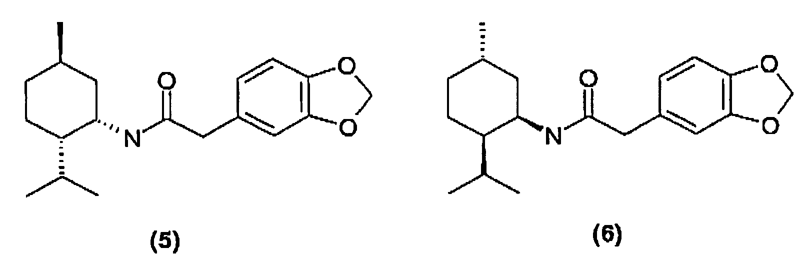

5. Verwendung nach einem der Ansprüche 1 bis 4 zum Erzeugen, Vermitteln, Modifizieren oder Verstärken eines Umami-Geschmacks.

6. Einzelne Verbindung oder Mischung wie in den Ansprüchen 1 bis 4 definiert, mit der Maßgabe, dass
(i) einzelne Verbindungen der Formel (I), in welchen der aromatische Rest Ar ausgewählt ist aus der Gruppe bestehend aus 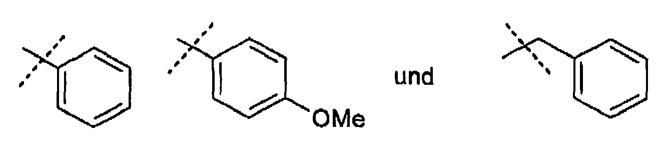 und
(ii) Mischungen, welche aus einem Enantiomerenpaar der Formeln (I)/(*ent*-I) bestehen mit einem aromatischen Rest ausgewählt aus der Gruppe bestehend aus 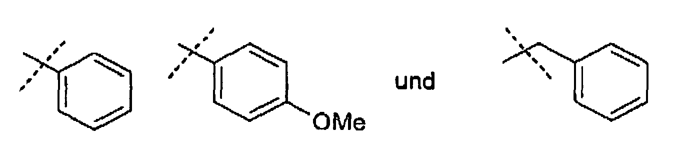 und
(iii) Mischungen bestehend aus
Benzoyl-d-neo-Menthylamin und Benzoyl-l-menthylamin bzw. Benzoyl-d-neo-Menthylamin und Benzoyl-d-iso-Menthyiamin ausgeschlossen sind.

7. Einzelne Verbindung oder Mischung wie in Anspruch 6 definiert,
wobei für die einzelne Verbindung der Formeln (I) und (*ent*-I) bzw. für eine, mehrere oder sämtliche Verbindungen der Formeln (I) und (*ent*-I) in der Mischung gilt:
R¹ und R² in den aromatischen Resten A bzw. B sind unabhängig voneinander ausgewählt aus der Gruppe bestehend aus H, OCH₃, COOCH₃,
oder R¹ und R² sind benachbart und bilden zusammen eine Gruppe OCH₂O, und X im aromatischen Rest C ist O.

8. Einzelne Verbindung oder Mischung wie in einem der Ansprüche 6 oder 7 definiert, wobei in der einzelnen Verbindung der Formeln (I) und (*ent*-I) bzw. in einer, mehreren oder sämtlichen Verbindungen der Formeln (I) und (*ent*-I) in der Mischung der aromatische Rest Ar ausgewählt ist aus der Gruppe bestehend aus:

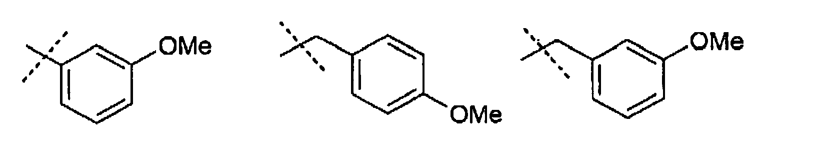

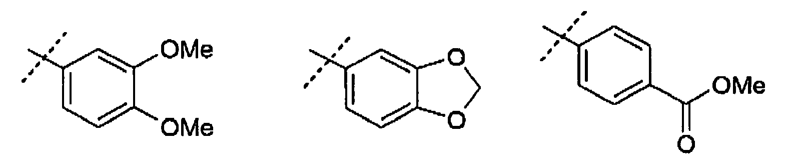

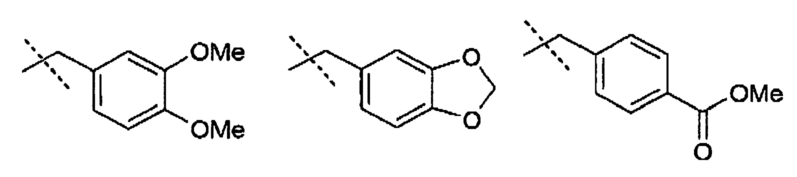

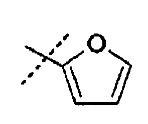

9. Einzelne Verbindung oder Mischung wie in einem der Ansprüche 6 bis 8 definiert, wobei die einzelne Verbindung der Formeln (I) und (*ent-*I) bzw. eine, mehrere oder sämtliche Verbindungen der Formeln (I) und (*ent*-I) in der Mischung ausgewählt sind aus der Gruppe bestehend aus:
(1) *N*-((1*S*,2*S*,5*R*)-2-Isopropyl-5-methyl-cyclohexyl)-terephthalsäuremethylester
(2) *N*-((1*R*,2*R*,5*S*)-2-Isopropyl-5-methyl-cyclohexyl)-terephthalsäuremethylester
(3) Benzo[1,3]dioxol-5-carbonsäure ((1*S*,2*S*,5*R*)-2-isopropyl-5-methylcyclohexyl)-amid
(4) Benzo[1,3]dioxol-5-carbonsäure ((1*R*,2*R*,5*S*)-2-isopropyl-5-methylcyclohexyl)-amid
(5) 2-Benzo[1,3]dioxol-5-yl-*N*-((1*S*,2*S*,5*R*)-2-isopropyl-5-methylcyclohexyl)acetamid
(6) 2-Benzo[1,3]dioxot-5-yl-*N*-((1*R*,2*R*,5*S*)-2-isopropyl-5-methyl cyclohexyl)acetamid

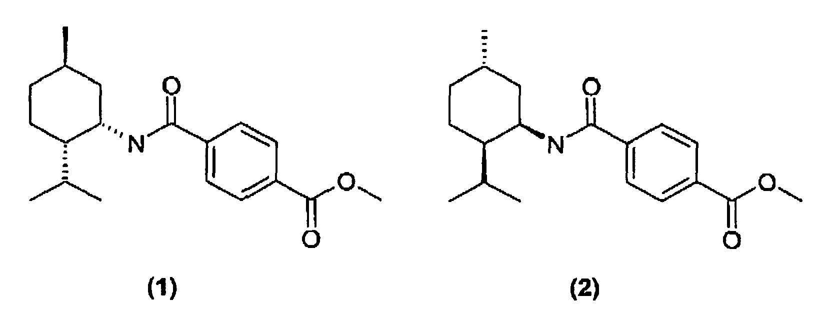

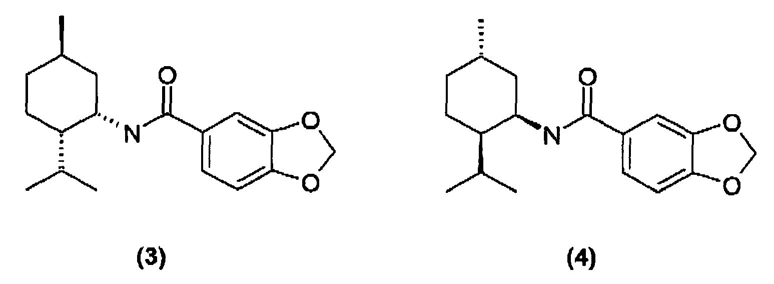

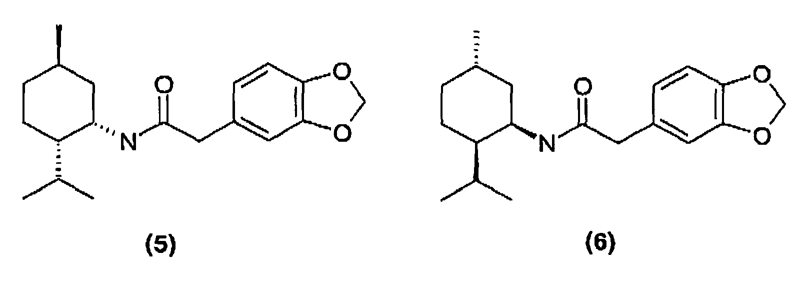

10. Zum Verzehr geeignete Zusammensetzung, umfassend bzw. bestehend aus einer geschmacklich wirksamen Menge einer Verbindung oder einer Mischung wie in einem der Ansprüche 1 bis 4 definiert
und einem oder mehreren weiteren zum Verzehr geeigneten Bestandteilen,
(i) wobei die Zusammensetzung keine Umkristallisationsmischung ist von einer Verbindung der Formel (I), in welcher der aromatische Rest Ar ausgewählt ist aus der Gruppe bestehend aus 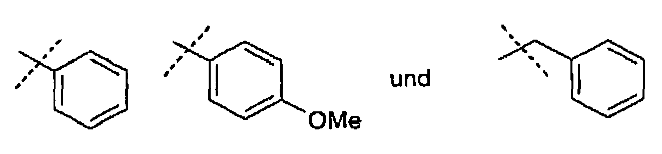 und wässrigem Alkohol als Lösungsmittel zur Umkristallisation,
oder
(ii) wobei die weiteren Bestandteile sind:
- feste Trägerstoffe oder
- feste Trägerstoffe und Aromakompositionen oder
- Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung.

11. Zusammensetzung nach Anspruch 10, wobei
die weiteren Bestandteile feste Trägerstoffe sind
und das Gewichtsverhältnis der Gesamtmasse der Verbindungen der Formeln (I) und (*ent-*I) wie in einem der Ansprüche 1 bis 4 definiert zu den festen Trägerstoffen im Bereich von 1 : 10 bis 1 : 100000, bevorzugt im Bereich von 1 : 50 bis 1 : 20000, besonders bevorzugt im Bereich von 1 : 100 bis 1 : 5000, liegt, bezogen auf die Trockenmasse der Zusammensetzung.

12. Verzehrbare Zubereitung oder Halbfertigware, umfassend eine geschmacklich wirksame Menge
- einer Verbindung oder Mischung wie in einem der Ansprüche 1 bis 4 definiert oder
- eine Zusammensetzung nach Anspruch 10 oder 11.
wobei die verzehrbare Zubereitung oder Halbfertigware keine Umkristallisationsmischung ist von einer Verbindung der Formel (I), in welcher der aromatische Rest Ar ausgewählt ist aus der Gruppe bestehend aus 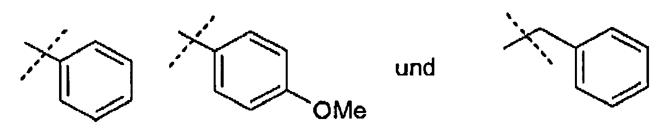 und wässrigem Alkohol als Lösungsmittel zur Umkristallisation.

13. Der Ernährung, der Mundpflege oder dem Genuss dienende gebrauchs- oder verzehrfertige Zubereitung nach Anspruch 12, umfassend insgesamt 0,01 ppm bis 100 ppm, bevorzugt 0,1 ppm bis 50 ppm, besonders bevorzugt 0,5 ppm bis 30 ppm an einer oder mehreren Verbindungen der Formel (I) und (*ent*-I) wie in einem der Ansprüche 1 bis 4 definiert bezogen auf das Gesamtgewicht der verzehrfertigen Zubereitung.

14. Halbfertigware gemäß Anspruch 12 umfassend
- insgesamt 10 ppm bis 100000 ppm, bevorzugt 25 ppm bis 5000 ppm, insbesondere 50 ppm bis 1200 ppm an einer oder mehreren Verbindungen der Formel (I) und (*ent*-I), wie in einem der Ansprüche 1 bis 4 definiert
- kein Natriumglutamat oder einen Anteil von 0,00001 bis 10 Gew.-%, bevorzugt 0,0001 bis 5 Gew.-%, insbesondere 0,001 Gew.-% bis 2 Gew.-% an Natriumglutamat,
- und gegebenenfalls einen Abteil von 0,0001 Gew.-% bis 90 Gew.-%, bevorzugt 0,001 Gew.-% bis 30 Gew.-% einer Aromakomposition,
bezogen auf das Gesamtgewicht der Halbfertigware.

## Claims

1. Use of a compound or of a mixture comprising two or more compounds or containing one or more compounds chosen from the group consisting of compounds of the formulae (I) and (*ent*-I) 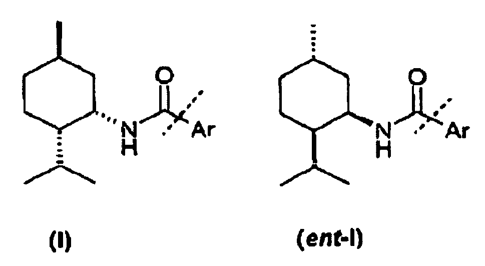 wherein in the formulae (I) and (*ent*-I):
the aromatic radical Ar is chosen from the group consisting of: 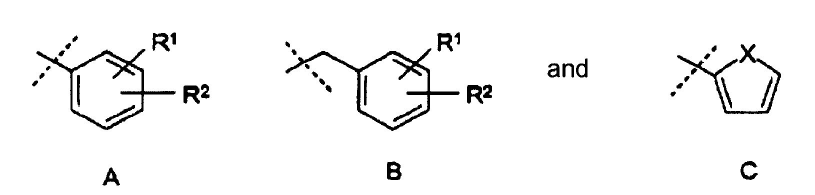
wherein the broken line marks the bond which links the aromatic radical Ar with the adjacent carbonyl-C atom in formula (I) or (*ent*-I),
and wherein in the aromatic radicals A and B:
R¹ and R² independently of each other are chosen from the group consisting of H, OH, OCH₃, OCH₂CH₃, OCH(CH₃)₂, COOCH₃, COOCH₂CH₃, COOCH(CH₃)₂,
or R¹ and R² are adjacent and together form an OCH₂O group,
and wherein in the aromatic radical C:
X is O or S,
as a flavouring substance or flavouring substance mixture.

2. Use of a compound or of a mixture as defined in claim 1, wherein for the compound of the formulae (I) and (*ent*-1) or for one, several or all of the compounds of the formulae (I) and (*ent*-1) in the mixture:
R¹ and R² in the aromatic radicals A and B independently of each other are chosen from the group consisting of H, OCH₃, COOCH₃,
or R¹ and R² are adjacent and together form an OCH₂O group,
and X in the aromatic radical C is O.

3. Use of a compound or of a mixture as defined in one of the preceding claims, wherein in the compound of the formulae (I) and (*ent*-1) or in one, several or all of the compounds of the formulae (I) and (*ent*-1) in the mixture the aromatic radical Ar is chosen from the group consisting of:

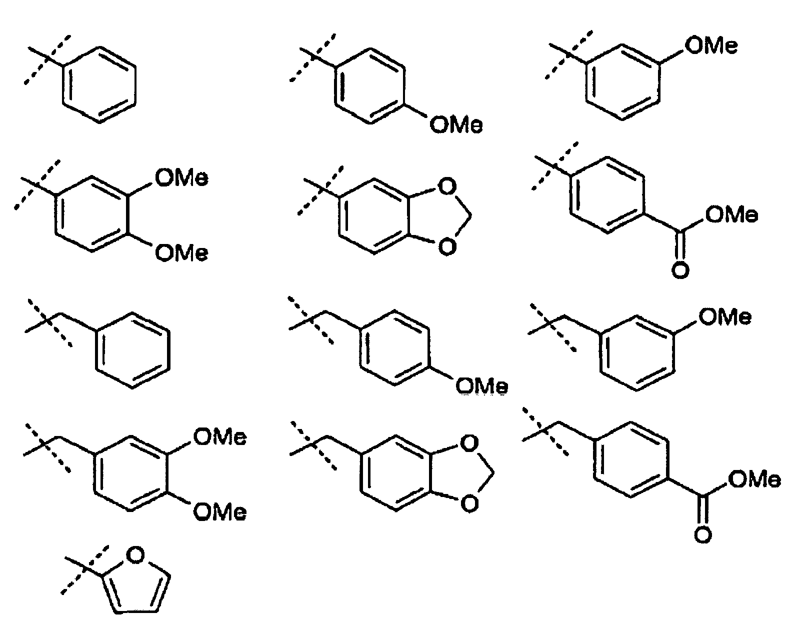

4. Use of a compound or of a mixture as defined in one of the preceding claims, wherein the compound of the formulae (I) and (*ent*-1) or one, several or all of the compounds of the formulae (I) and (*ent*-1) in the mixture are chosen from the group consisting of:
(1) *N*-((1*S*,2*S*,5*R*)-2-isopropyl-5-methyl-cyclohexyl)-terephthalic acid methyl ester
(2) *N*-((1*R*,2*R*,5*S*)-2-isopropyl-5-methyl-cyclohexyl)-terephthalic acid methyl ester
(3) benzo[1,3]dioxole-5-carboxylic acid ((1*S*,2*S*,5*R*)-2-isopropyl-5-methylcyclohexyl)-amide
(4) benzo[1,3]dioxole-5-carboxylic acid ((1*R*,2*R*,5*S*)-2-isopropyl-5-methylcyclohexyl)-amide
(5) 2-benzo [1,3]dioxol-5-yl-*N*-((1*S*,2*S*,5*R*)-2-isopropyl-5-methylcyclohexyl)acetamide
(6) 2-benzo[1,3]dioxol-5-yl-*N*-((1*R*,2*R*,5*S*)-2-isopropyl-5-methylcyclohexyl)acetamide

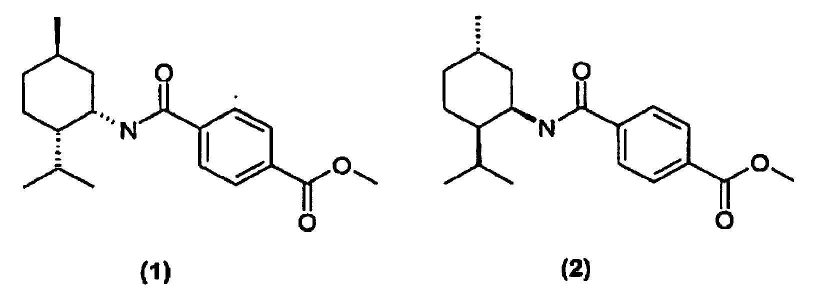

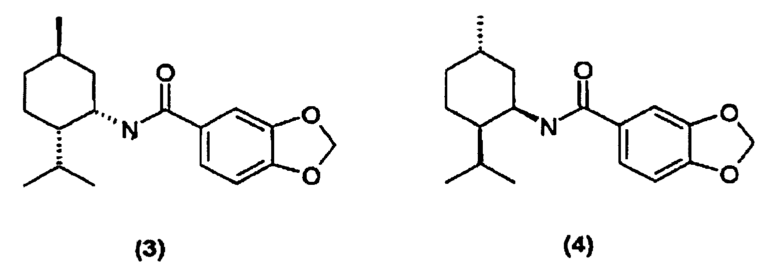

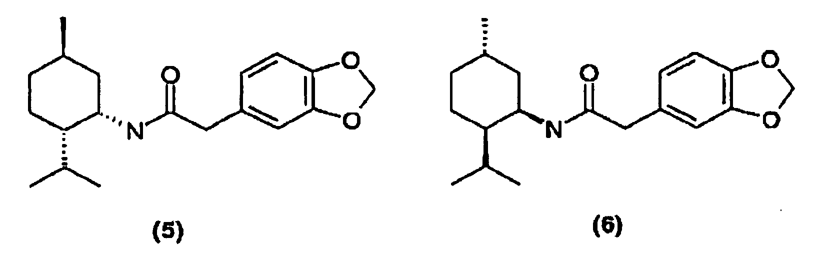

5. Use according to one of claims 1 to 4 for producing, imparting, modifying or intensifying an umami flavour.

6. Individual compound or mixture as defined in claims 1 to 4,
with the proviso the
(i) individual compounds of the formula (I), in which the aromatic radical Ar is chosen from the group consisting of 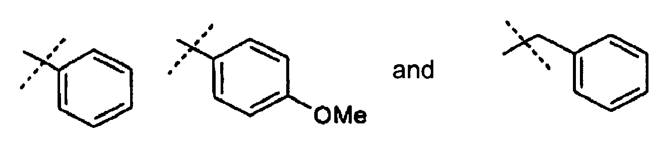 and
(ii) mixtures which comprise an enantiomer pair of the formulae (I) / (*ent*-1) with an aromatic radical chosen from the group consisting of 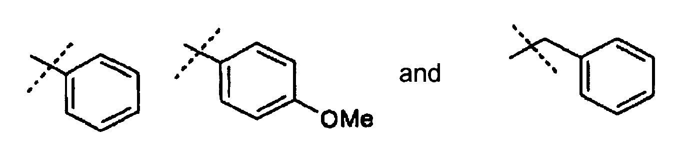 and
(iii) mixtures comprising
benzoyl-d-neo-menthylamine and benzoyl-1-menthylamine or
benzoyl-d-neo-menthylamine and benzoyl-d-iso-menthylamine
are excluded.

7. Individual compound or mixture as defined in claim 6,
wherein for the individual compound of the formulae (I) and (*ent*-1) or for one, several or all of the compounds of the formulae (I) and (*ent*-1) in the mixture:
R¹ and R² in the aromatic radicals A and B independently of each other are chosen from the group consisting of H, OCH₃, COOCH₃,
or R¹ and R² are adjacent and together form an OCH₂O group,
and X in the aromatic radical C is O.

8. Individual compound or mixture as defined in one of claims 6 or 7, wherein in the individual compound of the formulae (I) and (*ent-*1) or in one, several or all of the compounds of the formulae (I) and (*ent*-1) in the mixture the aromatic radical Ar is chosen from the group consisting of:

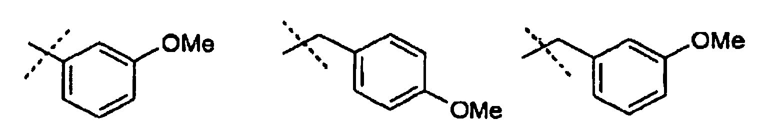

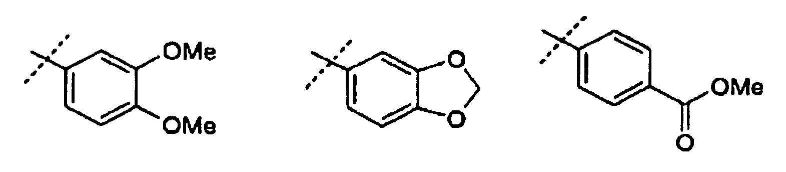

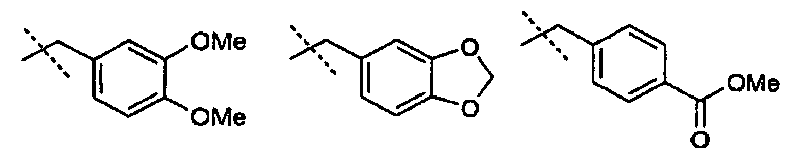

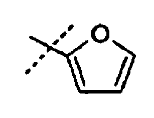

9. Individual compound or mixture as defined in one of claims 6 to 8, wherein the individual compound of the formulae (I) and (*ent*-1) or one, several or all of the compounds of the formulae (I) and (*ent*-1) in the mixture are chosen from the group consisting of:
(1) *N*-((1*S*,2*S*,5*R*)-2-isopropyl-5-methyl-cyclohexyl)-terephthalic acid methyl ester
(2) *N*-((1*R*,2*R*,5*S*)-2-isopropyl-5-methyl-cyclohexyl)-terephthalic acid methyl ester
(3) benzo[1,3]dioxole-5-carboxylic acid ((1*S*,2*S*,5*R*)-2-isopropyl-5-methylcyclohexyl)-amide
(4) benzo[1,3]dioxole-5-carboxylic acid ((1*R*,2*R*,5*S*)-2-isopropyl-5-methylcyclohexyl)-amide
(5) 2-benzo[1,3]dioxol-5-yl-*N*-((1S,2S,5R)-2-isopropyl-5-methylcyclohexyl)acetamide
(6) 2-benzo[1,3]dioxol-5-yl-*N*-((1*R*,2*R*,5*S*)-2-isopropyl-5-methylcyclohexyl)acetamide

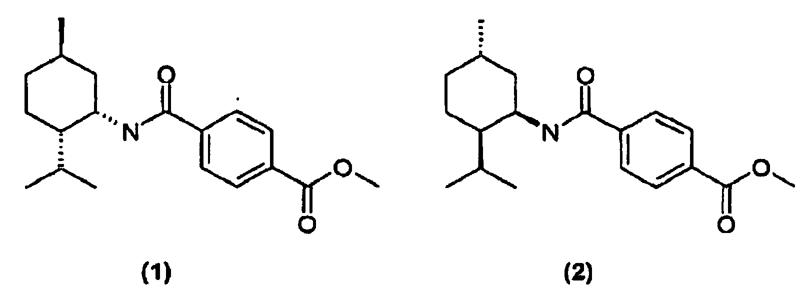

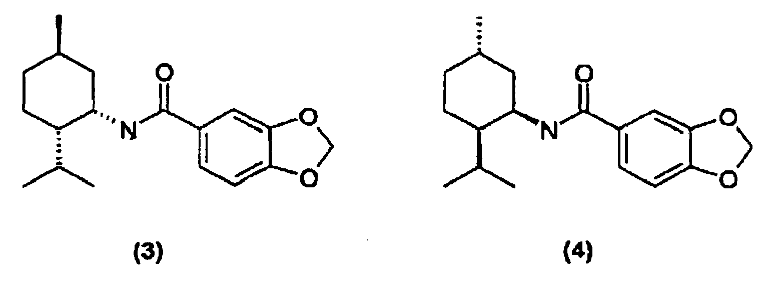

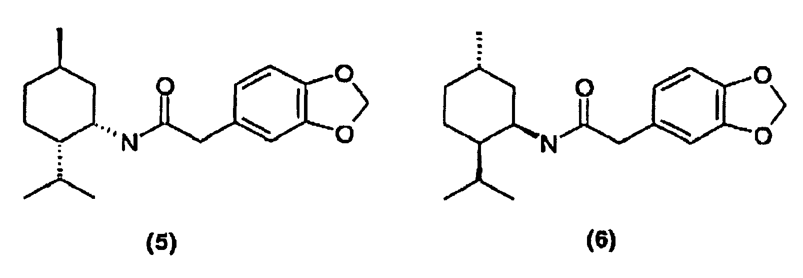

10. Composition suitable for consumption, comprising or consisting of
in an amount which has a flavouring effect, a compound or of a mixture as defined in one of claims 1 to 4
and one or more further constituents suitable for consumption,
(i) wherein the composition is not a recrystallization mixture of a compound of the formula (I), in which the aromatic radical Ar is chosen from the group consisting of 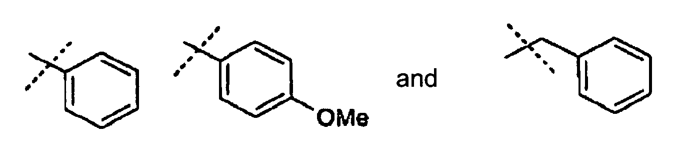 and aqueous alcohol as the solvent for the recrystallization,
or
(ii) wherein the further constituents are:
- solid carrier substances or
- solid carrier substances and aroma compositions or
- water, an oil phase, one or more W/O emulsifiers, optionally one or more antioxidants and optionally one or more substances for intensifying an antioxidative action.

11. Composition according to claim 10, wherein
the further constituents are solid carrier substances and the weight ratio of the total weight of the compounds of the formulae (I) and (ent-1) as defined in one of claims 1 to 4 to the solid carrier substances is in the range of from 1 : 10 to 1 : 100,000, preferably in the range of from 1 : 50 to 1 : 200,000, particularly preferably in the range of from 1 : 100 to 1 : 5,000, based on the dry weight of the composition.

12. Consumable preparation or semi-finished product, comprising, in an amount which has a flavouring effect,
- a compound or mixture as defined in one of claims 1 to 4 or
- a composition according to claim 10 or 11, wherein the consumable preparation or semi-finished product is not a recrystallization mixture of a compound of the formula (I), in which the aromatic radical Ar is chosen from the group consisting of 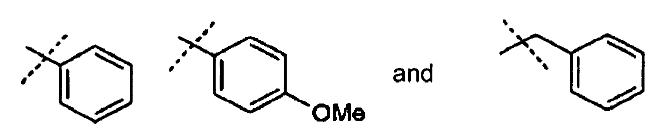 and aqueous alcohol as the solvent for the recrystallization.

13. Ready-to-use or ready-to-consume preparation for nutrition, oral care or consumption for pleasure, according to claim 12, comprising a total of 0.01 ppm to 100 ppm, preferably 0.1 ppm to 50 ppm, particularly preferably 0.5 ppm to 30 ppm of one or more compounds of the formula (I) and (*ent*-1) as defined in one of claims 1 to 4
based on the total weight of the ready-to-consume preparation.

14. Semi-finished product according to claim 12, comprising
- a total of 10 ppm to 100,000 ppm, preferably 25 ppm to 5,000 ppm, in particular 50 ppm to 1,200 ppm of one or more compounds of the formula (I) and (*ent*-1), as defined in one of claims 1 to 4,
- no sodium glutamate or a content of from 0.00001 to 10 wt.%, preferably 0.0001 to 5 wt.%, in particular 0.001 wt.% to 2 wt.% of sodium glutamate,
- and optionally a content of from 0.0001 wt.% to 90 wt.%, preferably 0.001 wt.% to 30 wt.%, of an aroma composition,
based on the total weight of the semi-finished product.

## Revendications

1. Utilisation d'un composé ou d'un mélange consistant en deux composés ou plus ou contenant un ou plusieurs composés choisis dans le groupe comprenant des composés de formule (I) et (*ent*-I) 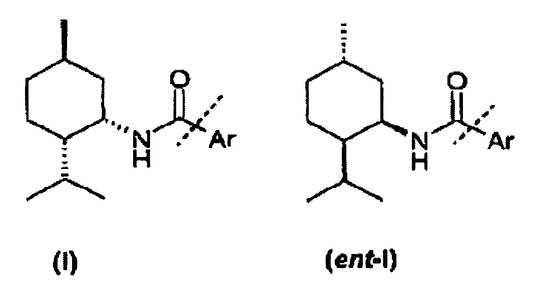 dans laquelle dans les formules (I) et (*ent*-I) :
le radical aromatique Ar est choisi dans le groupe comprenant : 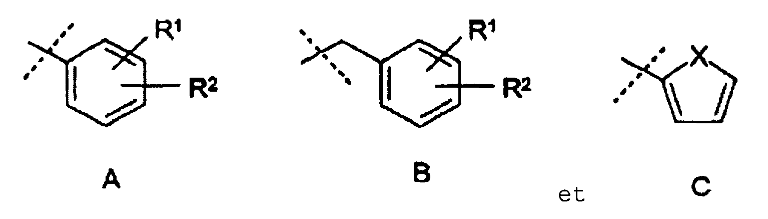 la ligne pointillée marquant la liaison qui relie le radical aromatique Ar
à l'atome de carbone du groupe carbonyle voisin dans la formule (I) ou (*ent*-I)
et dans laquelle, dans les radicaux aromatiques A et B :
R¹ et R² sont choisis indépendamment l'un de l'autre dans le groupe comprenant H, OH, OCH₃, OCH₂CH₃, OCH(CH₃)₂, COOCH₃, COOCH₂CH₃, COOCH(CH₃)₂,
ou R¹ et R² sont voisins et forment conjointement un groupe OCH₂O,
et dans laquelle dans le radical aromatique C :
X est O ou S,
en tant qu'agent de sapidité ou mélange d'agents de sapidité.

2. Utilisation d'un composé ou d'un mélange tel que défini dans la revendication 1, dans laquelle pour la liaison des formules (I) et (*ent*-I) ou pour un, plusieurs ou tous les composés de formules (I) et (*ent-*I) dans le mélange :
R¹ et R² dans les radicaux aromatiques A ou B sont choisis indépendamment l'un de l'autre dans le groupe comprenant H, OCH₃, COOCH₃,
ou R¹ et R² sont voisins et forment conjointement un groupe OCH₂O,
et X dans le radical aromatique C est O.

3. Utilisation d'un composé ou d'un mélange tel que défini dans l'une quelconque des revendications précédentes, dans laquelle dans le composé de formules (I) et (*ent*-I) ou dans un, plusieurs ou tous les composés de formules (I) et (*ent*-I) dans le mélange, le radical aromatique Ar est choisi dans le groupe comprenant :

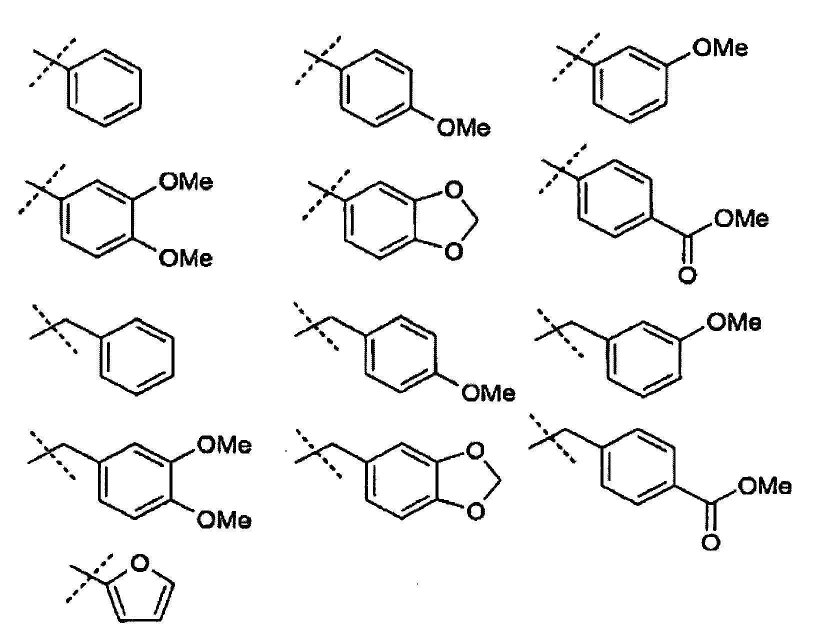

4. Utilisation d'un composé ou d'un mélange tel que défini dans l'une quelconque des revendications précédentes, dans laquelle le composé des formules (I) et (*ent*-I) ou un, plusieurs ou tous les composés de formules (I) et (*ent*-I) dans le mélange sont choisis dans le groupe comprenant :
(1) le méthylester d'acide N-((1S,2S,5R)-2-isopropyl-5-méthyl-cyclohexyl)-téréphtalique
(2) le méthylester d'acide N-((1R,2R,5S)-2-isopropyl-5-méthyl-cyclohexyl)-téréphtalique
(3) l'acide benzo[1,3]dioxol-5-carboxylique ((1S,2S,5R)-2-isopropyl-5-méthyl-cyclohexyl)-amide
(4) l'acide benzo[1,3]dioxol-5-carboxylique ((1R,2R,5S)-2-isopropyl-5-méthyl-cyclohexyl)-amide
(5) le 2-benzo[1,3]dioxol-5-yl-N-((1S,2S,5R)-2-isopropyl-5-méthyl-cyclohexyl)-acétamide
(6) le 2-benzo[l,3]dioxol-5-yl-N-((1R,2R,5S)-2-isopropyl-5-méthyl-cyclohexyl)-acétamide

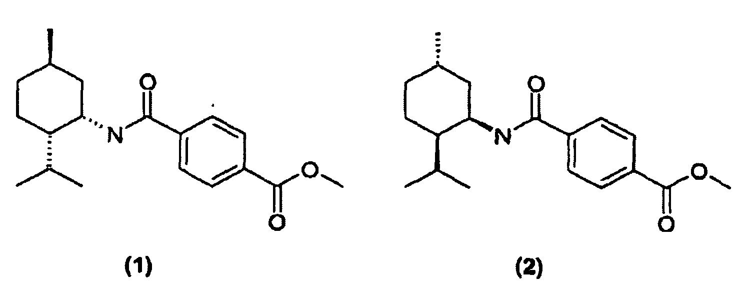

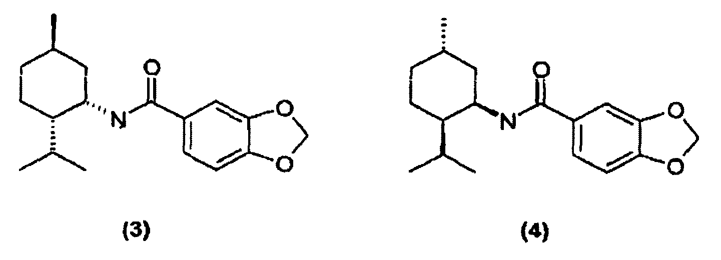

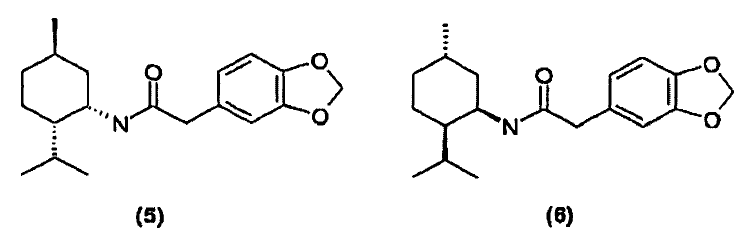

5. Utilisation selon l'une quelconque des revendications 1 à 4, pour générer, transmettre, modifier ou renforcer un goût umami.

6. Composé ou mélange individuels tel que défini dans les revendications 1 à 4, à condition que
(i) des composés individuel de formule (I) dans lesquels le radical aromatique Ar est choisi dans le groupe comprenant 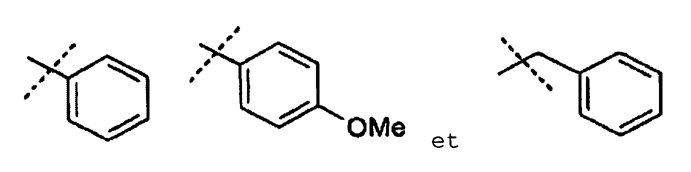 et
(ii) des mélanges qui consistent en une paire d'énantiomères de formules (I)/(*ent*-I) comportant un radical aromatique choisi dans le groupe comprenant 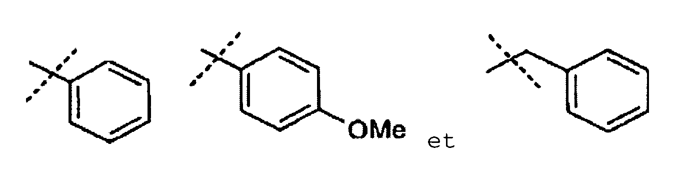 et
(iii) des mélanges consistant en ce que
du benzoyl-d-néo-menthylamine et du benzoyl-1-menthylamine ou
du benzoyl-d-néo-menthylamine et du benzoyl-d-iso-menthylamine
soient exclus.

7. Composé individuel ou mélange tel que défini dans la revendication 6,
dans lequel pour le composé individuel de formule (I) et (*ent*-I) ou pour un, plusieurs ou tous les composés de formule (I) et (*ent-*I) dans le mélange :
R¹ et R² dans les radicaux A ou B aromatiques sont choisis indépendamment l'un de l'autre dans le groupe comprenant H, OCH₃, COOCH₃,
ou R¹ et R² sont voisins et forment conjointement un groupe OCH₂O,
et X dans le radical C aromatique est O.

8. Composé individuel ou mélange tel que défini dans l'une des revendications 6 ou 7, dans lequel dans le composé individuel de formules (I) et (*ent*-I) ou dans un, plusieurs ou tous les composés de formule (I) et (*ent*-I) dans le mélange, le radical aromatique Ar est choisi dans le groupe comprenant :

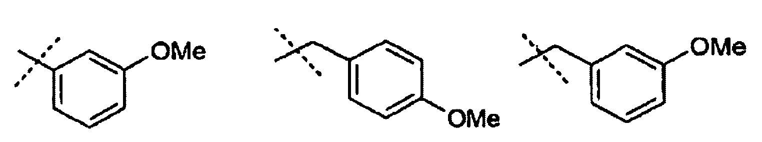

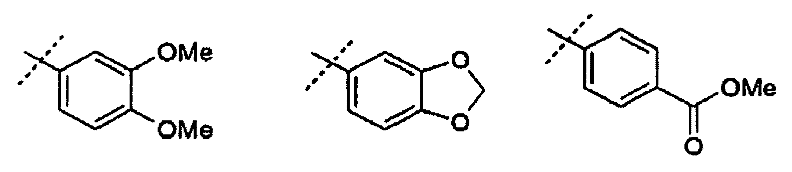

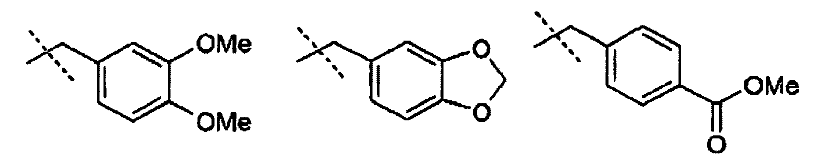

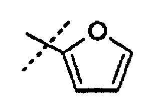

9. Composé individuel ou mélange tel que défini dans l'une des revendications 6 à 8, dans lequel le composé individuel de formules (I) et (*ent*-I) ou un, plusieurs ou tous les composés de formule (I) et (*ent*-I) dans le mélange sont choisis dans le groupe comprenant :
(1) le méthylester d'acide N-((1S,2S,5R)-2-isopropyl-5-méthyl-cyclohexyl)-téréphtalique
(2) le méthylester d'acide N-((1R,2R,5S)-2-isopropyl-5-méthyl-cyclohexyl)-téréphtalique
(3) l'acide benzo[1,3]dioxol-5-carboxylique ((1S,2S,5R)-2-isopropyl-5-méthyl-cyclohexyl)-amide
(4) l'acide benzo[1,3]dioxol-5-carboxylique ((1R,2R,5S)-2-isopropyl-5-méthyl-cyclohexyl)-amide
(5) le 2-benzo[1,3]dioxol-5-yl-*N*-((1S,2S,5R)-2-isopropyl-5-méthyl-cyclohexyl)-acétamide
(6) le 2-benzo[1,3]dioxol-5-yl-*N*-((1R,2R,5S)-2-isopropyl-5-méthyl-cyclohexyl)-acétamide

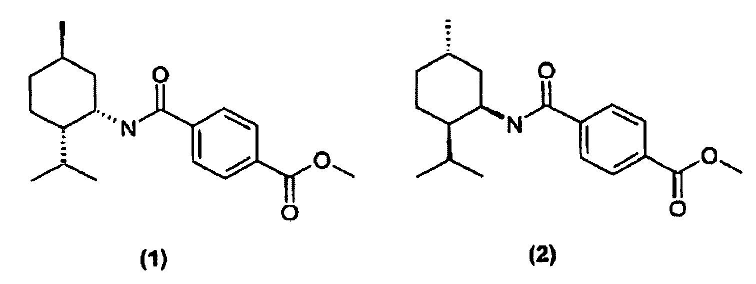

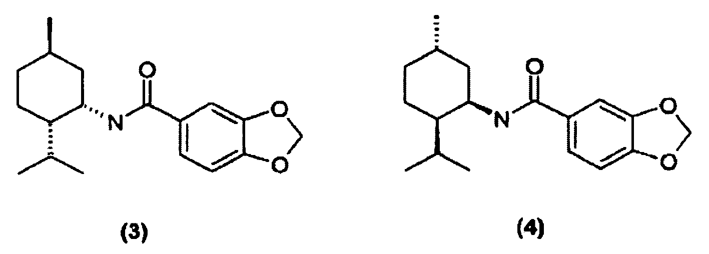

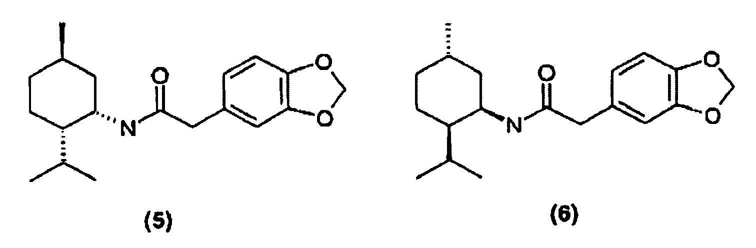

10. Composition appropriée pour la consommation, comprenant ou consistant en
une quantité efficace d'un point de vue gustatif d'un composé ou d'un mélange tel que défini dans l'une quelconque des revendications 1 à 4,
et un ou plusieurs autres composants appropriés pour la consommation,
(i) la composition n'étant pas un mélange de recristallisation d'un composé de formule (I) dans lequel le radical aromatique Ar est choisi dans le groupe comprenant 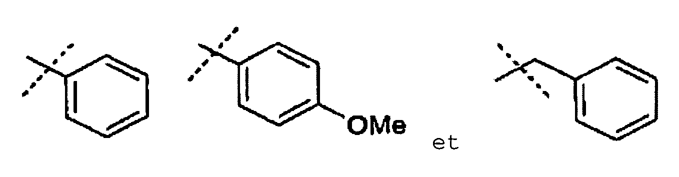 et un alcool aqueux comme solvant de recristallisation,
ou
(ii) les autres composants étant :
- des porteurs solides ou
- des porteurs solides et des compositions aromatiques ou
- de l'eau, une phase huileuse, un ou plusieurs émulsifiant(s) eau/huile, éventuellement un ou plusieurs antioxydant(s) et éventuellement une ou plusieurs substance(s) pour renforcer un effet antioxydant.

11. Composition selon la revendication 10, dans laquelle
les autres composants sont des porteurs solides
et le rapport en poids de la masse totale des composés de formules (I) et (*ent*-I) tels que définis dans l'une quelconque des revendications 1 à 4, par rapport aux porteurs solides se situe dans une plage de 1:10 à 1:100000, de préférence dans une plage de 1:50 à 1:20000, de manière particulièrement préférée dans une plage de 1:100 à 1:5000, par rapport au poids sec de la composition.

12. Préparation consommable ou produit semi-transformé, comprenant une quantité efficace d'un point de vue gustatif
- d'un composé ou d'un mélange tel que défini dans l'une quelconque des revendications 1 à 4, ou
- d'une composition selon la revendication 10 ou 11,
la préparation consommable ou le produit semi-transformé n'étant pas un mélange de recristallisation d'un composé de formule (I), dans lequel le radical aromatique Ar est choisi dans le groupe comprenant 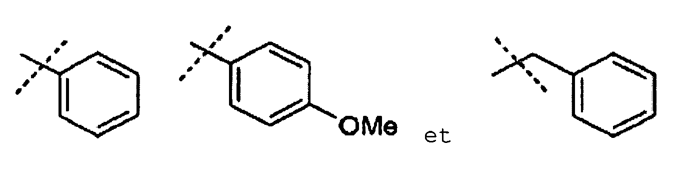 et de l'alcool aqueux comme solvant de recristallisation.

13. Préparation prête à l'emploi ou prête à la consommation pour l'alimentation, l'hygiène buccale ou le plaisir gustatif selon la revendication 12, comprenant
au total 0,01 ppm à 100 ppm, de préférence 0,1 ppm à 50 ppm, de manière particulièrement préférée 0,5 ppm à 30 ppm d'un ou plusieurs composés de formules (I) et (*ent*-I) tel(s) que défini dans l'une quelconque des revendications 1 à 4,
par rapport au poids total de la préparation consommable.

14. Produit semi-transformé selon la revendication 12, comprenant
- au total, 10 ppm à 100000 ppm, de préférence 25 ppm à 5000 ppm, en particulier 50 ppm à 1200 ppm d'un ou plusieurs composés de formule (I) et (*ent*-I) tel(s) que définis dans l'une quelconque des revendications 1 à 4,
- ne comprenant pas de glutamate de sodium ou comprenant une proportion de 0,00001 à 10 % en poids, de préférence de 0,0001 à 5 % en poids, en particulier de 0,001 % en poids à 2 % en poids de glutamate de sodium
- et éventuellement une proportion de 0,0001 % en poids à 90 % en poids, de préférence de 0,001 % en poids à 30 % en poids d'une composition aromatique par rapport au poids total du produit semi-transformé.
